# FASCICULE DE BREVET EUROPEEN

(11) **EP 2 178 879 B1**
(45) Date de publication et mention de la délivrance du brevet: **03.07.2013**
(21) Numéro de dépôt: 08831515.5
(22) Date de dépôt: 18.07.2008
(51) Int. Cl.: C07D 487/04, C07D 519/00, A61K 31/5025, A61P 35/00, A61P 25/20, A61P 25/22, C07D 237/20, C07D 233/56, C07D 207/08, C07D 211/26, C07D 471/10, C07D 498/10, C07D 295/00, A61K 31/519, A61P 25/30, A61P 25/28

(54) **DERIVES DE 6-CYCLOAMINO-S-(PYRIDAZIN-YL)IMIDAZO[1,2-b]-PYRIDAZINE, LEUR PREPARATION ET LEUR APPLICATION EN THERAPEUTIQUE.**
6-CYCLOAMINO-S-(PYRIDAZIN-4-YL)-IMIDAZO-[1,2-B]-PYRIDAZIN UND DERIVATE DAVON, IHRE HERSTELLUNG UND IHRE THERAPEUTISCHE VERWENDUNG
6-CYCLOAMINO-S-(PYRIDAZIN-4-YL)IMIDAZO[1,2-B]-PYRIDAZINE AND DERIVATIVES THEREOF PREPARATION AND THERAPEUTIC APPLICATION THEREOF

(30) Priorité: 19.07.2007 US 950711 P; 19.07.2007 FR 0705224
(43) Date de publication de la demande: 28.04.2010
(73) Titulaire: SANOFI, 75008 Paris (FR)
(72) Inventeur: BURNIER, Philippe, F-75013 Paris (FR); CHIANG, Yulin, Bridgewater, NJ 08807 (US); COTE-DES-COMBES, Sylvain, F-75013 Paris (FR); LI, Adrien-Tak, F-75013 Paris (FR); PUECH, Frédéric, F-75013 Paris (FR)
(74) Mandataire: Le Coupanec, Pascale A.M.P.
(86) Numéro de dépôt international: PCT/FR2008/001057
(87) Numéro de publication internationale: WO 2009/037394

(56) Documents cités:
- WO-A-89/01333
- WO-A-2005/080355
- WO-A-2007/009773
- WO-A-2007/013673
- WO-A-2007/025540

## Description

La présente invention se rapporte à des dérivés de 6-cycloamino-3-(pyridazin-4-yl)imidazo[1,2-*b*]pyridazine, à leur préparation et à leur application en thérapeutique, dans le traitement ou la prévention de maladies impliquant la caséine kinase 1 epsilon et/ou la caséine kinase 1 delta.

Des dérivés d'inidazo[1,2-b]pyridazine sont décrits dans WO 89/01333 et WO 2007/013673.

La présente invention a pour objet les composés répondant à la formule générale (I) 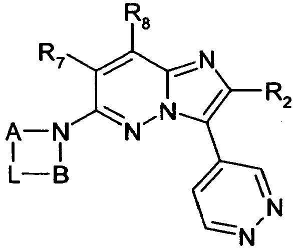 dans laquelle :
- R₂ représente un groupe aryle éventuellement substitué par un ou plusieurs substituants choisis parmi les atomes d'halogène et les groupes C₁₋₆ alkyle, C₁₋₆ alkyloxy, C₁₋₆ alkylthio, C₁₋₆ fluoroalkyle, C₁₋₆-fluoroalkyloxy, -CN ;
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente, soit un atome d'azote éventuellement substitué par un groupe R_{c} ou R_{d}, soit un atome de carbone substitué par un groupe Rₑ₁ et un groupe R_{d} ou deux groupes Rₑ₂ ;
   les atomes de carbone de A et de B étant éventuellement substitués par un ou plusieurs groupes R_{f} identiques ou différents l'un de l'autre ;
- Rₐ, R_{b} et R_{c} sont définis tels que :
   deux groupes Rₐ peuvent former ensemble un groupe C₁₋₆-alkylène ;
   Rₐ et R_{b} peuvent former ensemble une liaison ou un groupe C₁₋₆-alkylène ;
   Rₐ et R_{c} peuvent former ensemble une liaison ou un groupe C₁₋₆-alkylène ;
   R_{b} et R_{c} peuvent former ensemble une liaison ou un groupe C₁₋₆-alkylène ;
- R_{d} représente un groupe choisi parmi l'atome d'hydrogène et les groupes C₁₋₆-alkyle, C₃₋₇₋cycloalkyle, C₃₋₇-cycloalkyl-C₁₋₆-alkyle, hydroxy-C₁₋₆-alkyle, C₁₋₆-alkyloxy-C₁₋₆-alkyle, C₁₋₆-alkylthio-C-₁₋₆-alkyle, C₁₋₆-fluoroalkyle, benzyle ;
- Rₑ₁ représente un groupe -NR₄R₅ ou une monoamine cyclique comportant éventuellement un atome d'oxygène, la monoamine cyclique étant éventuellement substituée par un ou plusieurs substituants choisis parmi l'atome de fluor et les groupes C₁₋₆-alkyle, C₁₋₆₋alkyloxy, hydroxyle ;
- Deux Rₑ₂ forment avec l'atome de carbone qui les porte une monoamine cyclique comportant éventuellement un atome d'oxygène, cette monoamine cyclique étant éventuellement substituée par un ou plusieurs groupes R_{f} identiques ou différents l'un de l'autre ;
- R_{f} représente un groupe C₁₋₆-alkyle, C₃₋₇-cycloalkyle, C₃₋₇-cycloalkyle-C₁₋₆-alkyle, C₁₋₆-alkyloxy-C₁₋₆-alkyle, hydroxy-C₁₋₆-alkyle, C₁₋₆-fluoroalkyle ou benzyle ;
- R₄ et R₅ représentent, indépendamment l'un de l'autre, un atome d'hydrogène ou un groupe C₁₋₆-alkyle, C₃₋₇-cycloalkyle, C₃₋₇-cycloalkyle-C₁₋₆-alkyle ;
- R₇ et R₈ représentent, indépendamment l'un de l'autre, un atome d'hydrogène ou un groupe un groupe C₁₋₆-alkyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention. Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de l'invention, on entend par :
- C_{t-z} où t et z peuvent prendre les valeurs de 1 à 7, une chaîne carbonée pouvant avoir de t à z atomes de carbone, par exemple C₁₋₇ une chaîne carbonée qui peut avoir de 1 à 7 atomes de carbone ;
- alkyle, un groupe aliphatique saturé, linéaire ou ramifié; par exemple un groupe C₁₋₇-alkyle représente une chaîne carbonée de 1 à 7 atomes de carbone, linéaire ou ramifiée, par exemple un méthyle, éthyle, propyle, isopropyle, butyle, isobutyle, terbutyle, pentyle, hexyle, heptyle ;
- alkylène, un groupe alkyle divalent saturé, linéaire ou ramifié, par exemple un groupe C₁₋₆-alkylène représente une chaîne carbonée divalente de 1 à 6 atomes de carbone, linéaire ou ramifiée, par exemple un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, par exemple un cyclopropyle, cyclobutyle, cyclopentyle, cyclohexyle, cycloheptyle ;
- hydroxyle, un groupe -OH ;
- -CN, un groupe nitrile ;
- monoamine cyclique, une chaîne carbonée saturée cyclique ou polycyclique, éventuellement pontée ou fusionnée comportant 1 atome d'azote ;
   A titre d'exemple de composé formé par N, A, L et B constituant une monoamine cyclique comportant éventuellement un atome d'oxygène, on peut notamment citer l'aziridine, l'azétidine, la pyrrolidine, la pipéridine, l'azépine, la morpholine, l'homopipéridine, la décahydroquinoline, la décahydroisoquinoline, l'azabicyclo-heptane, l'azabicyclo-octane, l'azabicyclo-nonane, l'aza-oxo-bicyclo-heptane, l'aza-oxo-bicyclo-octane ;
- diamine cyclique, une chaîne carbonée saturée cyclique ou polycyclique, éventuellement pontée ou fusionnée comportant 2 atomes d'azote ;
   A titre d'exemple de composé formé par N, A, L et B constituant une diamine cyclique comportant éventuellement un atome d'oxygène, on peut notamment citer la pipérazine, l'homopipérazine, la diaza-cyclo-octane, le diaza-cyclo-nonane, le diaza-cyclo-décane, le diaza-cyclo-undécane, l'octahydro-pyrrolo-pyrazine, l'octahydro-pyrrolo-diazépine, l'octahydro-pyrrolo-pyrrole, l'octahydro-pyrrolo-pyridine, le décahydro-naphthyridine, le diaza-bicyclo-heptane, le diaza-bicyclo-octane, le diaza-bicyclo-nonane, diaza-spiro-heptane, diaza-spiro-octane, le diaza-spiro-nonane, le diaza-spiro-décane, le diaza-spiro-undécane, l'oxa-diaza-spiro-undécane ;
- hydroxyakyle, un groupe alkyle dont un atome d'hydrogène a été substitué par un groupe hydroxyle ;
- alkyloxy, un groupe -O-alkyle ;
- alkylthio, un groupe -S-alkyle ;
- fluoroalkyle, un groupe alkyle dont un ou plusieurs atomes d'hydrogène ont été substitués par un atome de fluor ;
- fluoroalkyloxy, un groupe alkyloxy dont un ou plusieurs atomes d'hydrogène ont été substitués par un atome de fluor ;
- un atome d'halogène, un atome de fluor, de chlore, de brome ou d'iode ;
- aryle, un groupe aromatique mono- ou bicyclique comprenant entre 6 et 10 atomes de carbones. A titre d'exemple de groupe aryle, on peut citer les groupes phényle ou naphtyle.

Parmi les composés de formule générale (I) objets de l'invention, un premier groupe de composés est constitué par les composés pour lesquels R₂ représente un phényle, éventuellement substitué par un ou plusieurs atomes d'halogène ou groupes C₁₋₆ alkyle, C₁₋₆-fluoroalkyle ;
A, L, B, R₇ et R₈ étant tels que définis ci-dessus.

Parmi les composés de formule générale (I) objets de l'invention, un second groupe de composés est constitué par les composés pour lesquels R₂ représente un phényle, éventuellement substitué par un ou plusieurs atomes de fluor, chlore ou groupes méthyle, trifluorométhyle ;
A, L, B, R₇ et R₈ étant tels que définis ci-dessus.

Parmi les composés de formule générale (I) objets de l'invention, un troisième groupe de composés est constitué par les composés pour lesquels R₂ représente un phényle, 3-fluoro-phényle, 4-fluoro-phényle, 3,4-difluoro-phényle, 3,5-difluoro-phényle, 3,5-dichloro-phényle, 3,5-diméthyl-phényle, 3,5-di(trifluorométhyl)phényle ;
A, L, B, R₇ et R₈ étant tels que définis ci-dessus.

Parmi les composés de formule générale (I) objets de l'invention, un quatrième groupe de composés est constitué par les composés pour lesquels R₇ et R₈ représentent, indépendamment l'un de l'autre, un atome d'hydrogène ou un groupe méthyle ;
R₂, A, L et B étant tels que définis ci-dessus.

Parmi les composés de formule générale (I) objets de l'invention, un cinquième groupe de composés est constitué par les composés pour lesquels :
- A représente un groupe C₁₋₇-éventuellement substitué par un ou deux groupes Rₐ ;
- B représente un groupe C₁₋₇-alkylène éventuellement substitué par un groupe R_{b} ;
- L représente un atome d'azote éventuellement substitué par un groupe R_{c} ou R_{d} ;
   les atomes de carbone de A et de B étant éventuellement substitués par un ou plusieurs groupes R_{f} identiques ou différents l'un de l'autre ;
- Rₐ et R_{b} peuvent former ensemble une liaison ou un groupe C₁₋₆-alkylène ;
- Rₐ et R_{c} peuvent former ensemble une liaison ou un groupe C₁₋₆-alkylène ;
- R_{b} et R_{c} peuvent former ensemble une liaison ou un groupe C₁₋₆-alkylène ;
- R_{d} représente un substituant choisi parmi un groupe C₁₋₆-alkyle, C₃₋₇-cycloalkyle, C₃₋₇ cycloalkyle-C₁-₆-alkyle, hydroxy-C₁₋₆-alkyle, C₁₋₆-alkyloxy-C₁₋₆-alkyle, C₁₋₆-fluoroalkyle, benzyle ;
- R_{f} représente un groupe C₁₋₆-alkyle, hydroxy-C₁₋₆-alkyle, C₁₋₆-alkyloxy-C₁₋₆-alkyle, C₁₋₆-fluoroalkyle ;
   R₂, R₇ et R₈ étant tels que définis ci-dessus.

Parmi les composés de formule générale (I) objets de l'invention, un sixième groupe de composés est constitué par les composés pour lesquels :
l'amine cyclique formée par -N-A-L-B- représente un groupe pipérazinyle, octa-hydro-pyrrolo-pyrazinyle, diaza-bicyclo-heptyle, hexa-hydro-pyrrolo-pyrrolyte, éventuellement substitué par un ou plusieurs groupes méthyle, éthyle, propyle, isopropyle, cyclopropyle, méthylpropyle, hydroxyméthyle, hydroxy-éthyle, hydroxy(méthyl)propyle, hydroxy(méthyl)butyle, fluorométhyle, fluoroéthyle, benzyle ;
R₂, R₇ et R₈ étant tels que définis ci-dessus.

Parmi les composés de formule générale (I) objets de l'invention, un septième groupe de composés est constitué par les composés pour lesquels :
l'amine cyclique formée par -N-A-L-B- représente un groupe pipérazin-1-yle, 3-(*R*)-méthyl-pipérazin-1-yle, 3,3-diméthyl-pipérazin-1-yle, *cis*-3,5-diméthyl-pipérazin-1-yle, 4-méthyl-pipérazin-1-yle, 4-(2-hydroxy-éthyl)-pipérazin-1-yle, 4-(2-fluoroéthyl)-pipérazin-1-yle, 4-isopropyl-pipérazin-1-yle, 4-cyclopropyl-pipérazin-1-yle, 4-benzyl-pipérazin-1-yle, 4-(2-hydroxy-2-méthylpropyl)-pipérazin-1-yle, 4-(3-hydroxy-3-méthylbutyl)-pipérazin-1-yle, (*S*)-octahydro-pyrrolo[1,2-a]pyrazin-2-yle, (1*S*,4*S*)-2,5-diazabicyclo[2.2.1]hept-2-yle, 5-(2-hydroxy-éthyl)-(1*S*,4*S*)-2,5-diaza-bicyclo[2.2.1]hept-2-yle, 5-isopropyl-(1*S*,4*S*)-2,5-diazabicyclo[2.2.1]hept-2-yle, hexahydro-pyrrolo[3,4-*c*]pyrrol-2(1*H*)-yle, 5-méthyl-hexahydro-pyrrolo[3,4-*c*]pyrrol-2(1*H*)-yle, 5-(2-isopropyl)-hexahydropyrrolo[3,4-*c*]pyrrol-2(1*H*)-yle, 5-(2-hydroxy-éthyl)-hexahydropyrrolo[3,4-*c*]pyrrol-2(1*H*)-yle, 5-(benzyl)-hexahydro-pyrrolo[3,4-*c*]pyrrol-2(1*H*)-yle, 3-(hydroxyméthyl)-pipérazin-1-yle, 3-fluorométhyl-pipérazin-1-yle ;
R₂, R₇ et R₈ étant tels que définis ci-dessus.

Parmi les composés de formule générale (I) objets de l'invention, un huitième groupe de composés est constitué par les composés pour lesquels :
l'amine cyclique formée par -N-A-L-B- représente un groupe pipérazinyle, octa-hydro-pyrrolo-pyrazinyle, diaza-bicyclo-heptyle, hexa-hydro-pyrrolo-pyrrolyle, éventuellement substitué par un ou plusieurs groupes méthyle, éthyle, propyle, isopropyle, cyclopropyle, méthylpropyle, hydroxy-éthyle, hydroxy(méthyl)propyle, hydroxy(méthyl)butyle, fluoroéthyle, benzyle ;
R₂, R₇ et R₈ étant tels que définis ci-dessus.

Parmi les composés de formule générale (I) objets de l'invention, un neuvième groupe de composés est constitué par les composés pour lesquels :
l'amine cyclique formée par -N-A-L-B- représente un groupe pipérazin-1-yle, 3-(*R*)-méthyl-pipérazin-1-yle, 3,3-diméthyl-pipérazin-1-yle, *cis*-3,5-diméthyl-pipérazin-1-yle, 4-méthyl-pipérazin-1-yle, 4-(2-hydroxy-éthyl)-pipérazin-1-yle, 4-(2-fluoroéthyl)-pipérazin-1-yle, 4-isopropyl-pipérazin-1-yle, 4-cyclopropyl-pipérazin-1-yle, 4-benzyl-pipérazin-1-yle, 4-(2-hydroxy-2-méthylpropyl)-pipérazin-1-yle, 4-(3-hydroxy-3-méthylbutyl)-pipérazin-1-yle, (*S*)-octahydro-pyrrolo[1,2-a]pyrazin-2-yle, (1*S*,4*S*)-2,5-diazabicyclo[2.2.1]hept-2-yle, 5-(2-hydroxy-éthyl)-(1*S*,4*S*)-2,5-diaza-bicyclo[2.2.1]hept-2-yle, 5-isopropyl-(1*S*,4*S*)-2,5-diazabicyclo[2.2.1]hept-2-yle, hexahydro-pyrrolo[3,4-*c*]pyrrol-2(1*H*)-yle, 5-méthyl-hexahydro-pyrrolo[3,4-*c*]pyrrol-2(1*H*)-yle, 5-(2-isopropyl)-hexahydropyrrolo[3,4-*c*]pyrrol-2(1*H*)-yle, 5-(2-hydroxy-éthyl)-hexahydropyrrolo[3,4-*c*]pyrrol-2(1*H*)-yle, 5-(benzyl)-hexahydro-pyrrolo[3,4-*c*]pyrrol-2(1*H*)-yle ;
R₂, R₇ et R₈ étant tels que définis ci-dessus.

Parmi les composés de formule générale (I) objets de l'invention, un dixième groupe de composés est constitué par les composés pour lesquels :
- A représente un groupe C₁₋₇-alkylène ;
- B représente un groupe C₁₋₇-alkylène ;
- L représente un atome de carbone substitué par deux groupes Rₑ₂ ;
   les atomes de carbone de A et de B étant éventuellement substitués par un ou plusieurs groupes R_{f} identiques ou différents l'un de l'autre ;
- R_{f} représente un groupe C₁₋₆-alkyle ;
- Deux Rₑ₂ forment, avec l'atome de carbone qui les porte, un groupe azétidine, pyrrolidine, pipéridine ou morpholine ;
   R₂, R₇ et R₈ étant tels que définis ci-dessus.

Parmi les composés de formule générale (I) objets de l'invention, un onzième groupe de composés est constitué par les composés pour lesquels :
- l'amine cyclique formée par -N-A-L-B- représente un groupe diaza-spiro-undécyle, oxa-diaza-spiro-undécyle ;
   R₂, R₇ et R₈ étant tels que définis ci-dessus.

Parmi les composés de formule générale (I) objets de l'invention, un douzième groupe de composés est constitué par les composés pour lesquels :
- l'amine cyclique formée par -N-A-L-B- représente un groupe 2,9-diaza-spiro[5.5]undéc-9-yle, 1-oxa-4,9-diazaspiro[5.5]undéc-9-yle ;
- R₂, R₇ et R₈ étant tels que définis ci-dessus.

Parmi les composés de formule générale (I) objets de l'invention, un treizième groupe de composés est constitué par les composés pour lesquels :
- A représente un groupe C₁₋₇-alkylène ;
- B représente un groupe C₁₋₇-alkylène ;
- L représente un atome de carbone substitué par un groupe Rₑ₁ et un groupe R_{d} ;
- R_{d} représente un atome d'hydrogène ;
- Rₑ₁ représente un groupe -NR₄R₅ ou une monoamine cyclique ;
- R₄ et R₅ représentent, indépendamment l'un de l'autre, un atome d'hydrogène ou un groupe C₁₋₆-alkyle, C₃₋₇-cycloalkyle, C₃₋₇-cycloalkyle-C₁₋₆-alkyle ;
   R₂, R₇ et R₈ étant tels que définis ci-dessus.

Parmi les composés de formule générale (I) objets de l'invention, un quatorzième groupe de composés est constitué par les composés pour lesquels :
- l'amine cyclique formée par -N-A-L-B- représente un groupe pyrrolidine ou pipéridine, substitué par un groupe pyrrolidine ;
   R₂, R₇ et R₈ étant tels que définis ci-dessus.

Parmi les composés de formule générale (I) objets de l'invention, un quinzième groupe de composés est constitué par les composés pour lesquels :
- l'amine cyclique formée par -N-A-L-B- représente un groupe (*R*,*S*)-3-[pyrrolidin-1-yl]-pyrrolidin-1-yle ou 4-(pyrrolidin-1-yl)-pipéridin-1-yle ;
   R₂, R₇ et R₈ étant tels que définis ci-dessus.

Parmi les composés de formule générale (I) objets de l'invention, un seizième groupe de composés est constitué par les composés pour lesquels :
- R₂ représente un groupe phényle, 3-fluoro-phényle, 4-fluoro-phényle, 3,4-difluoro-phényle, 3,5-difluoro-phényle, 3,5-dichloro-phényle, 3.5-diméthyl-phényle, 3,5-di(trifluorométhyl)-phényle ;
- l'amine cyclique formée par -N-A-L-B- représente un groupe pipérazin-1-yle, 3-(*R*)-méthyl-pipérazin-1-yle, 3,3-diméthyl-pipérazin-1-yle, *cis*-3,5-diméthyl-pipérazin-1-yle, 4-méthyl-pipérazin-1-yle, 4-(2-hydroxy-éthyl)-pipérazin-1-yle, 4-(2-fluoroéthyl)-pipérazin-1-yle, 4-isopropyl-pipérazin-1-yle, 4-cyclopropyl-pipérazin-1-yle, 4-benzyl-pipérazin-1-yle, 4-(2-hydroxy-2-méthylpropyl)-pipérazin-1-yle, 4-(3-hydroxy-3-méthylbutyl)-pipérazin-1-yle, (*S*)-octahydro-pyrrolo[1,2-*a*]pyrazin-2-yle, (1*S*,4*S*)-2,5-diazabicyclo[2.2.1]hept-2-yle, 5-(2-hydroxy-éthyl)-(1*S*,4*S*)-2,5-diaza-bicyclo[2.2.1]hept-2-yle, 5-isopropyl-(1*S*,4*S*)-2,5-diazabicyclo[2.2.1]hept-2-yle, hexahydro-pyrrolo[3,4-*c*]pyrrol-2(1*H*)-yle, 5-méthyl-hexahydro-pyrrolo[3,4-*c*]pyrrol-2(1*H*)-yle, 5-(2-isopropyl)-hexahydropyrrolo[3,4-*c*]pyrrol-2(1*H*)-yle, 5-(2-hydroxy-éthyl)-hexahydropyrrolo[3,4-*c*]pyrrol-2(1*H*)-yle, 5-(benzyl)-hexahydro-pyrrolo[3,4-*c*]pyrrol-2(1*H*)-yle, 3-(hydroxyméthyl)-pipérazin-1-yle, 3-fluorométhyl-pipérazin-1-yle ;
- R₇ et R₈ représentent, indépendamment l'un de l'autre, un atome d'hydrogène ou un groupe un groupe méthyle.

Parmi les composés de formule générale (I) objets de l'invention, un dix-septième groupe de composés est constitué par les composés pour lesquels :
- R₂ représente un groupe phényle, 3-fluoro-phényle, 4-fluoro-phényle, 3,4-difluoro-phényle, 3,5-difluoro-phényle, 3,5-dichloro-phényle, 3,5-diméthyl-phényle, 3,5-di(trifluorométhyl)-phényle ;
- l'amine cyclique formée par -N-A-L-B- représente un groupe pipérazin-1-yle, 3-(*R*)-méthyl-pipérazin-1-yle, 3,3-diméthyl-pipérazin-1-yle, *cis*-3,5-diméthyl-pipérazin-1-yle, 4-méthyl-pipérazin-1-yle, 4-(2-hydroxy-éthyl)-pipérazin-1-yle, 4-(2-fluoroéthyl)-pipérazin-1-yle, 4-isopropyl-pipérazin-1-yle, 4-cyclopropyl-pipérazin-1-yle, 4-benzyl-pipérazin-1-yle, 4-(2-hydroxy-2-méthylpropyl)-pipérazin-1-yle, 4-(3-hydroxy-3-méthylbutyl)-pipérazin-1-yle, (*S*)-octahydro-pyrrolo[1,2-a]pyrazin-2-yle, (1*S*,4*S*)-2,5-diazabicyclo[2.2.1]hept-2-yle, 5-(2-hydroxy-éthyl)-(1*S*,4*S*)-2,5-diaza-bicyclo[2.2.1]hept-2-yle, 5-isopropyl-(1*S*,4*S*)-2,5-diazabicyclo[2.2.1]hept-2-yle, hexahydro-pyrrolo[3,4-*c*]pyrrol-2(1*H*)-yle, 5-méthyl-hexahydro-pyrrolo[3,4-*c*]pyrrol-2(1*H*)-yle, 5-(2-isopropyl)-hexahydropyrrolo[3,4-*c*]pyrrol-2(1*H*)-yle, 5-(2-hydroxy-éthyl)-hexahydropyrrolo[3,4-*c*]pyrrol-2(1*H*)-yle, 5-(benzyl)-hexahydro-pyrrolo[3,4-*c*]pyrrol-2(1*H*)-yle ;
- R₇ et R₈ représentent, indépendamment l'un de l'autre, un atome d'hydrogène ou un groupe un groupe méthyle.

Parmi les composés de formule générale (I) objets de l'invention, un dix-huitième groupe de composés est constitué par les composés pour lesquels :
- R₂ représente un groupe 4-fluoro-phényle ;
- l'amine cyclique formée par -N-A-L-B- représente un groupe 2,9-diaza-spiro[5.5]undéc-3-yle, 1-oxa-4,9-diazaspiro[5.5]undéc-9-yle ;
- R₇ et R₈ représentent un atome d'hydrogène.

Parmi les composés de formule générale (I) objets de l'invention, un dix-neuvième groupe de composés est constitué par les composés pour lesquels :
- R₂ représente un groupe 4-fluoro-phényle, 3,5-difluoro-phényle, 3,5-dichloro-phényle, 3,5-diméthyl-phényle, 3,5-di(trifluorométhyl)-phényle ;
- l'amine cyclique formée par -N-A-L-B- représente un groupe (*R*,*S*)-3-[pyrrolidin-1-yl]-pyrrolidin-1-yle ou 4-(pyrrolidin-1-yl)-pipéridin-1-yle ;
- R₇ et R₈ représentent un atome d'hydrogène.

Parmi les composés de formule générale (I) objets de l'invention, on peut notamment citer les composés suivants :
2-(4-Fluoro-phényl)-6-pipérazin-1-yl-3-pyridazin-4-yl-imidazo[1,2-*b*]pyridazine;
2-(4-Fluoro-phényl)-6-((*R*)-3-méthyl-pipérazin-1-yl)-3-pyridazin-4-yl-imidazo[1,2-*b*]pyridazine ;
6-(3,3-Diméthyl-pipérazin-1-yl)-2-(4-fluoro-phényl)-3-pyridazin-4-yl-imidazo[1,2-*b*]pyridazine ;
6-((*cis*)-3,5-Diméthyl-pipérazin-1-yl)-2-(4-fluoro-phényl)-3-pyridazin-4-yl-imidazo[1,2-*b*]pyridazine ;
2-(4-Fluoro-phényl)-6-(4-méthyl-pipérazin-1-yl)-3-pyridazin-4-yl-imidazo[1,2-*b*]pyridazine
2-{4-[2-(4-Fluoro-phényl)-3-pyridazin-4-yl-imidazo[1,2-*b*]pyridazin-6-yl]-pipérazin-1-yl}-éthanol ;
6-[4-(2-Fluoro-éthyl)-pipérazin-1-yl]-2-phényl-3-pyridazin-4-yl-imidazo[1,2-*b*]pyridazine ;
6-[4-(2-Fluoro-éthyl)-pipérazin-1-yl]-2-(4-fluoro-phényl)-3-pyridazin-4-yl-imidazo[1,2-*b*]pyridazine ;
6-(4-Isopropyl-pipérazin-1-yl)-2-phényl-3-pyridazin-4-yl-imidazo[1,2-*b*]pyridazine;
2-(3-Fluoro-phényl)-6-(4-isopropyl-pipérazin-1-yl)-3-pyridazin-4-yl-imidazo[1,2-*b*]pyridazine ;
2-(4-Fluoro-phényl)-6-(4-isopropyl-pipérazin-1-yl)-3-pyridazin-4-yl-imidazo[1,2-*b*]pyridazine ;
2-(4-Fluoro-phényl)-6-(4-isopropyl-pipérazin-1-yl)-7,8-diméthyl-3-pyridazin-4-yl-imidazo[1,2-*b*]pyridazine ;
2-(3,5-Diméthyl-phényl)-6-(4-isopropyl-pipérazin-1-yl)-3-pyridazin-4-yl-imidazo[1,2-*b*]pyridazine ;
2-(3,4-Difluoro-phényl)-6-(4-isopropyl-pipérazin-1-yl)-3-pyridazin-4-yl-imidazo[1,2-*b*]pyridazine ;
2-(3,5-Difluoro-phényl)-6-(4-isopropyl-pipérazin-1-yl)-3-pyridazin-4-yl-imidazo[1,2-*b*]pyridazine ;
2-(3,5-Dichloro-phényl)-6-(4-isopropyl-pipérazin-1-yl)-3-pyridazin-4-yl-imidazo[1,2-*b]*pyridazine ;
2-(3,5-Di(trifluorométhyl)-phényl)-6-(4-isopropyl-pipérazin-1-yl)-3-pyridazin-4-yl-imidazo[1,2-*b*]pyridazine ;
6-(4-Cyclopropyl-pipérazin-1-yl)-2-(4-fluoro-phényl)-3-pyridazin-4-yl-imidazo[1,2-*b*]pyridazine ;
6-(4-Benzyl-pipérazin-1-yl)-2-(4-fluoro-phényl)-3-pyridazin-4-yl-imidazo[1,2-*b*]pyridazine ;
1-{4-[2-(4-Fluoro-phényl)-3-pyridazin-4-yl-imidazo[1,2-*b*]pyridazin-6-yl]-pipérazin-1-yl}-2-méthyl-propan-2-ol ;
4-{4-[2-(4-Fluoro-phényl)-3-pyridazin-4-yl-imidazo[1,2-*b*]pyridazin-6-yl]-pipérazin-1-yl}-2-méthyl-butan-2-ol ;
2-(4-Fluoro-phényl)-6-(*S*)-hexahydro-pyrrolo[1,2-a]pyrazin-2-yl-3-pyridazin-4-yl-imidazo[1,2-*b*]pyridazine ;
6-((1*S*,4*S*)-2,5-Diaza-bicyclo[2.2.1]hept-2-yl)-2-(4-fluoro-phényl)-3-pyridazin-4-yl-imidazo[1,2-*b*]pyridazine ;
2-{5-[2-(4-Fluoro-phényl)-3-pyridazin-4-yl-imidazo[1,2-*b*]pyridazin-6-yl]-(1*S*,4*S*)-2,5-diaza-bicyclo[2.2.1]hept-2-yl}-éthanol ;
2-(4-Fluoro-phényl)-6-(5-isopropyl-(1*S*,4*S*)-2,5-diaza-bicyclo[2.2.1]hept-2-yl)-3-pyridazin-4-yl-imidazo[1,2-*b*]pyridazine ;
2-(4-Fluoro-phényl)-6-hexahydro-pyrrolo[3,4-*c*]pyrrol-2(1*H*)-yl-3-pyridazin-4-yl-imidazo[1,2-*b*]pyridazine ;
2-(4-Fluoro-phényl)-6-(5-méthyl-hexahydro-pyrrolo[3,4-*c*]pyrrol-2(1*H*)-yl)-3-pyridazin-4-yl-imidazo[1,2-*b*]pyridazine ;
2-(4-Fluoro-phényl)-6-(5-isopropyl-hexahydro-pyrrolo[3,4-*c*]pyrrol-2(1*H*)-yl)-3-pyridazin-4-y)limidazo[1,2-*b*]pyridazine :
2-{(5-[2-(4-Fluoro-phényl)-3-pyridazin-4-yl-imidazo[1,2-*b*]pyridazin-6-yl]-hexahydro-pyrrolo[3,4-*c*]pyrrol-2(1*H*)-yl}-éthanol ;
2-(4-Fluoro-phényl)-6-(5-benzyl-hexahydro-pyrrolo[3,4-*c*]pyrrol-2(1*H*)-yl)-3-pyridazin-4-yl-imidazo[1,2-*b*]pyridazine ;
9-[2-(4-Fluoro-phényl)-3-pyridazin-4-yl-imidazo[1,2-*b*]pyridazin-6-yl]-2,9-diaza-spiro[5.5]undécane ;
9-[2-(4-Fluoro-phényl)-3-pyridazin-4-yl-imidazo[1,2-*b*]pyridazin-6-yl]-1-oxa-4,9-diaza-spiro[5.5]undécane ;
6-[1,3']Bipyrrolidinyl-1'-yl-2-(4-fluoro-phényl)-3-pyridazin-4-yl-imidazo[1,2-*b*]pyridazine ;
2-(4-Fluoro-phényl)-3-pyridazin-4-yl-6-(4-pyrrolidin-1-yl-pipéridin-1-yl)-imidazo[1,2-*b*]pyridazine ;
2-(3,5-Diméthyl-phényl)-3-pyridazin-4-yl-6-(4-pyrrolidin-1-yl-pipéridin-1-yl)-imidazo[1,2-*b*]pyridazine ;
2-(3,5-Difluoro-phényl)-3-pyridazin-4-yl-6-(4-pyrrolidin-1-yl-pipéridin-1-yl)-imidazo[1,2-*b*]pyridazine ;
2-(3,5-Dichloro-phényl)-3-pyridazin-4-yl-6-(4-pyrrolidin-1-yl-pipéridin-1-yl)-imidazo[1,2-*b*]pyridazine ;
2-(3,5-Di(trifluorométhyl)-phényl)-3-pyridazin-4-yl-6-(4-pyrrolidin-1-yl-pipéridin-1-yl)-imidazo[1,2-*b*]pyridazine ;
(+/)-{4-[2-(4-Fluoro-phényl)-3-pyridazin-4-yl-imidazo[1,2-*b*]pyridazin-6-yl]-pipérazin-2-yl}-méthanol ;
(+/-)-6-(3-Fluorométhyl-pipérazin-1-yl)-2-(4-fluoro-phényl)-3-pyridazin-4-yl-imidazo[1,2-*b*]pyridazine ;
6-(5-Méthyl-hexahydro-pyrrolo[3,4-*c*]pyrrol-2(1*H*)-yl)-2-phényl-3-pyridazin-4-yl-imidazo[1,2-*b*]pyridazine ;
2-(3-Fluoro-phényl)-6-(5-méthyl-hexahydro-pyrrolo[3,4-*c*]pyrrol-2(1*H*)-yl)-3-pyridazin-4-yl-imidazo[1,2-*b*]pyridazine ;
2-(4-Fluoro-phényl)-7-méthyl-6-(5-méthyl-hexahydro-pyrrolo[3,4-*c*]pyrrol-2(1*H*)-yl)-3-pyridazin-4-yl-imidazo[1,2-*b*]pyridazine ;
2-(4-Fluoro-phényl)-8-méthyl-6-(5-méthyl-hexahydro-pyrrolo[3,4-*c*]pyrrol-2(1*H*)-yl)-3-pyridazin-4-yl-imidazo[1,2-*b*]pyridazine ;
2-(3,4-Difluoro-phényl)-6-(5-méthyl-hexahydro-pyrrolo[3,4-*c*]pyrrol-2(1*H*)-yl)-3-pyridazin-4-yl-imidazo[1,2-*b*]pyridazine.

L'invention a également pour objet un procédé de préparation des composés de l'invention de formule (I).

Conformément à l'invention, on peut préparer les composés de formule générale (I) selon le procédé général décrit dans le schéma 1 ci-après.

De manière générale et comme illustré dans le schéma 1, les dérivés de 6-amino-3-(pyridazin-4-yl)imidazo[1,2-*b*]pyridazine de formule générale (I) dans laquelle R₂, A, L, B, R₇ et R₈ sont tels que définis ci-dessus peuvent être préparés à partir d'un dérivé de (pyridazin-4-yl)imidazo[1,2-*b*]pyridazine de formule générale (IIa), dans laquelle R₂, R₇ et R₈ sont tels que définis ci-dessus et X₆ représente un groupe partant tel qu'un halogène par traitement au moyen d'une amine de formule générale (IIb) dans laquelle A, L et B sont tel que définis précédemment. Cette réaction peut être effectuée par chauffage des réactifs dans un solvant polaire tel que le pentanol ou le diméthylsulfoxyde.

Les dérivés de 6-amino-3-(pyridazin-4-yl)imidazo[1,2-*b*]pyridazine de formule générale (I), dans laquelle R₂, A, L, B, R₇ et R₈ sont tels que définis ci-dessus peuvent également être préparés par condensation entre un dérivé de pyridazin-3-ylamine de formule générale (IIIa) dans laquelle A, L, B, R₇ et R₈ sont tels que définis ci-dessus et un dérivé de 2-bromo-2-(pyridazin-4-yl)-éthan-1-one de formule générale (IIIb) dans laquelle R₂ est tel que défini ci-dessus. Cette réaction peut être effectuée par chauffage des réactifs dans un solvant polaire tel que les alcools aliphatiques, par exemple l'éthanol ou le butanol.

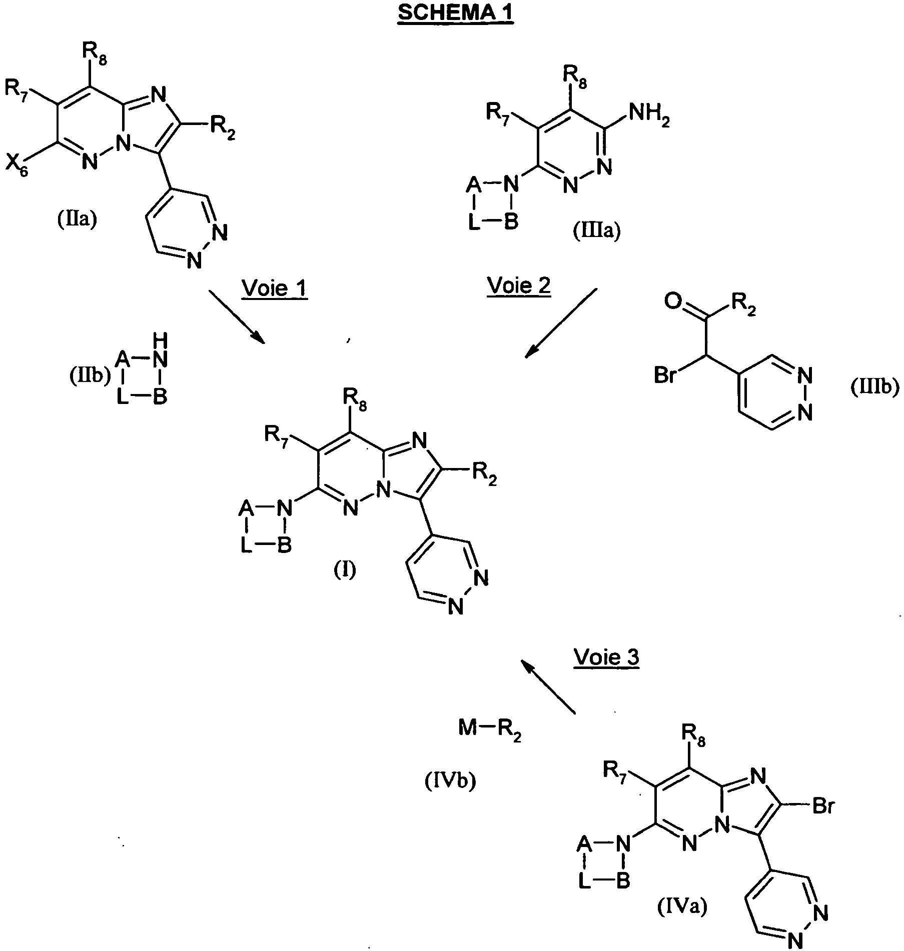

Les dérivés de 6-amino-3-(pyridazin-4-yl)imidazo[1,2-*b*]pyridazine de formule générale (I), dans laquelle R₂, A, L, B, R₇ et R₈ sont tels que définis ci-dessus peuvent alternativement être préparés par couplage métallocatalysé entre un dérivé de 2-bromo-3-(pyridazin-4-yl)imidazo[1,2-*b*]pyridazine de formule générale (IVa) dans laquelle A, L, B, R₇ et R₈ sont tels que définis ci-dessus et un dérivé d'aryle de formule générale (IVb) dans laquelle R₂ est tel que défini ci-dessus et M représente un groupe trialkylstannyle, le plus fréquemment un groupement tributylstannyle ou un groupe dihydroxyboryle ou dialkyloxyboryle, le plus fréquemment un groupe 4,4,5,5-tétraméthyl-1,3,3,2-dioxaborolan-2-yle selon les conditions de Stille ou de Suzuki.
Les couplages selon la méthode de Stille sont par exemple réalisés par chauffage en présence d'un catalyseur tel que le tétrakis(triphénylphosphine)palladium, l'iodure de cuivre, dans un solvant tel que le N,N-diméthylacétamide.
Les couplages selon la méthode de Suzuki sont par exemple réalisés par chauffage en présence d'un catalyseur tel que le 1,1'-bis (diphénylphosphino)ferrocènedichloropalladium, d'une base minérale telle que le carbonate de césium, dans un mélange de solvant tels que le dioxane et l'eau.

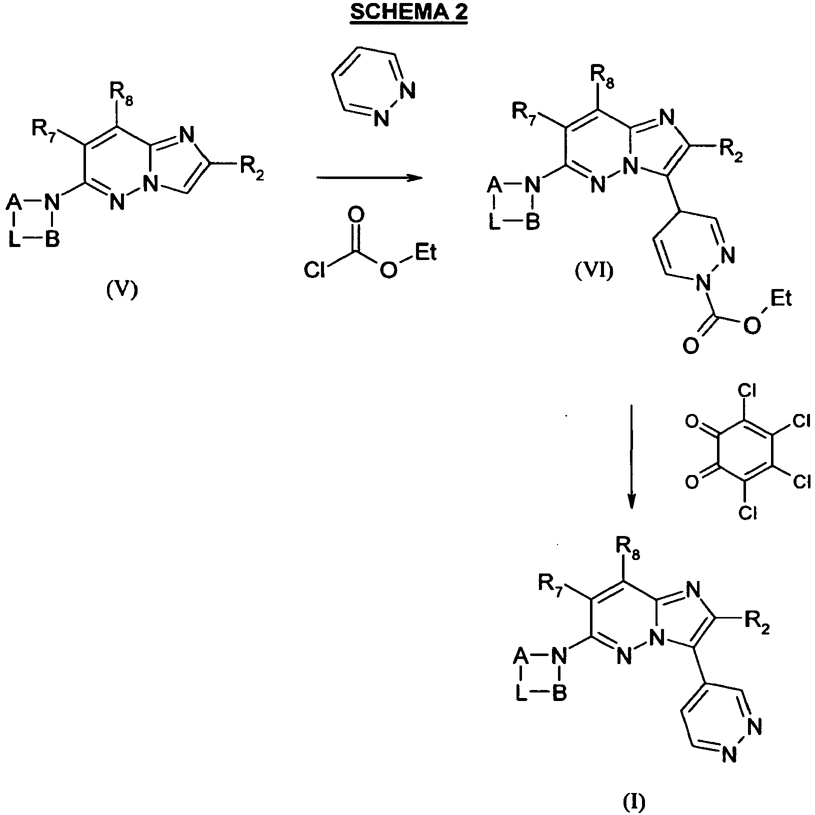

Enfin, les dérivés de 6-amino-3-(pyridazin-4-yl)imidazo[1,2-*b*]pyridazine de formule générale (I) peuvent être préparés en deux étapes à partir d'un dérivé de 6-amino-imidazo[1,2-*b*]pyridazine de formule générale (V) dans laquelle R₂, A, L, B, R₇ et R₈ sont tels que définis ci-dessus.
Ainsi, la réaction d'un dérivé de 6-amino-imidazo[1,2-*b*]pyridazine de formule générale (V) sur de la pyridazine et un chloroformiate d'alkyle, par exemple le chloroformiate d'éthyle, conduit au dérivé de formule générale (VI) dans laquelle R₂, A, L, B, R₇ et R₈ sont tels que définis ci-dessus. Le dérivé de formule générale (VI) est ensuite oxydé au moyen d'ortho-chloranile dans un solvant tel que le toluène pour conduire aux dérivés de 6-amino-(pyridazin-4-yl)imidazo[1,2-*b*]pyridazine de formule générale (I).

Dans certains cas, les dérivés de 6-amino-3-(pyridazin-4-yl)imidazo[1,2-*b*]pyridazine de formule générale (I) pour lequels l'amine formée par N, L, A et B comporte une seconde amine secondaire ou tertiaire peuvent être préparés respectivement à partir de l'amine primaire ou secondaire correspondante par alkylation ou amino-réduction selon des méthodes usuelles pour l'homme du métier.

### Synthèse des précurseurs

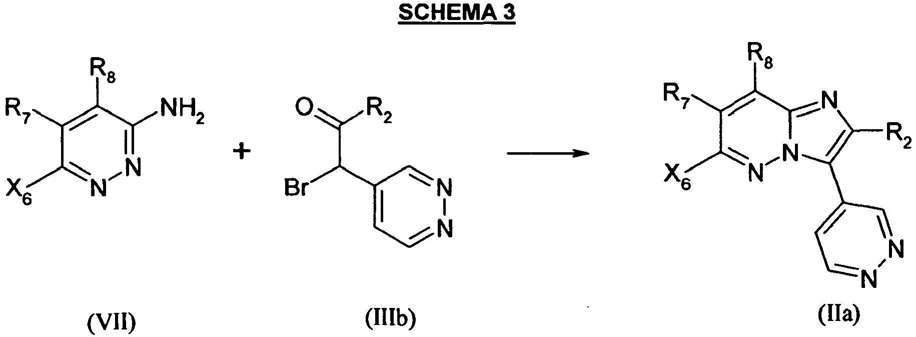

Les dérivés de 3-(pyridazin-4-yl)imidazo[1,2-*b*]pyridazine de formule générale (IIa), dans laquelle R₂, R₇, R₈ et X₆ sont tels que définis ci-dessus peuvent être préparés par condensation entre un dérivé de pyridazin-3-ylamine de formule générale (VII) dans laquelle R₇ et R₈ sont tels que définis ci-dessus et X₆ représente un groupe partant et un dérivé de 2-bromo-2-(pyridazin-4-yl)-éthan-1-one de formule générale (IIIb) dans laquelle R₂ est tel que défini ci-dessus.
La réaction peut être effectuée par chauffage des réactifs dans un solvant polaire tel que les alcools aliphatiques, par exemple l'éthanol ou le butanol.

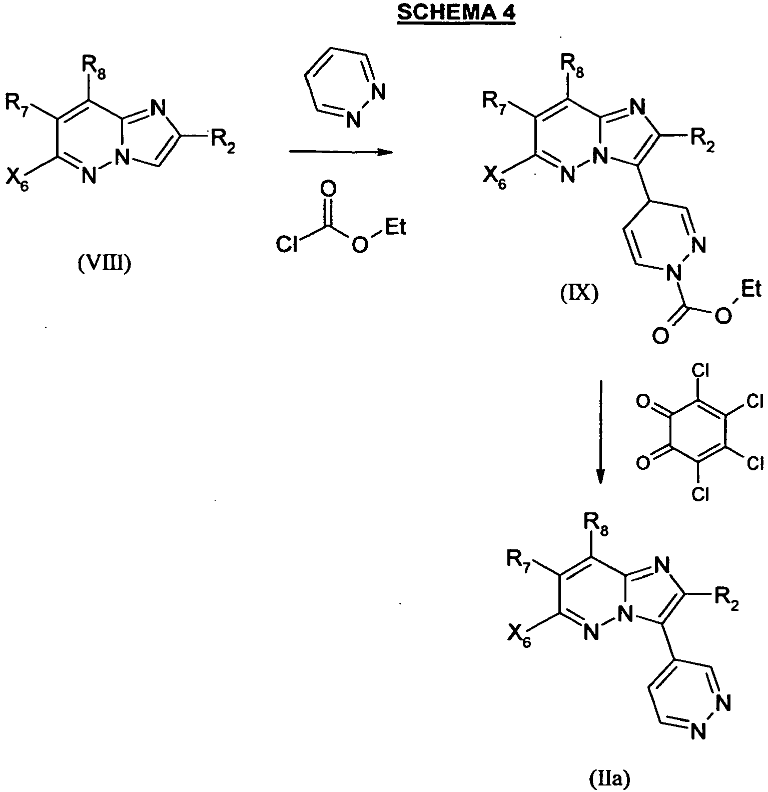

Les dérivés de 3-(pyridazin-4-yl)imidazo[1,2-*b*]pyridazine de formule générale (IIa), dans laquelle R₂, R₇, R₈ et X₆ sont tels que définis ci-dessus peuvent également être préparés en deux étapes à partir d'un dérivé d'imidazo[1,2-*b*]pyridazine de formule générale (VIII) dans laquelle X₆, R₂, R₇ et R₈ sont tels que définis ci-dessus.
Ainsi, la réaction d'un dérivé d'imidazo[1,2-*b*]pyridazine de formule générale (VIII) sur de la pyridazine et un chloroformiate d'alkyle, par exemple le chloroformiate d'éthyle, conduit au dérivé de formule générale (IX) dans laquelle X₆, R₂, R₇ et R₈ sont tels que définis ci-dessus. Le dérivé de formule générale (IX) est ensuite oxydé au moyen d'ortho-chloranile dans un solvant tel que le toluène, pour conduire aux dérivés de 3-(pyridazin-4-yl)imidazo[1,2-*b*]pyridazine de formule générale (IIa).

Les préparations de dérivés de pyridazine de formule générale (IIIa) dans laquelle A, L, B, R₇ et R₈ sont tels que définis ci-dessus ou de dérivés de 6-amino-imidazo[1,2-*b*]pyridazine de formule générale (V) peuvent être réalisées selon les méthodes décrites dans les documents DE-2737542 et DE-3542661 ou bien selon des méthodes analogues bien connues de l'homme du Métier.

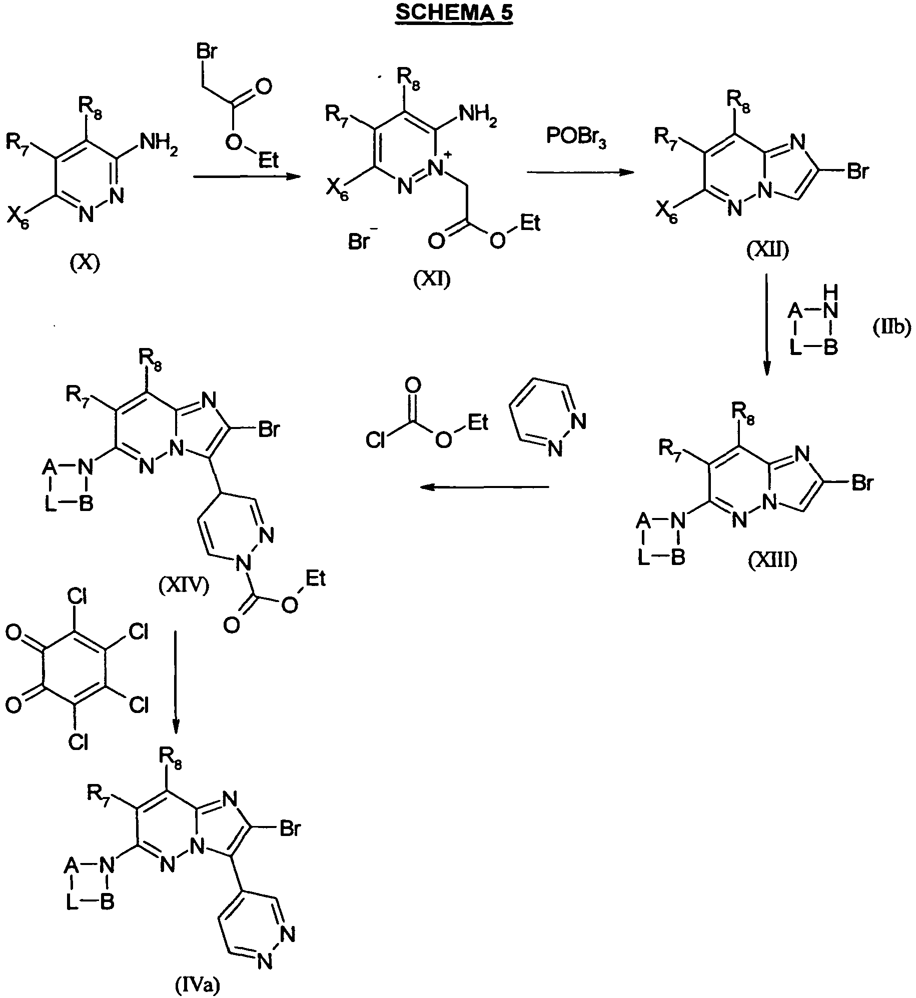

Les dérivés de 2-bromo-3-(pyridazin-4-yl)imidazo[1,2-*b*]pyridazine de formule générale (IVa), dans laquelle R₇, R₈ et A, L et B sont tels que définis ci-dessus peuvent être préparés en cinq étapes à partir d'un dérivé de 3-amino-6-halogénopyridazine de formule générale (X) dans laquelle X₆, R₇ et R₈ sont tels que définis ci-dessus.
Ainsi, l'alkylation d'un dérivé de 3-amino-6-halogénopyridazine de formule générale (X) au moyen d'un 2-bromoacétate d'alkyle tel que le 2-bromoacétate d'éthyle par chauffage dans un solvant polaire tel que l'éthanol conduit au bromhydrate de formule (XI) dans lequel X₆, R₇ et R₈ sont tels que définis ci-dessus. Ce dernier est cyclisé par chauffage en présence d'oxybromure de phosphore dans un solvant aprotique tel que le toluène, pour conduire à un dérivé de 6-halogéno-2-bromo-imidazo[1,2-*b*]pyridazine de formule générale (XII) dans lequel X₆, R₇ et R₈ sont tels que définis ci-dessus.
Celui-ci est ensuite traité par chauffage avec une amine de formule générale (IIb) dans laquelle A, L et B sont tels que définis précédemment, pour conduire à une 6-amino-2-bromo-imidazo[1,2-*b*]pyridazine de structure générale (XIII) où A, L, B, R₇ et R₈ sont tels que définis précédemment.
Ce dérivé est traité avec de la pyridazine et un chloroformiate d'alkyle, par exemple le chloroformiate d'éthyle, pour conduire au dérivé de formule générale (XIV) dans laquelle A, L, B, R₇ et R₈ sont tels que définis ci-dessus. Enfin, le dérivé de formule générale (XIV) est oxydé au moyen d'ortho-chloranile dans un solvant tel que le toluène, pour conduire aux dérivés de 6-amino-(pyridazin-4-yl)imidazo[1,2-*b*]pyridazine de formule générale (IVa).

Dans ce qui précède, on entend par groupe partant un groupe pouvant être facilement clivé d'une molécule par rupture d'une liaison hétérolytique, avec départ d'une paire électronique. Ce groupe peut, par exemple, être ainsi remplacé facilement par un autre groupe lors d'une réaction de substitution. De tels groupes partants sont, par exemple, les halogènes ou un groupe hydroxy activé tel qu'un mésyle, tosyle, triflate, acétyle, etc. Des exemples de groupes partants ainsi que des références pour leurs préparations sont données dans « Advances in Organic Chemistry », J. March, 3rd Edition, Wiley Interscience, p. 310-316.

### Groupes protecteurs

Pour les composés de formule générale (I) ou (IIIa) telles que définies ci-dessus et dans le cas ou le groupe N-A-L-B comporte une fonction amine primaire ou secondaire, cette fonction peut éventuellement être protégée lors de la synthèse par un groupe protecteur, par exemple un benzyle ou un t-butyloxycarbonyle.

Les produits de structure générale (I) tels que définis ci-dessus sont obtenus selon les procédés décrits après une étape finale supplémentaire de déprotection du groupe protecteur selon les conditions usuelles connues de l'homme du métier.

Les exemples suivants décrivent la préparation de certains composés conformes à l'invention. Ces exemples ne sont pas limitatifs et ne font qu'illustrer l'invention. Les numéros des composés exemplifiés renvoient à ceux donnés dans le tableau 1, ci-après, qui illustrent les structures chimiques et les propriétés physiques respectivement de quelques composés selon l'invention.

### Exemple 1 (composé n° 30) : 6-(5-Benzyl-hexahydro-pyrrolo[3,4-c]pyrrol-2(1H)-yl)-2-(4-fluoro-phényl)-3-pyridazln-4-yl-imidazo[1,2-b]pyridazine

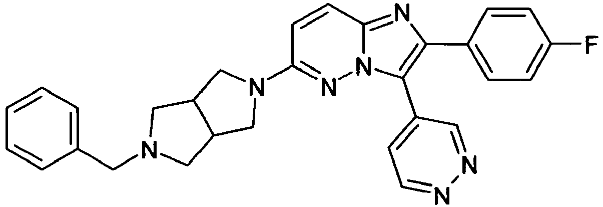

### Etape 1.1. 4-[6-Chloro-2-(4-fluoro-phényl)-imidazo[1,2-b]pyridazin-3-yl]-4H-pyridazine-1-carboxylate d'éthyle

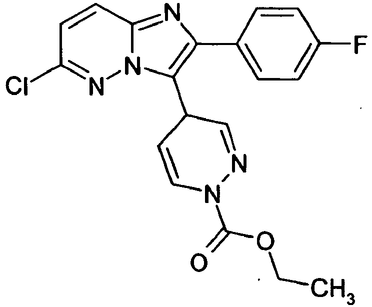

A une solution de 3,00 g (12,1 mmoles) de 6-chloro-2-(4-fluoro-phényl)-imidazo[1,2-*b*]pyridazine (CAS : 244081-70-7) et de 4,40 ml (60,6 mmoles) de pyridazine dans 40 ml de dichlorométhane à 10°C, on additionne goutte à goutte en 30 minutes 2,90 ml (30,3 mmoles) de chloroformiate d'éthyle. Le milieu est agité à 10°C pendant encore une heure puis laissé sous agitation pendant 3 heures. Il est ensuite versé sur de l'eau glacée et le produit est extrait avec de l'acétate d'éthyle.
La phase organique est séchée sur sulfate de sodium et le solvant est concentré sous pression réduite. Le produit est chromatographié sur colonne de gel de silice en éluant avec un mélange d'ammoniaque, de méthanol et de dichlorométhane (0,2/2/98) pour conduire à 2,3 g de mousse orangée contenant le produit avec une pureté suffisante pour être engagée dans l'étape suivante.
RMN ¹H (CDCl₃) δ : 7,9 (m, 1H) ; 7,5-7,6 (m, 2H) ; 7,0-7,2 (m, 5H) ; 6,75 (m, 1H) ; 5,9 (2m, 1H) ; 5,0-4,8 (2m, environ 2H) ; 4,35 (m, >2H) ; 1,35 (m, >3H) ppm.

### Etape 1.2. 6-Chloro-2-(4-fluoro-phényl)-3-(pyridazin-4-yl)-imidazo[1,2-b]pyridazine

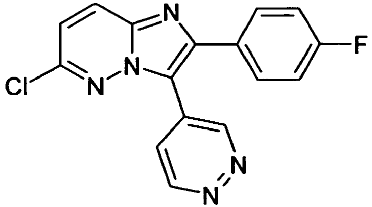

2,30 g (5,75 mmoles) du produit obtenu à l'étape précédente sont dissous dans 30 ml de toluène et on ajoute 1,66 g (6,33 mmoles) d'ortho-chloranile en solution dans le toluène. Le milieu réactionnel est agité 30 minutes à température ambiante puis versé sur une solution aqueuse de soude 2N. Le produit est extrait avec de l'acétate d'éthyle.
La phase organique est séchée sur sulfate de sodium puis concentrée sous pression réduite. Le produit est chromatographié sur colonne de gel de silice en éluant avec un mélange d'ammoniaque, de méthanol et de dichlorométhane (0,2/2/98) pour conduire à 1,0 g de solide blanc.
RMN ¹H (CDCl₃) δ : 9,35 (d, 1H) ; 9,25 (d, 1H) ; 7,95 (d, 1H) ; 7,85 (d, 1H) ; 7,55 (m, 2H) ; 7,0-7,2 (m, 3H) ppm.

### Etape 1.3. 6-(5-Benzyl-hexahydro-pyrrolo[3,4-c]pyrrol-2(1H)-yl)-2-(4-fluoro-phényl)-3-pyridazin-4-yl-imidazo[1,2-b]pyridazine

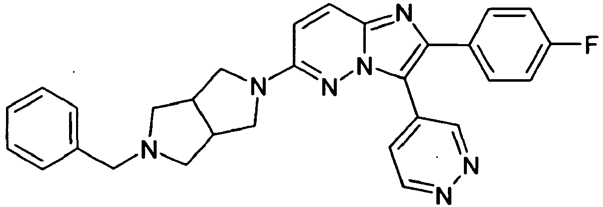

Le mélange de 0,90 g (2,8 mmoles) de 6-chloro-2-(4-fluoro-phényl)-3-(pyridazin-4-yl)-imidazo[1,2-*b*]pyridazine et 1,7 g (8,3 mmoles) de 5-benzyl-octahydro-pyrrolo[3,4-*c*]pyrrole dans 15 ml de pentanol est chauffé dans un tube scellé à 150°C pendant 18 heures.
Le milieu réactionnel est versé sur une solution aqueuse d'acide chlorhydrique 1N et la phase aqueuse est lavée avec de l'acétate d'éthyle. La phase aqueuse est ensuite basifiée au moyen de soude aqueuse et le produit est extrait avec du dichlorométhane. La phase organique est séchée sur sulfate de sodium puis concentrée sous pression réduite. Le produit est chromatographié sur colonne de gel de silice en éluant avec un mélange d'ammoniaque, de méthanol et de dichlorométhane (0,2/2/98) pour conduire à 1,2 g de solide sous forme de meringue blanche amorphe.
RMN ¹H (CDCl₃) δ : 9,60 (d, 1H) ; 9,10 (d, 1H) ; 7,75 (m, 1H) ; 7,70 (d, 1H) ; 7,5 (m, 2H) ; 7,2 (m, 5H) ; 6,80 (d, 1 H) ; 3,6-3,7 (m, 2H) ; 3,50 (s, 2H) ; 3,3 (m, 2H) ; 2,9 (m, 2H) ; 2,4-2,7 (d, 1 H) ppm.

### Exemple 2 (composé n° 26) : 2-(4-Fluoro-phényl)-6-(hexahydro-pyrrolo[3,4-c]pyrrol-2(1H)-yl)-3-(pyridazin-4-yl)-imidazo[1,2-b]pyridazine

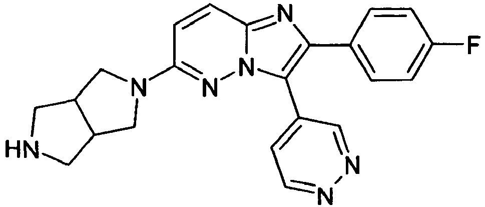

Le mélange de 1,2 g (2,4 mmoles) de 6-(5-benzyl-hexahydro-pyrrolo [3,4-*c*]pyrrol-2(1*H*)-yl)-2-(4-fluoro-phényl)-3-(pyridazin-4-yl)-imidazo[1,2-*b*]pyridazine et 2,3 g (36 mmoles) de formiate d'ammonium dans 40 ml de méthanol sont chauffés à reflux pendant 2 heures en présence de Palladium sur charbon (10%) contenant 50% d'humidité.
Le milieu réactionnel est concentré sous pression réduite, le résidu repris avec du dichlorométhane et lavé avec de l'eau. La phase organique est séchée sur sulfate de sodium puis concentrée sous pression réduite. Le produit est chromatographié sur colonne de gel de silice en éluant avec un mélange de méthanol et de dichlorométhane (10/90) pour conduire à 0,60 g de solide blanc.
PF : 130-135°C
RMN ¹H (CDCl₃) δ : 9,75 (d, 1H) ; 9,20 (d, 1H) ; 7,8 (m, 2H) ; 7,60 (m, 2H) ; 7,5 (m, 2H) ; 7,1 (m, 2H) ; 6,80 (d, 1H) ; 3,7 (m, 2H) ; 3,40 (dd, 2H) ; 3,2 (m, 2H) ; 2,8-3,1 (m, 4H) ; 2,7(signal large) ppm.

### Exemple 3 (Composé n° 12) : 2-(4-Fluoro-phényl)-6-(4-isopropyl-pipérazin-1-yl)-7,8-diméthyl-3-pyridazin-4-yl-imidazo[1,2-b]pyridazine

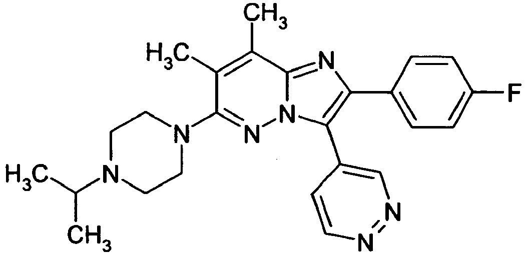

### Etape 3.1. 6-Chloro-2-(4-fluoro-phényl)-7,8-diméthyl-imidazo[1,2-b]pyridazine

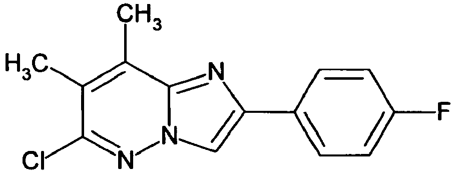

Le mélange de 13,0 g (82.5 mmoles) de 6-chloro-4,5-diméthyl-pyridazin-3-ylamine et 23,2 g (107 mmoles) de 2-bromo-1-(4-fluoro-phényl)-éthanone dans 130 ml d'éthanol est chauffé à reflux pendant 16 heures. Le solvant est évaporé sous pression réduite, le résidu repris avec du chloroforme et lavé avec une solution diluée d'ammoniaque. La phase organique est séchée sur sulfate de sodium puis concentrée sous pression réduite. Le solide brun obtenu est trituré dans de l'acétone pour conduire à 19,2 g de poudre beige après filtration et séchage.
PF: 172-174°C
RMN ¹H (CDCl₃) δ : 8,10 (s, 1H) ; 8,00 (pseudo dd, 1H) ; 7,15 (pseudo t, 1H) ; 2,70 (s, 3H) ; 2,4 (s, 3H) ppm

### Etape 3.2. 4-[6-Chloro-2-(4-fluoro-phényl)-7,8-diméthyl-imidazo[1,2-b]pyridazin-3-yl]-4H-pyridazine-1-carboxylate d'éthyle

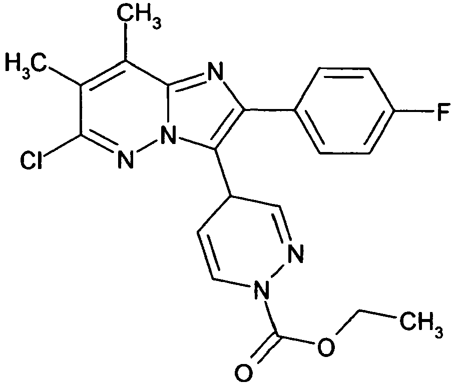

A une suspension de 9,00 g (32,6 mmoles) de 6-chloro-2-(4-fluoro-phényl)-7,8-diméthyl-imidazo[1,2-*b*]pyridazine et de 11,9 ml (163 mmoles) de pyridazine dans 160 ml de dichlorométhane à 10°C, on additionne goutte à goutte 7,8 ml (82 mmoles) de chloroformiate d'éthyle. Le milieu est agité à 10°C pendant encore une heure puis laissé sous agitation pendant 3 heures. Il est ensuite versé sur de l'eau glacée et le produit est extrait avec de l'acétate d'éthyle. Après plusieurs lavages avec de l'eau, la phase organique est séchée sur sulfate de sodium et le solvant est concentré sous pression réduite. Le résidu noir est ensuite solubilisé dans du dichlorométhane, agité en présence de noir animal pour conduire à un solide marron foncé après filtration sur Büchner et évaporation du solvant sous pression réduite. Le produit est chromatographié sur colonne d'alumine neutre en éluant avec un mélange de dichlorométhane et d'éther de pétrole (80/20) pour conduire à 11,5 g de poudre blanche après cristallisation dans l'éther diéthylique et séchage.
PF : 159-161 °C
RMN ¹H (CDCl₃) δ : 7,65 (m, 2H) ; 7,1-7,25 (m, 3H) ; 6,8 (m, 1H) ; 5,0 (m, 1H) ; 4,90 (m, 1H) ; 4,45 (q, 2H) ; 2,70 (s, 3H) ; 2,45 (s, 3H) ; 1,40 (m, 3H) ppm.

### Etape 3.3. 6-Chloro-2-(4-fluoro-phényl)-7,8-diméthyl-3-pyridazin-4-yl-imidazo[1,2-b]pyridazine

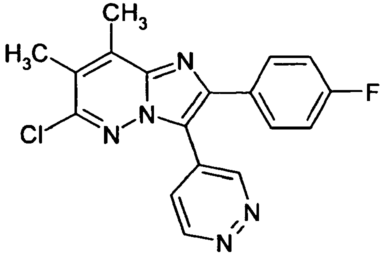

11,5 g (5,6 mmoles) de 4-[6-chloro-2-(4-fluoro-phényl)-7,8-diméthyl-imidazo[1,2-*b*]pyridazin-3-yl]-4*H*-pyridazine-1-carboxylate d'éthyle sont dissous dans 300 ml de toluène et environ 50 ml de chloroforme. On additionne par portions 7,3 g (30 mmoles) d'ortho-chloranile sous vive agitation.
Le milieu réactionnel est agité 1 heure à température ambiante puis versé sur une solution de soude 2N aqueuse. Le produit est extrait avec du dichlorométhane.
La phase organique est séchée sur sulfate de sodium puis concentrée sous pression réduite. Le solide noir obtenu est chromatographié sur colonne de gel de silice en éluant avec un mélange de d'ammoniaque, de méthanol et de dichlorométhane (0,2/2/98) pour donner 6,0 g de poudre beige.
PF : 276-278°C
RMN ¹H (CDCl₃) δ : 9,40 (d, 1H) ; 9,30 (dd, 1H) ; 7,95 (dd, 1H) ; 7,65 (m, 2H) ; 7,15 (pt, 2H) ; 2,80 (s, 3H) ; 2,50 (s, 3H) ppm.

### Etape 3.4. 2-(4-Fluoro-phényl)-6-(4-isopropyl-pipérazin-1-yl)-7,8-diméthyl-3-pyridazin-4-yl-imidazo[1,2-b]pyridazine

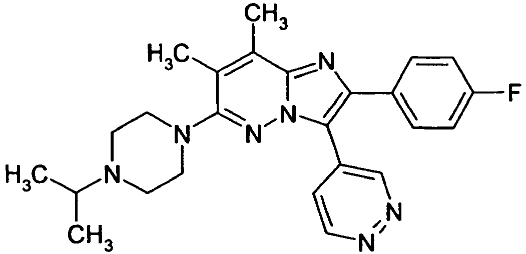

Le mélange de 0,40 g (1,1 mmoles) de 6-chloro-2-(4-fluoro-phényl)-7,8-diméthyl-3-pyridazin-4-yl-imidazo[1,2-*b*]pyridazine, de 0,16 ml (1,1 mmoles de triéthylamine) et 0,29 g (2,3 mmoles) de 1-isopropylpipérazine dans 4 ml de pentanol est chauffé au micro-ondes à 180°C (300 W) pendant 6 heures.
Le milieu réactionnel est versé sur une solution aqueuse d'acide chlorhydrique 1 N aqueuse et la phase aqueuse est lavée avec de l'acétate d'éthyle. La phase aqueuse est ensuite basifiée au moyen d'ammoniaque et le produit est extrait avec du dichlorométhane. La phase organique est séchée sur sulfate de sodium puis concentrée sous pression réduite. Le solide marron obtenu est chromatographié sur colonne de gel de silice en éluant avec un mélange d'ammoniaque, de méthanol et de dichlorométhane (0,3/3/97) pour conduire à 0,39 g de solide blanc.
PF : 222-224°C
RMN ¹H (CDCl₃) δ : 9,50 (d, 1H) ; 9,20 (d, 1H) ; 7,90 (dd, 1H) ; 7,75 (m, 1H) ; 7,5 (m, 2H) ; 7,15 (pt, 2H) ; 3,2 (m, 4H) ; 2,75 (m, 5H) ; 2,65 (s, 3H) ; 2,50 (s, 3H) ; 1,15 (d, 6H) ppm.

### Exemple 4 (composé n° 19) : 2-(4-Fluoro-phényl)-6-(4-benzylpipérazin-1-yl)-3-(pyridazin-4-yl)-imidazo[1,2-b]pyridazine

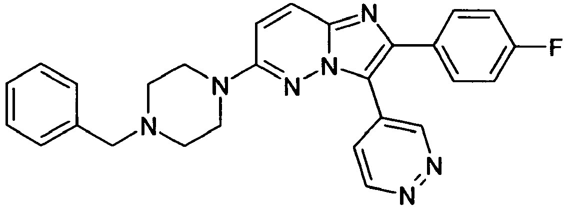

### Etape 4.1. : 6-(4-Benzyl-pipérazin-1-yl)-pyridazin-3-ylamine

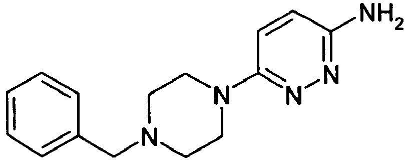

48,9 g (278 mmoles) de 1-benzylpipérazine et 12,0 g (92,6 mmoles) de 3-amino-6-chloropyridazine sont chauffés à 160°C pendant 1 heure. L'huile marron obtenue est versée dans 500 mL d'une solution de bicarbonate de sodium aqueuse et le produit est extrait avec du dichlorométhane. La phase organique est séchée puis concentrée sous pression réduite. L'huile obtenue est triturée dans l'éther diéthylique et 20,5 g de solide sont isolés après filtration et séchage.
RMN ¹H (CDCl₃) δ : 7,45-7,65 (m, 6H) ; 7,20 (s, 1H) ; 5,5 (massif large, 2H) ; 3,80 (s, 2H) ; 3,60-3,75 (m, 4H) ; 2,80-2,85 (m, 4H) ppm.

### Etape 4.2. 1-(4-Fluoro-phényl)-2-pyridazin-4-yl-éthanone

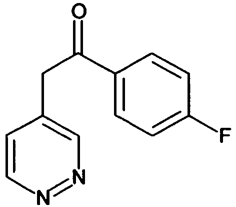

Une solution sous argon de 1,88 g (20,0 mmoles) de 4-méthylpyridazine et de 3,36 g (20,0 mmoles) de 4-fluorobenzoate d'éthyle dans 50 ml de tétrahydrofurane anhydre est refroidie à 0°C puis on additionne goutte à goutte 20,0 ml (40,0 mmoles) d'une solution 2M d'hexaméthyldisilazane de sodium dans le tétrahydrofurane (THF). Après l'addition, on laisse la température revenir à la température ambiante tandis que l'on observe une prise en masse progressive du milieu rédactionnel. Après une heure, le mélange est versé sur une solution aqueuse de chlorure d'ammonium et le produit est extrait avec de l'acétate d'éthyle. La phase organique est séchée sur sulfate de sodium, filtrée et concentrée sous pression réduite pour donner un solide rouge-orangé. Celui-ci est trituré au reflux dans 80 ml d'éther di-isopropylique contenant quelques ml d'alcool isopropylique puis le solide est isolé par filtration après refroidissement à 0°C. On isole ainsi 3,35 g de poudre jaune pâle après séchage.
RMN ¹H (CDCl₃) δ : 9,20 (m, 2H) ; 8,10 (dd, 2H) ; 7,50 (dd, 2H) ; 7,2 (d, 2H) ; 4,35 (s, 2H) ppm.

### Etape 4.3. 2-Bromo-1-(4-fluoro-phényl)-2-pyridazin-4-yl-éthanone

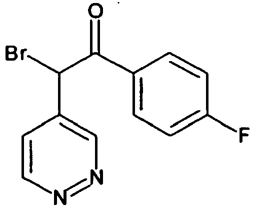

A une solution de 1,02 g (4,70 mmoles) de 1-(4-fluoro-phényl)-2-pyridazin-4-yl-éthanone dans 20 ml d'acide acétique on ajoute 0,70 g (5,2 mmoles) d'acétate de sodium, puis on additionne goutte à goutte 0,26 ml (5,2 mmoles) de brome en solution dans quelque ml d'acide acétique (légère exothermie). Après 30 minutes à température ambiante, le milieu réactionnel est refroidit en on additionne 60 ml d'eau. Le produit est extrait avec de l'éther di-éthylique. La phase organique refroidie est lavée avec une solution aqueuse d'hydrogénocarbonate de sodium, séchée sur sulfate de sodium et filtrée.
Cette solution (50 ml environ) est rapidement utilisée telle quelle dans la suite de la synthèse.

### Etape 4.4. 6-(4-Benzyl-pipérazin-1-yl)-2-(4-fluoro-phényl)-3-pyridazin-4-yl-imidazo[1,2-b]pyridazine

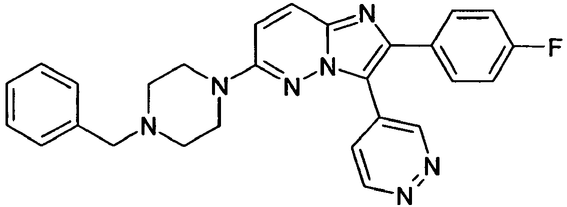

A une solution de 1,30 g (4,41 mmoles) de 6-(4-benzyl-pipérazin-1-yl)-pyridazin-3-yl-amine dans 50 ml de butanol, on additionne précautionneusement la solution éthérée de 2-bromo-1-(4-fluoro-phényl)-2-pyridazin-4-yl-éthanone obtenue précédemment (50 ml environ). L'éther diéthylique est séparé par distillation et le mélange est agité à reflux pendant plus d'une heure. Après refroidissement, le mélange est versé sur une solution saturée de bicarbonate de sodium, le produit est extrait avec de l'acétate d'éthyle et la solution concentrée sous pression réduite. L'huile brune obtenue est reprise avec une solution aqueuse d'acide chlorhydrique 3N et cette solution est lavée avec de l'éther diéthylique, basifiée au moyen d'ammoniaque. Le produit est enfin extrait avec du chloroforme, la phase organique séchée sur sulfate de sodium, filtrée et concentrée sous pression réduite. On obtient 1,6 g d'une meringue brune qui est chromatographiée sur gel de silice en éluant avec un mélange d'ammoniaque, de méthanol et de dichlorométhane (0,7/7/93) pour conduire à 0,6 g de solide jaunâtre.
PF : 198-201 °C
RMN ¹H (CDCl₃) δ : 9,55 (d, 1H) ; 9,20 (d, 1H) ; 7,85 (d, 1H) ; 7,75 (m, 1H) ; 7,60 (m, 2H) ; 7,35 (m, 5H) ; 7,1 (m, 2H) ; 7,0 (m, 1H) ; 3.6 (m, 6H) ; 3,50 (s, 2H) ; 2,6 (m, 4H) ppm.

### Exemple 5 (composé n° 1): 2-(4-Fluoro-phényl)-6-(pipérazin-1-yl)-3-(pyridazin-4-yl)-imidazo[1,2-b]pyridazine

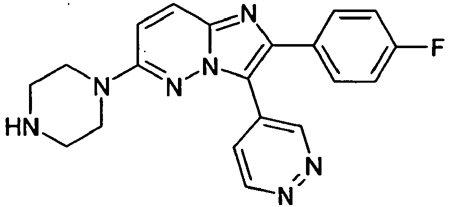

Le mélange de 0,40 g (0,86 mmole) de 6-(4-benzyl-pipérazin-1-yl)-2-(4-fluoro-phényl)-3-(pyridazin-4-yl)-imidazo[1,2-*b*]pyridazine, de 0,81 g (13 mmoles) de formiate d'ammonium et de 0,5 g de palladium sur charbon (10%) contenant 50% d'humidité dans 60 ml de méthanol est agité à reflux pendant 15 minutes.
Le solvant est ensuite éliminé par distillation sous pression réduite et le résidu obtenu est repris avec une solution aqueuse d'acide chlorhydrique 3N et cette solution est lavée avec de l'éther diéthylique, basifiée au moyen d'ammoniaque. Le produit est enfin extrait avec du dichlorométhane, la phase organique séchée sur sulfate de sodium, filtrée et concentrée sous pression réduite. On obtient 0,27 g d'un solide jaunâtre qui est chromatographié sur gel de silice en éluant avec un mélange d'ammoniaque, de méthanol et de dichlorométhane (1/10/90) pour conduire à 0,15 g de solide jaunâtre.
Ce dernier est recristallisé dans 25 ml d'acétonitrile à reflux auquel on ajoute du butanol jusqu'à la solubilité.
On isole enfin 0,11 g de solide après filtration et séchage.
PF : 240-246°C
RMN ¹H (CDCl₃) δ : 9,60 (d, 1H) ; 9,20 (dd, 1H) ; 7,85 (d, 1H) ; 7,75 (m, 1H) ; 7,60 (m, 2H) ; 6,95-7,15 (m, 3H) ; 3,55 (m, 4H) ; 3,05 (m, 4H) ; 2,1 (sl, 1H) ppm.

### Exemple 6 (composé n° 11) : 2-(4-Fluoro-phényl)-6-(4-isopropyl-pipérazin-1-yl)-3-(pyridazin-4-yl)-imidazo[1,2-b]pyridazine

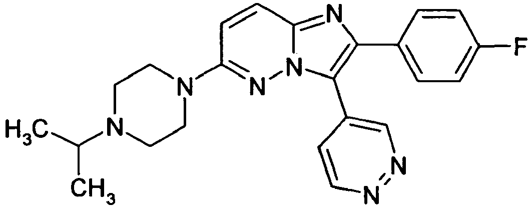

Le mélange de 0,26 g (0,8 mmole) de 6-chloro-2-(4-fluoro-phényl)-3-(pyridazin-4-yl)-imidazo[1,2-*b*]pyridazine et 0,66 g (3,2 mmoles) de 1-isopropyl-pipérazine dans 6 ml de pentanol est chauffé dans un tube scellé à 130°C pendant 10 heures.
Le solvant est évaporé sous pression réduite et le résidu est repris avec du dichlorométhane et la phase organique est lavée avec une solution aqueuse d'hydrogénocarbonate de sodium. La phase organique est séchée sur sulfate de sodium puis concentrée sous pression réduite. Le produit est chromatographié sur colonne de gel de silice en éluant avec un mélange d'ammoniaque, de méthanol et de dichlorométhane (2/4/96) pour conduire à 0,25 g de solide beige après cristallisation dans l'éther di-isopropylique et séchage.
PF : 172-174°C
RMN 1H (DMSO d6) δ : 9,30 (d, 1H) ; 9,25 (d, 1H) ; 8,00 (d, 1H) ; 7,90 (dd, 1H) ; 7,55 (m, 2H) ; 7,35 (d, 1H) ; 7,20 (pseudo t, 2H) ; 3,45 (m, 4H) ; 2,65 (m, 1H) ; 2,55 (m, 4H) ; 1,00 (d, 6H) ppm.

### Exemple 7 (composé n° 2) : 2-(4-Fluoro-phényl)-6-((R)-3-méthyl-pipérazin-1-yl)-3-(pyridazin-4-yl)-imidazo[1,2-b]pyridazine

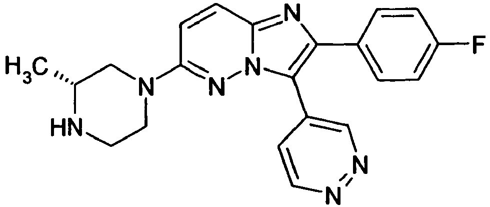

Le mélange de 0,26 g (0,8 mmole) de 6-chloro-2-(4-fluoro-phényl)-3-(pyridazin-4-yl)-imidazo[1,2-*b*]pyridazine et 0,32 g (3,2.mmoles) de (*R*)-3-méthyl-pipérazine dans 4 ml de pentanol est chauffé dans un tube scellé à 130°C pendant 32 heures.
Le solvant est évaporé sous pression réduite et le résidu est repris avec du dichlorométhane et la phase organique est lavée avec une solution aqueuse d'hydrogénocarbonate de sodium. La phase organique est séchée sur sulfate de sodium puis concentrée sous pression réduite. Le produit est chromatographié sur colonne de gel de silice en éluant avec un mélange d'ammoniaque, de méthanol et de dichlorométhane (2/4/96) pour conduire à 0,24 g de solide beige après cristallisation dans l'éther di-isopropylique et séchage.
PF : 162-164°C
[Alpha]_{D} = -7,9° (c=0,44, MeOH)
RMN ¹H (DMSO d6) δ : 9,35 (d, 1H) ; 9,25 (d, 1H) ; 7,95 (d, 1H) ; 7,85 (dd, 1H) ; 7,55 (m, 2H) ; 7,35 (d, 1H) ; 7,25 (pseudo t, 2H) ; 3,95 (m, 2H) ; 2,65-3,05 (m, 5H) ; 1,00 (d, 3H) ppm.

### Exemple 8 (composé n° 6) : 2-{4-[2-(4-Fluoro-phényl)-3-(pyridazin-4-yl)-imidazo[1,2-b]pyridazin-6-yl]-pipérazin-1-yl}-éthanol

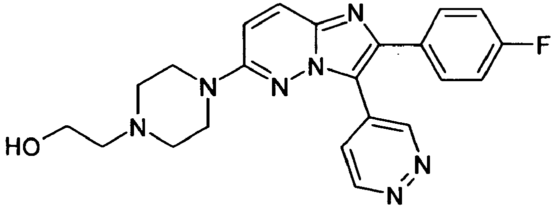

Le mélange de 0,26 g (0,8 mmole) de 6-chloro-2-(4-fluoro-phényl)-3-(pyridazin-4-yl)-imidazo[1,2-*b*]pyridazine et 0,425 g (3,2 mmoles) de 2-hydroxy-éthyl-pipérazine dans 4 ml de pentanol est chauffé dans un tube scellé à 130°C pendant 32 heures.
Le solvant est évaporé sous pression réduite et le résidu est repris avec du dichlorométhane et la phase organique est lavée avec une solution aqueuse d'hydrogénocarbonate de sodium. La phase organique est séchée sur sulfate de sodium puis concentrée sous pression réduite. Le produit est chromatographié sur colonne de gel de silice en éluant avec un mélange d'ammoniaque, de méthanol et de dichlorométhane (2/4/96) pour conduire à 0,29 g de solide beige après cristallisation dans l'éther di-isopropylique et séchage.
PF : 200-202°C
RMN ¹H (DMSO d6) δ : 9,35 (d, 1H) ; 9,25 (d, 1H) ; 8,00 (d, 1H) ; 7,90 (dd, 1H) ; 7,55 (m, 2H) ; 7,35 (d, 1H) ; 7,25 (pseudo t, 2H) ; 4,45 (t, 1H) ; 3,4-3,6 (m, 6H) ; 2,35-2,65 (m, >6H) ppm.

### Exemple 9 (composé n° 8) : 2-(4-Fluoro-phényl)-6-(2-fluoroéthyl)plpérazin-1-yl)-3-(pyridazin-4-yl)-imidazo[1,2-b]pyridazine

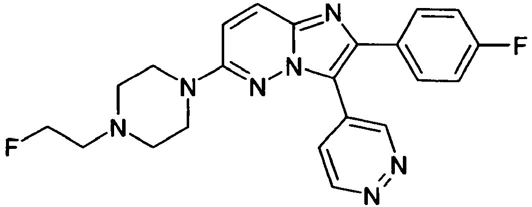

Le mélange de 0,35 g (1,07 mmole) de 6-chloro-2-(4-fluoro-phényl)-3-(pyridazin-4-yl)-imidazo[1,2-*b*]pyridazine, de 0,441 g (2,15 mmoles) de dichlorhydrate de 2-fluoroéthyl-pipérazine et de 0,75 ml (5,4 mmoles) de triéthylamine dans 4 ml de diméthylsulfoxyde est chauffé au micro-ondes à 85°C pendant 1 heure. La phase organique est séchée sur sulfate de sodium puis concentrée sous pression réduite. Le produit est chromatographié sur colonne de gel de silice en éluant avec un mélange d'ammoniaque, de méthanol et de dichlorométhane (0,5/5/95) pour conduire à 0,17 g de poudre marron après cristallisation dans l'acétonitrile et séchage.
PF : 177-179°C
RMN ¹H (DMSO d6) δ : 9,60 (d, 1H) ; 9,20 (d, 1H) ; 7,85 (d, 1H) ; 7,00 (dd, 1H) ; 7,60 (m, 2H) ; 7,15 (pseudo t, 2H) ; 7,00 (d, 1H) ; 4,75 (m, 1H) ; 4,60 (m, 1H) ; 3,6 (m, 4H) ; 2,85-2,75 (m, 5H) ppm.

### Exemple 10 (composé n° 9): 2-Phényl-6-(4-isopropyl-pipérazin-1-yl)-3-(pyridazin-4-yl)-imidazo[1,2-b]pyridazine

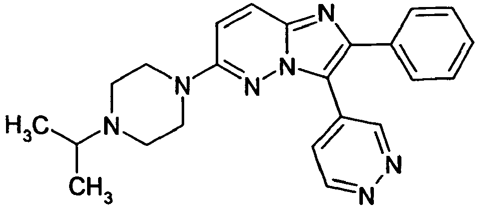

### Etape 10.1. 4-[6-Chloro-2-phényl-imidazo[1,2-b]pyridazin-3-yl]-4H-pyridazine-1-carboxylate d'éthyle

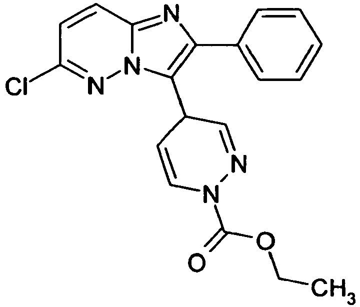

A une solution de 25,2 g (109 mmoles) de 6-chloro-2-phényl-imidazo[1,2-*b*]pyridazine et de 39,8 ml (548 mmoles) de pyridazine dans 550 ml de dichlorométhane à 8°C, on additionne goutte à goutte en 30 minutes 26,2 ml (30,3 mmoles) de chloroformiate d'éthyle. Le milieu est agité à 10°C pendant encore une heure puis laissé sous agitation pendant 1 heure supplémentaire. On refroidit le milieu à 8°C et on additionne alors 14,9 ml (273 mmoles) de pyridazine et 13,1 ml (137 mmoles) de chloroformiate d'éthyle en quinze minutes. On laisse la réaction revenir à température ambiante sous agitation pendant une heure supplémentaire. Le solvant est évaporé sous pression réduite et le résidu est repris avec 1 L d'acétate d'éthyle. La phase organique est lavée avec une solution saturée d'hydrogénocarbonate de sodium puis avec de l'eau. La phase organique est séchée sur sulfate de sodium et le solvant est évaporé sous pression réduite. Le produit est chromatographié sur colonne d'alumine neutre en éluant avec un mélange de chloroforme et de cyclohexane (6/4) pour conduire à 35 g de solide orangé qui est cristallisé dans 250 ml d'éther di-éthylique pour conduire à 30,1 g de solide beige.
PF : 126-131 °C
RMN ¹H (DMSOd₆) δ : 8,30 (d, 1H) ; 7,4 (d, 1H) ; 7,4-7,6 (m, 7H) ; 7,2 (d, 1H) ; 6,95 (d, 1 H) ; 5,1 (m, 2H) ; 4,3 (q, 2H) ; 1,33 (t, 3H) ppm.

### Etape 10.2. 6-Chloro-2-phényl-3-(pyridazin-4-yl)-imidazo[1,2-b]pyridazine

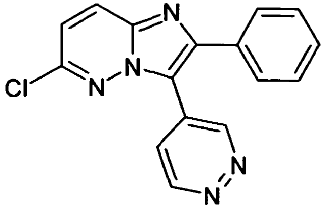

22,3 g (58,4 mmoles) de 4-[6-chloro-2-phényl-imidazo[1,2-*b*]pyridazin-3-yl]-4*H*-pyridazine-1-carboxylate d'éthyle sont dissous dans 450 ml de chloroforme et on ajoute par portions 15,1 g (61,3 mmoles) d'ortho-chloranile. Le milieu réactionnel est agité 30 minutes à température ambiante puis versé sur une solution aqueuse de soude 2N. Le produit est extrait avec du chloroforme. La phase organique est séchée sur sulfate de sodium puis concentrée sous pression réduite. Le résidu beige foncé est cristallisé dans un mélange de 80 ml d'isopropanol et de 60 ml d'éther di-isopropylique à chaud pour conduire 12,8 g de poudre beige après refroidissement isolation par filtration et séchage.
PF : 200-207°C
RMN ¹H (CDCl₃) δ : 9,40 (s, 1H) ; 9,30 (d, 1H) ; 8,05 (d, 1H) ; 7,90 (m, 1H) ; 7,65 (m, 2H) ; 7,45 (m, 3H) ; 7,25 (m, 1 H) ppm.

### Etape 10.3. 2-Phényl-6-(4-isopropyl-pipérazin-1-yl)-3-(pyridazin-4-yl)-imidazo[1,2-b]pyridazine

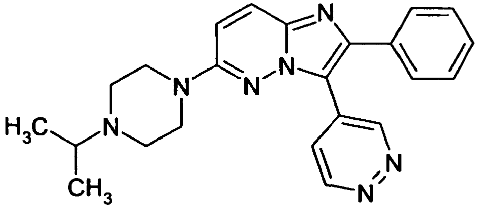

Le mélange de 0,45 g (1,5 mmoles) de 6-chloro-2-phényl-3-(pyridazin-4-yl)-imidazo[1,2-*b*]pyridazine et 0,38 g (2,9 mmoles) de 1-isopropyl-pipérazine dans 6 ml de pentanol est chauffé dans un tube scellé à 150°C pendant 2 jours. Le mélange est refroidit et versé sur une solution aqueuse d'acide chlorhydrique 1N. Cette solution est lavée avec de l'éther diéthylique puis basifiée au moyen d'une solution aqueuse de soude. Le produit est enfin extrait avec du dichlorométhane, la phase organique séchée sur sulfate de sodium, filtrée et concentrée sous pression réduite. On obtient une huile brune qui est chromatographiée sur gel de silice en éluant avec un mélange d'ammoniaque, de méthanol et de dichlorométhane (0,4/4/96) pour conduire à 0,50 g de solide.
Ce dernier est recristallisé dans 30 ml d'éther diéthylique. On isole enfin 0,29 g de solide blanc après filtration et séchage.
PF : 154-156°C
RMN ¹H (DMSO d6) δ : 9,35 (d, 1H) ; 9,30 (d, 1H) ; 8,05 (d, 1H) ; 7,95 (dd, 1H) ; 7,60 (m, 2H) ; 7,4 (m, 4H) ; 3,50 (m, 4H) ; 2,70 (m, 1H) ; 2,60 (m, 4H) ; 1,00 (d, 6H) ppm.

### Exemple 11 (composé n° 18) : 2-(4-Fluorophényl)-6-(4-cyclo-propyl-pipérazin-1-yl)-3-(pyridazin-4-yl)-imidazo[1,2-b]pyridazine

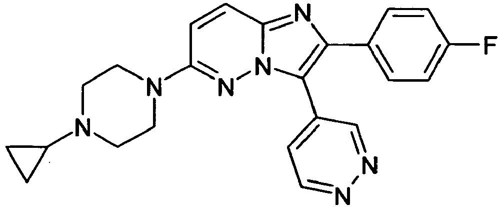

Dans un réacteur, à 0,10 g (0,27 mmole) de 2-(4-fluoro-phényl)-6-(pipérazin-1-yl)-3-(pyridazin-4-yl)-imidazo [1,2-*b*] pyridazine dans 3 ml de méthanol, on additionne 0,15 ml d'acide acétique et 0,32 ml (1,6 mmoles) de (1-éthoxycyclopropoxy)triméthylsilane. On additionne ensuite 1,2 ml (1,2 mmoles) d'une solution molaire de cyanoborohydrure de sodium dans le tétrahydrofurane. Après 15 minutes d'agitation, le mélange est chauffé à 50 °C pendant 2 heures. Après refroidissement, le produit est extrait avec du dichlorométhane, la phase organique est filtrée sur cartouche hydrophobe puis concentrée sous pression réduite. Le résidu est chromatographié sur colonne de gel de silice pour conduire à 0,064 g de produit. Celui-ci est cristallisé dans l'éther di-isopropylique pour donner 0,053 g de cristaux jaunes clairs après séchage.
PF : 206-209°C
RMN 1 H (DMSO d6) δ: 9,35 (s, 1H) ; 9,30 (d, 1 H) ; 8,05 (d, 1H) ; 7,90 (dd, 1 H) ; 7,60 (m, 2H) ; 7,40 (d, 1 H) ; 7,25 (pseudo t, 2H) ; 3,45 (m, 4H) ; 2,70 (m, 4H) ; 1,70 (m, 1 H) ; 0,45 (m, 2H) ; 0,35 (m, 2H) ppm.

### Exemple 12 (composé n° 36) : 2-(3,5-Difluorophényl)-3-(pyridazin-4-yl)-6-(4-pyrrolidin-1-yl-pipéridin-1-yl)-imidazo[1,2-b]pyridazine

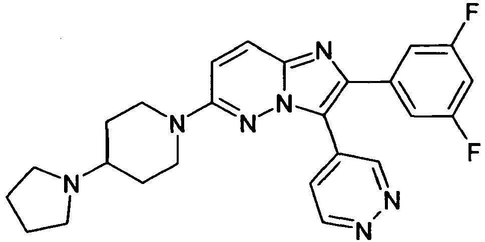

### Etape 12.1. Bromure de 6-amino-3-chloro-1-(éthoxycarbonylméthyl)-pyridazin-1-ium

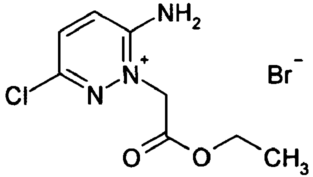

Un mélange de 25,6 g (198 mmoles) de 6-chloro-2-phényl-imidazo[1,2-*b*]pyridazine dans 230 ml d'éthanol chaud est traité avec 34,0 g (206 mmoles) de bromoacétate d'éthyle. Après, chauffage à reflux pendant 24 heures, le mélange est refroidit et les cristaux sont séparés par filtration.
On isole 36,6 g de produit après séchage.
On isole 7,1 g supplémentaires par évaporation du solvant sous pression réduite et recristallisation dans l'éthanol.
RMN 1H (DMSO d6) δ : 9,8 (signal large, 1H) ; 9,4 (signal large, 1H) ;8,0 (d, 1H) ; 7,7 (d, 1H) ; 5,3 (s, 1 H) ; 4,1 (d, 2H) ; 1,2 (t, 3H) ppm.

### Etape 12.2. 2-Bromo-6-chloro-imidazo[1,2-b]pyridazine et 2,6-dibromo-imidazo[1,2-b]pyridazine

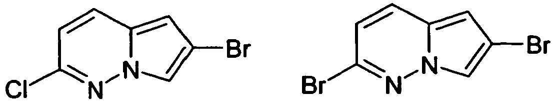

Le mélange de 20 g (65 mmoles) de bromure de 6-amino-3-chloro-1-(éthoxycarbonylméthyl)-pyridazin-1-ium et de 63 g d'oxybromure de phosphore dans 50 ml de toluène est chauffé à 160°C pendant 3 heures. Le mélange est ensuite versé sur de la glace (300 ml). Après agitation, le solide est séparé par filtration puis purifié par chromatographie sur gel de silice en éluant avec un mélange de 0 à 10% de méthanol dans le dichlorométhane. On isole ainsi 8,05 g de mélange des deux produits utilisés tel quel pour la suite de la synthèse.

### Etape 12.3. 2-Bromo-6-(4-pyrrolidin-1-yl-pipéridin-1-yl)-imidazo[1,2-b]pyridazine

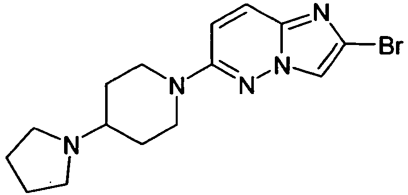

1,42 g (5,1 mmoles) du mélange obtenu à l'étape précédente et 2,42 g (15,7 mmoles) de 4-pyrrolidin-1-yl-pipéridine dans 15 ml de butanol sont chauffés au micro-ondes à 180°C pendant 5 heures dans un tube scellé. Le solvant est ensuite éliminé par évaporation sous pression réduite et le résidu est chromatographié sur colonne de gel de silice en éluant avec un mélange de 0 à 10% de méthanol dans le dichlorométhane. On isole ainsi 1,58 g de produit.
RMN ¹H (CDCl₃) δ : 7,6 (d+s, 2H) ; 6,8 (d, 1H) ; 4,05 (m, 2H) ; 3,0 (m, 2H) ; 2.6 (m, 4H) ; 2,2 (m, 1H) ; 2,0 (m, 2H) ; 1,8 (m, 4H) ; 1,6 (m, 2H) ppm.

### Etape 12.4. 4-[2-Bromo-6-(4-pyrrolidin-1-yl-pipéridin-1-yl)-imidazo[1,2-b]pyridazin-3-yl]-4H-pyridazine-1-carboxylate d'éthyle

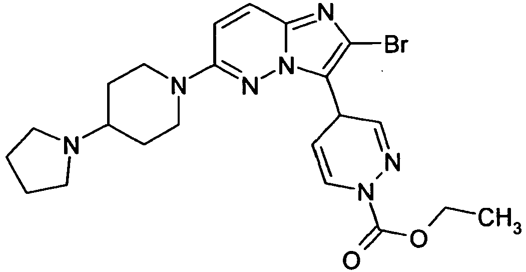

A un mélange de 1,68 g (4,8 mmoles) de 2-bromo-6-(4-pyrrolidin-1-yl-pipéridin-1-yl)-imidazo[1,2-*b*]pyridazine et 1,92 g (24 mmoles) de pyridazine dans 25 ml de chloroforme, on additionne 1,3 g (12 mmoles) de chloroformiate d'éthyle goutte à goutte en 5 minutes en refroidissant au bain de glace. Le mélange est agité à température ambiante pendant une heure puis il est dilué avec du dichlorométhane et la solution est lavée successivement avec une solution de bicarbonate de sodium puis avec une solution saturée de chlorure de sodium. La phase organique est séchée sur sulfate de sodium, puis concentrée sous pression réduite après addition de gel de silice. Le résidu d'évaporation est déposé sur colonne de gel de silice et le produit est purifié par chromatographie sur colonne de gel de silice en éluant avec un mélange de 0 à 10% de méthanol dans le dichlorométhane. On isole ainsi 1,93 g de produit suffisamment pur pour la suite de la synthèse.
RMN ¹H (CDCl₃) δ : 7,5 (d, 1H) ; 7,3 (m) ; 6,8 (m, 2H) ; 4,8 (m, 1H) ; 4,7 (m, 1H) ; 4,3 (q, 2H) ; 3,95 (m, 2H) ; 2,9 (m, 2H) ; 2,5 (m, 4H) ; 2,2 (m, 1 H) ; 1,9 (m, 2H) ; 1,8 (m, 4H) ; 1,5 (m, 4H) ; 1,3 (t, 3H) ppm.

### Etape 12.5. 2-Bromo-3-pyridazin-4-yl-6-(4-pyrrolidin-1-yl-pipéridin-1-yl)-imidazo[1,2-b]pyridazine

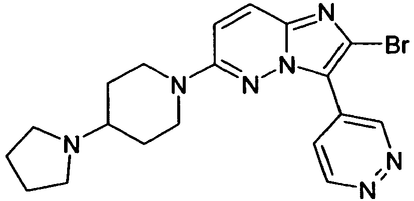

A un mélange de 0,16 g (0,32 mmole) de 4-[2-bromo-6-(4-pyrrolidin-1-yl-pipéridin-1-yl)-imidazo[1,2-*b*]pyridazin-3-yl]-4*H*-pyridazine-1-carboxylate d'éthyle dans 4 ml de chloroforme, on additionne 0,101 g d'orthochloranile (0,41 mmole) en solution dans 1 ml de toluène. Le mélange est agité à température ambiante pendant 1 heure puis dilué avec 40 ml de dichlorométhane. La solution est lavée avec 2 ml d'une solution aqueuse de soude 2N puis avec une solution saturée de chlorure de sodium. La phase organique est séchée sur sulfate de sodium, puis concentrée sous pression réduite après addition de gel de silice. Le résidu d'évaporation est déposé sur colonne de gel de silice et le produit est purifié par chromatographie sur colonne de gel de silice en éluant avec un mélange de 0 à 10% de méthanol dans le dichlorométhane. On isole ainsi 0,080 g de produit.
RMN ¹H (CDCl₃) δ: 9,95 (d, 1 H) ; 9,29 (d, 1H); 8,15 (dd, 1 H) ; 7,60 (d, 1H) ; 6,85 (d, 1H) ; 4,1 (m, 2H) ; 3,7 (m, 2H) ; 2,7 (m, 4H) ; 2,3 (m, 1H) ; 2,0 (m, 2H) ; 1,8 (m, 4H) ; 1,7 (m, 2H) ppm.

### Etape 12.6. 2-(3,5-Difluorophényl)-3-(pyridazin-4-yl)-6-(4-pyrrolidin-1-yl-pipéridin-1-yl)-imidazo[1,2-b]pyridazine

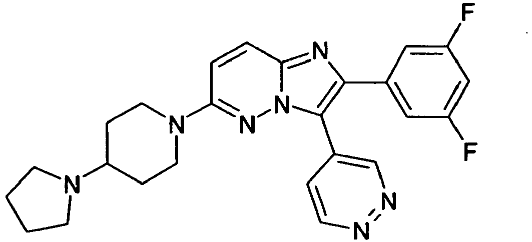

Un mélange de 0,090 g (0,21 mmole) de 2-bromo-3-pyridazin-4-yl-6-(4-pyrrolidin-1-yl-pipéridin-1-yl)-imidazo[1,2-*b*]pyridazine, de 0,014 g (0,02 mmole) de 1,1'-bis(diphénylphosphino)ferrocènedichloropalladium et de 0,043 g (0,27 mmole) d'acide 3,5-difluorophénylboronique et d'une solution aqueuse de carbonate de césium 2M dans 2 ml de dioxane est chauffé à 120°C pendant 20 minutes au four micro-ondes. On injecte ensuite dans le tube 1 ml d'une solution aqueuse saturée de chlorure de sodium et 4 ml d'acétate d'éthyle. Après agitation, la phase organique est prélevée à la seringue et passée sur cartouche de sulfate de sodium. La solution est directement injectée sur colonne de gel de silice et est chromatographiée en éluant avec un mélange de 0 à 10% de méthanol dans le dichlorométhane. On isole ainsi 0,037 g de produit.
RMN ¹H (CDCl₃) δ : 9,6 (m, 1H) ; 9,2 (d, 1H) ; 7,8 (m, 2H) ; 7,2 (m, 2H) ; 7,05 (d, 1H) ; 6,9 (m, 1 H) ; 4,10 (m, 2H) ; 3,1 (m, 2H) ; 2,7 (m, 4H) ; 2,35 (m, 1 H) ; 2,1 (m, 2H) ; 1,9 (m, 4H) ; 1,7 (m, 2H) ppm.

Le tableau 1 qui suit illustre les structures chimiques et les propriétés physiques de quelques composés selon l'invention.

Dans ce tableau :
- la colonne "PF°C" renseigne les points de fusion des produits en degrés Celsius. "N.D" signifie que le point de fusion est non déterminé,
- la colonne "LC-MS ou (MS)" renseigne le résultat d'analyse des produits par LC-MS (liquid chromatography coupled to Mass Spectroscopy) réalisée sur un appareil de type Agilent LC-MSD Trap en mode ESI positif ou par MS (Mass Spectroscopy) sur un appareil Autospec M (EBE) en utilisant la technique DCI-NH3,
- « CH₃- » signifie méthyle,

**TABLEAU 1**

| **No** | **-N-A-L-B-** | **R₇** | **R₈** | **R₂** | **PF** °**C** | **M+H** |
|---|---|---|---|---|---|---|
| 1 | Pipérazin-1-yl | H | H | 4-Fluoro-phényl | 240-246 | 376 |
| 2 | 3-(*R*)-Méthyl-pipérazin-1-yl | H | H | 4-Fluoro-phényl | 162-164 | 390 |
| 3 | 3,3-Diméthyl-pipérazin-1-yl | H | H | 4-Fluoro-phényl | 226-228 | 404 |
| 4 | *cis*-3,5-Diméthyl-pipérazin-1-yl | H | H | 4-Fluoro-phényl | 197-199 | 404 |
| 5 | 4-Méthyl-pipérazin-1-yl | H | H | 4-Fluoro-phényl | 232-233 | 390 |
| 6 | 4-(2-hydroxy-éthyl)-pipérazin-1-yl | H | H | 4-Fluoro-phényl | 200-202 | 420 |
| 7 | 4-(2-Fluoroéthyl)-pipérazin-1-yl | H | H | Phényl | 156-158 | 404 |
| 8 | 4-(2-Fluoroéthyl)-pipérazin-1-yl | H | H | 4-Fluoro-phényl | 177-179 | 422 |
| 9 | 4-*Iso*propyl-pipérazin-1-yl | H | H | Phényl | 154-156 | 400 |
| 10 | 4-*Iso*propyl-pipérazin-1-yl | H | H | 3-Fluoro-phényl | 197-199 | 418 |
| 11 | 4-*Iso*propyl-pipérazin-1-yl | H | H | 4-Fluoro-phényl | 172-174 | 418 |
| 12 | 4-*Iso*propyl-pipérazin-1-yl | CH₃- | CH₃- | 4-Fluoro-phényl | 222-224 | 446 |
| 13 | 4-*Iso*propyl-pipérazin-1-yl | H | H | 3,5-Diméthyl-phényl | 183-184 | 428 |
| 14 | 4-*Iso*propyl-pipérazin-1-yl | H | H | 3,4-Difluoro-phényl | 192-194 | 436 |
| 15 | 4-*Iso*propyl-pipérazin-1-yl | H | H | 3,5-Difluoro-phényl | N D. | 436 |
| 16 | 4-*Iso*propyl-pipérazin-1-yl | H | H | 3,5-Dichloro-phényl | N.D | 468 |
| 17 | 4-*Iso*propyl-pipérazin-1-yl | H | H | 3,5-Di(trifluorométhyl)-phényl | N.D. | 536 |
| 18 | 4-Cyclo-propyl-pipérazin-1-yl | H | H | 4-Fluoro-phényl | 206-209 | 416 |
| 19 | 4-Benzyl-pipérazin-1-yl | H | H | 4-Fluoro-phényl | 198-201 | 466 |
| 20 | 4-(2-Hydroxy-2-méthylpropyl)-pipérazin-1-yl | H | H | 4-Fluoro-phényl | 200-202 | 448 |
| 21 | 4-(3-Hydroxy-3-méthylbutyl)-pipérazin-1-yl | H | H | 4-Fluoro-phényl | 148-150 | 462 |
| 22 | (*S*)-Octahydro-pyrrolo[1,2-*a*]pyrazin-2-yl | H | H | 4-Fluoro-phényl | 203-205 | 416 |
| | 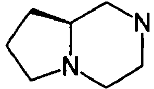 | | | | | |
| 23 | (1*S*,4*S*)-2,5-Diaza-bicyclo[2.2.1]hept-2-yl | H | H | 4-Fluoro-phényl | 205-207 | 388 |
| | 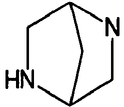 | | | | | |
| 24 | 5-(2-Hydroxy-éthyl)-(1*S*,4*S*)-2,5-diaza-bicyclo[2.2.1]hept-2-yl | H | H | 4-Fluoro-phényl | 175-177 | 432 |
| 25 | 5-*Iso*propyl-(1*S*,4*S*)-2,5-diaza-bicyclo[2.2.1]hept-2-yl | H | H | 4-Fluoro-phényl | 201-203 | 430 |
| 26 | Hexahydro-pyrrolo[3,4-*c*]pyrrol-2(1*H*)-yl | H | H | 4-Fluoro-phényl | 130-135 | 402 |
| | 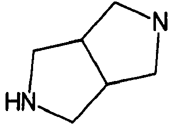 | | | | | |
| 27 | 5-Méthyl-hexahydro-pyrrolo[3,4-*c*]pyrrol-2(1*H*)-yl | H | H | 4-Fluoro-phényl | 150-152 | 416 |
| 28 | 5-(2-*Iso*propyl)-hexahydro-pyrrolo[3,4-*c*]pyrrol-2(1*H*)-yl | H | H | 4-Fluoro-phényl | 198-200 | 444 |
| 29 | 5-(2-hydroxy-éthyl)-hexahydro-pyrrolo[3,4-*c*]pyrrol-2(1*H*)-yl | H | H | 4-Fluoro-phényl | 208-210 | 446 |
| 30 | 5-(Benzyl)-hexahydro-pyrrolo[3,4-*c*]pyrrol-2(1*H*)-yl | H | H | 4-Fluoro-phényl | N.D. | 492 |
| 31 | 2,9-Diaza-spiro[5.5]undéc-9-yl | H | H | 4-Fluoro-phényl | 203-205 | 444 |
| | 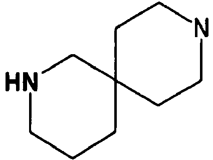 | | | | | |
| 32 | 1-Oxa-4,9-diazaspiro[5.5]undéc-9-yl | H | H | 4-Fluoro-phényl | 212-214 | 446 |
| | 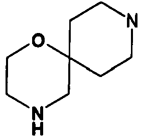 | | | | | |
| 33 | (*R*,*S*)-3-[Pyrrolidin-1-yl]-pyrrolidin-1-yl- | H | H | 4-Fluoro-phényl | 182-184 | 430 |
| 34 | 4-(Pyrrolidin-1-yl)-pipéridin-1-yl | H | H | 4-Fluoro-phényl | 196-198 | 444 |
| 35 | 4-(Pyrrolidin-1-yl)-pipéridin-1-yl | H | H | 3,5-Diméthyl-phényl | N.D. | 454 |
| 36 | 4-(Pyrrolidin-1-yl)-pipéridin-1-yl | H | H | 3,5-Difluoro-phényl | N.D. | 426 |
| 37 | 4-(Pyrrolidin-1-yl)-pipéridin-1-yl | H | H | 3,5-Dichloro-phényl | N.D. | 494 |
| 38 | 4-(Pyrrolidin-1-yl)-pipéridin-1-yl | H | H | 3,5-Di(trifluorométhyl)-phényl | N.D. | 526 |
| 39 | 3-(Hydroxyméthyl)-pipérazin-1-yl | H | H | 4-Fluoro-phényl | 205-212 | 406 |
| 40 | 3-(Fluorométhyl)-pipérazin-1-yl | H | H | 4-Fluoro-phényl | 184-189 | 408 |
| 41 | 5-Méthyl-hexahydro-pyrrolo[3 4-*c*]pyrro)-2(1*H*)-yl | H | H | Phényl | 154-157 | 398 |
| 42 | 5-Méthyl-hexahydro-pyrrolo[3,4-*c*]pyrrol-2(1*H*)-yl | H | H | 3-Fluoro-phényl | 168-170 | 416 |
| 43 | 5-Méthyl-hexahydro-pyrrolo[3 4-*c*]pyrrol-2(1*H*)-yl | CH₃- | H | 4-Fluoro-phényl | 187-189 | 430 |
| 44 | 5-Méthyl-hexahydro-pyrrolo[3,4-*c*]pyrro)-2(1*H*)-yl | H | CH₃- | 4-Fluoro-phényl | 255-257 | 430 |
| 45 | 5-Méthyl-hexahydro-pyrrolo[3,4-*c*]pyrrol-2(1*H*)-yl | H | H | 3,4-Difluoro-phényl | 186-188 | 434 |

### Exemples biologiques

La capacité des composés de l'invention à inhiber la phosphorylation de la caséine par les caséine kinase 1 epsilon et delta peut être évaluée selon la procédure décrite dans le document US20050131012.

### Dosage sur Plaque-Fiftre-d'ATP-³³P pour le criblage des inhibiteurs de CK1epsilon :

On mesure l'effet des composés pour inhiber la phosphorylation de la caséine par l'enzyme caséine kinase 1 epsilon (CK1 epsilon) en utilisant un dosage de la caséine par filtration d'ATP-³³P in vitro.
La Caséine Kinase 1 epsilon (0,58 mg/ml) est obtenue par des procédés de fermentation et de purification effectués selon des méthodes bien connues de l'homme du métier ou peut également être obtenue auprès d'Invitrogen Corporation™ (human CK1 epsilon). Les composés sont testés à cinq concentrations différentes de manière à générer des Cl₅₀, c'est à dire la concentration à laquelle un composé est capable d'inhiber l'activité enzymatique de 50%, ou bien l'inhibition en % à une concentration de 10 micromolaires.

On prépare des plaques Falcon à fond en « U » en plaçant 5 µL de solutions des composés selon l'invention aux concentrations de 10, 1, 0,1, 0,01 ou 0,001 µM dans différents puits. Les solutions des composés selon l'invention à ces différentes concentrations sont préparées par dilution dans un tampon d'essai (Tris 50 mM pH 7,5, MgCl2 10 M, DTT 2 mM et EGTA 1 mM) d'une solution mère dans le DMSO à la concentration de 10 mM. Ensuite, on additionne 5 µL de caséine déphosphorylée à la concentration finale de 0,2 µg/µL, 20 µL de CK1 epsilon à la concentration finale de 3 ng/µL, et 20 µL d'ATP-³³P à la concentration finale de 0,02 µCi/µL mélangée avec de l'ATP froide (10 µM final - environ 2× 106 CPM par puits). Le volume total final d'essai par puits est égal à 50 µL.
La plaque d'essai Falcon^{®} à fond en « U » citée ci-dessus est agitée au vortex, puis incubée à la température ambiante pendant 2 heures. Après 2 heures, la réaction est arrêtée par addition d'une solution glacée de 65 µL d'ATP froid (2 mM) préparée dans du tampon d'essai.
On transfère ensuite 100 µL du mélange réactionnel de la plaque Falcon^{®} à fond en U dans des plaques de filtration MAPH Millipore^{®}, préalablement imprégnées avec 25 µL de TCA glacé à 100 %
Les plaques de filtration MAPH Millipore sont agitées doucement et on les laisse au repos à la température ambiante pendant au moins 30 minutes pour précipiter les protéines. Après 30 minutes, les plaques de filtration sont séquentiellement lavées et filtrées avec 2×150 µL de TCA à 20%, 2×150 µL de TCA à 10% et 2×150 µL de TCA à 5% (6 lavages au total par plaque/900 µL par puits).
On laisse les plaques sécher pendant une nuit à la température ambiante. Ensuite, on ajoute 40 µL de liquide de scintillation Microscint-20 Packard^{®} par puits et les plaques sont fermées de manière étanche. On mesure alors le rayonnement émis par chaque puits pendant 2 minutes dans un compteur à scintillation Topcount NXT Packard^{®} où les valeurs de CPM /puits sont mesurées.
On détermine l'inhibition en % de la capacité de l'enzyme à phosphoryler le substrat (caséine) pour chaque concentration de composé testé. Ces données d'inhibition exprimées en % sont utilisées pour calculer la valeur de Cl₅₀ pour chaque composé comparativement aux contrôles.

Les études cinétiques ont déterminé la valeur de K_{M} pour ATP comme étant de 21 µM dans ce système d'essai.

Le tableau 2 ci-dessous présente les Cl₅₀ d'inhibition de la phosphorylation de la Caséine Kinase 1 Epsilon pour quelques composés selon l'invention.

**Tableau 2**

| *Composé N*° | CK1 epsilon Cl₅₀ (nM) |
|---|---|
| 5 | 16 - 33 |
| 21 | 134 - 166 |
| 28 | 54 - 69 |
| 39 | 98 |

Dans ces conditions, les composés les plus actifs de l'invention présentent des Cl₅₀ (concentration inhibant de 50 % l'activité enzymatique de la Caséine Kinase 1 Epsilon) comprises entre 1 nM et 2 µM.

La capacité des composés de l'invention à inhiber la phosphorylation de la caséine par les caséines kinases 1 epsilon et delta peut être évaluée en utilisant un test de fluorescence FRET (« transfert d'énergie entre molécules fluorescentes », de l'anglais « Fluorescence Resonance Energy Transfert ») à partir du kit « Z'Lyte^{™} kinase assay Kit » (référence PV3670 ; Invitrogen Corporation™) selon les instructions du fournisseur.

Les Caséines Kinases 1 utilisées sont obtenues chez Invitrogen Corporation (human CK1 epsilon PV3500 et human CK1 delta PV3665).

Un peptide substrat, marqué à ses deux extrémités par un groupe fluorophore donneur (la coumarine) et un groupe fluorophore accepteur (la fluorescéine) constituant un système FRET est phosphorylé en présence d'ATP par la caséine kinases 1 epsilon ou delta en présence de concentrations croissantes de composés de l'invention.
Le mélange est traité au moyen d'une protéase site spécifique coupant spécfiquement le peptide substrat pour former deux fragments fluorescents présentant un grand ratio d'émission par fluorescence.
La fluorescence observée est donc reliée à la capacité des produits de l'invention à inhiber la phosphorylation du peptide substrat par la caséine kinase 1 epsilon ou de la caséine kinase 1 delta.

Les composés de l'invention sont mis en solution à des concentrations différentes à partir d'une solution mère à 10 mM dans le DMSO diluée dans un tampon contenant 50 mM HEPS, pH 7,5, 1 mMEGTA, 0,01% Brij-35, 10 mM MgCl pour la caséine kinase 1 epsilon et supplémenté avec Trizma Base (50 mM), pH 8,0 et NaN3 (0,01% finaux) pour la caséine kinase 1 delta.
La phosphorylation du peptide substrat SER/THR 11 obtenu chez Invitrogen Corporation™ est réalisée à la concentration finale de 2 µM. La concentration en ATP est de 4 fois le K_{M}, celui-ci étant de 2 µM pour la caséine kinase 1 epsilon et de 4 µM pour la caséine kinase 1 delta.
La mesure de la fluorescence émise est mesurée aux longueurs d'onde de 445 et 520 nm (excitation à 400 nm).

Le tableau 3 ci-dessous présente les Cl₅₀ d'inhibition de la phosphorylation de la Caséine Kinase 1 Delta pour quelques composés selon l'invention.

**Tableau 3**

| Composé N° | CK1 delta Cl₅₀ (nM) |
|---|---|
| 1 | 1 |
| 5 | 41 - 56 |
| 11 | 50 - 65 |

Dans ces conditions, les composés les plus actifs de l'invention présentent des Cl₅₀ (concentration inhibant de 50 % l'activité enzymatique de la Caséine Kinase 1 Delta) comprises entre 1 nM et 2 µM.

Il apparaît donc que les composés selon l'invention ont une activité inhibitrice sur l'enzyme Caséine Kinase 1 epsilon ou Caséine Kinase 1 delta.

### Protocoles expérimentaux de dosage circadien cellulaire

Des cultures de fibroblastes Mper1-luc Rat-1 (P2C4) ont été réalisées en divisant les cultures tous les 3-4 jours (environ 10-20 % de confluence) sur des flacons de culture de tissus en polystyrène dégazés de 150 cm² (Falcon® # 35-5001) et maintenues en milieu de croissance [EMEM (Cellgro #10-010-CV) ; sérum bovin foetal à 10 % (FBS; Gibco #16000-044) ; et 50 I.U./mL de pénicilline-streptomycine (Cellgro #30-001-Cl)] à 37°C et sous CO₂ 5 %.
Des cellules issues de cultures de fibroblastes Rat-1 à 30-50 % de confluence telle que décrite ci-dessus ont été co-transfectées avec des vecteurs contenant le marqueur de sélection pour la résistance à la Zéocine pour une transfection stable et un gène rapporteur de la luciférase dirigé par le promoteur mPer-1. Après 24 à 48 heures, les cultures ont été divisées sur des plaques de 96 puits et maintenues en milieu de croissance additionné de 50-100 µg/mL de Zéocine (Invitrogen^{®} #45-0430) pendant 10-14 jours. Les transfectants stables résistant à la Zéocine ont été évalués pour l'expression du rapporteur en ajoutant au milieu de croissance de la luciférine 100 µM (Promega^{®} #E1603^{®}) et en dosant l'activité de la luciférase sur un compteur à scintillation TopCount^{®} (Packard Modèle #C384V00). Les clones de cellule Rat-1 exprimant aussi bien la résistance à la Zéocine que l'activité de la luciférase dirigée par mPer1 ont été synchronisés par choc au sérum avec du sérum de cheval à 50 % [HS (Gibco^{®} #16050-122)] et l'activité du rapporteur circadien a été évaluée. Le clone P2C4 de fibroblastes Mper1-luc Rat-1 a été sélectionné pour l'essai du composé.

Des fibroblastes Mper1-luc Rat-1 (P2C4) à 40-50 % de confluence obtenus selon le protocole décrit précédemment ont été étalés sur des plaques de culture de tissu opaques de 96 puits (Perkin Elmer^{®} #6005680). Les cultures sont maintenues en milieu de croissance additionné de 100 µg/mL de Zéocine (Invitrogen #45-0430). jusqu'à ce qu'elles aient atteint 100 % de confluence (48-72 h). Les cultures ont ensuite été synchronisées avec 100 µL de milieu de synchronisation [EMEM (Cellgro #10-010-CV) ; 100 I.U. /mL de pénicilline-streptomycine (Cellgro #30-001-C1) ; HS à 50% (Gibco #16050-122)] pendant 2 heures à 37°C et sous CO₂ 5%. Après synchronisation, les cultures ont été rincées avec 100 µL d'EMEM (Cellgro #10-010-CV) pendant 10 minutes à température ambiante. Après rinçage, le milieu a été remplacé par 300 µL de milieu indépendant de CO₂ [CO₂I (Gibco #18045-088) ; L-glutamine 2 mM (Cellgro #25-005-Cl) ; 100 U.I./mL de pénicilline-streptomycine (Cellgro #30-001-C1) ; luciférine 100 µM (Promega #E 1603)]. Les composés de l'invention testés pour les effets circadiens ont été ajoutés à du milieu indépendant de CO₂ dans du DMSO à 0,3 % (concentration finale). Les cultures ont été fermées immédiatement de manière étanche avec du film TopSeal-A^{®} (Packard #6005185) et transférées pour la mesure de l'activité de luciférase.
Après synchronisation, les plaques d'essai ont été maintenues à 37°C dans une étuve de culture de tissu (Forma Scientific Modèle #3914). L'activité de luciférase In Vivo a été estimée en mesurant l'émission relative de lumière sur un compteur à scintillation TopCount (Packard Modèle #C384V00).
L'analyse de périodes a été effectuée soit en déterminant l'intervalle entre les minimums d'émission relative de lumière sur plusieurs jours ou par transformation de Fourier. Les deux méthodes ont produit une estimation de période pratiquement identique sur une gamme de périodes circadiennes. La puissance est rapportée en CE Delta (t+1h), qui est présentée comme la concentration micromolaire efficace qui a induit un prolongement de la période de 1 heure. Les données ont été analysées par ajustement d'une courbe hyperbolique aux données exprimées en changement de période (ordonnée) en fonction de la concentration du composé à tester (abscisse) dans le logiciel XLfit™ et la CE Delta (t+1 h) a été interpolée à partir de cette courbe.

Le tableau 4 ci-dessous présente les CE Delta (t+1h) pour quelques composés selon l'invention.

**Tableau 4**

| *Composé N°* | CE Delta (t+1h) (nM) |
|---|---|
| 2 | 164 |
| 5 | 5 |
| 14 | 62 |
| 39 | 178 |

Dans ces conditions, les composés les plus actifs de l'invention présentent des CE Delta (t+1h) (concentration micromolaire efficace qui a induit un prolongement de la période de 1 heure) comprises entre 1 nM et 2 µM.

En inhibant les enzymes CK1epsilon et/ou de CK1delta, les composés objets de l'invention modulent la rythmicité circadienne, et peuvent être utiles pour le traitement des désordres liés au rythme circadien.

Les composés selon l'invention peuvent notamment être utilisés pour la préparation d'un médicament destiné à prévenir ou à traiter les désordres du sommeil ; les troubles du rythme circadien, tels que notamment ceux dus au décalage horaire, au travail posté. Parmi les troubles du sommeil, on distingue notamment les troubles primaires du sommeil tels que la dyssomnie (par exemple l'insomnie primaire), la parasomnie, l'hypersomnie (par exemple la somnolence excessive), la narcolepsie, les troubles du sommeil liés à l'apnée du sommeil, les troubles du sommeil liés au rythme circadien et les dyssomnies non spécifiées par ailleurs, les troubles du sommeil associés à des troubles médicaux/psychiatriques.

Les composés objets de l'invention provoquent également un déplacement de la phase circadienne et une telle propriété peut être utile dans le cadre d'une monothérapie ou une thérapie combinée potentielle cliniquement efficace pour les troubles de l'humeur.
Parmi les troubles de l'humeur, on distingue notamment les troubles dépressifs (dépression unipolaire), les troubles bipolaires, les troubles de l'humeur dus à une affection médicale générale ainsi que les troubles de l'humeur induits par des substances pharmacologiques.

Parmi les troubles bipolaires, on distingue notamment les troubles bipolaires I est troubles bipolaires II dont notamment les troubles affectifs saisonniers.

Les composés objets de l'invention modulant la rythmicité circadienne, peuvent être utiles dans le traitement des troubles anxieux et dépressifs dus en particulier à une altération sur la sécrétion de CRF.
Parmi les troubles dépressifs, on distingue notamment les troubles dépressifs majeurs, troubles dysthymiques, les troubles dépressifs non spécifiés par ailleurs.

Les composés objets de l'invention modulant la rythmicité circadienne, peuvent être utiles pour la préparation d'un médicament destiné à traiter les maladies liées à la dépendance à des substances d'abus telles que la cocaïne, la morphine, la nicotine, l'éthanol, le cannabis.

En inhibant la caséine kinase 1 epsilon et/ou la caséine kinase 1 delta, les composés selon l'invention peuvent être utilisés pour la préparation de médicaments, notamment pour la préparation d'un médicament destiné à prévenir ou à traiter des maladies reliées à l'hyperphosphorylation de la protéine tau, notamment la maladie d'Alzheimer.

Ces médicaments trouvent également leur emploi en thérapeutique, notamment dans le traitement ou la prévention des maladies causées ou exacerbées par la prolifération des cellules et en particulier des cellules tumorales.

Comme inhibiteur de la prolifération des cellules tumorales, ces composés sont utiles dans la prévention et le traitement des tumeurs liquides telles que les leucémies, des tumeurs solides à la fois primaires et métastasiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage ; cancer du foie, cancer de l'estomac, cancer des canaux biliaires, cancer de la vésicule biliaire, cancer du pancréas ; cancers des voies urinaires y compris rein, urothélium et vessie ; cancers du tractus génital féminin y compris cancer de l'utérus, du col de l'utérus, des ovaires, chloriocarcinome et trophoblastome ; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs malignes hématopoïétiques ; leucémies, (Acute Lymphocytic Leukemia (ALL), Acute Myeloid Leukemia (AML), Chronic Myeloid Leukemia (CML), Chronic lymphocytic leukemia (CLL)) chloromes, plasmocytomes, leucémies des cellules T ou B, lymphomes non hodgkiniens ou hodgkiniens, myélomes, hémopathies malignes diverses.

Les composés selon l'invention peuvent donc être utilisés pour la préparation de médicaments, en particulier de médicaments inhibiteurs de la caséine kinase 1 epsilon et/ou de la caséine kinase 1 delta.

Ainsi, selon un autre de ses aspects, l'invention a pour objet des médicaments qui comprennent un composé de formule (I), ou un sel d'addition de ce dernier à un acide pharmaceutiquement acceptable ou encore un hydrate ou un solvate du composé de formule (I).

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention ou un sel pharmaceutiquement acceptable, un hydrate ou solvate dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intraveineuse, topique, locale, intra trachéale, intra nasale, transdermique ou rectale, le principe actif de formule (I) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intra trachéale, intraoculaire, intra nasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,1 à 20 mg/kg, en une ou plusieurs prises.
Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Est également décrit une méthode de traitement des pathologies ci-dessus indiquées qui comprend l'administration, à un patient, d'une dose efficace d'un composé selon l'invention, ou un de ses sels pharmaceutiquement acceptables ou hydrates ou solvates.

## Revendications

1. Composé de formule générale (I) 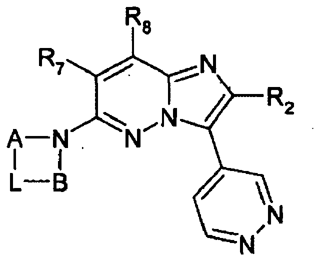 dans laquelle
- R₂ représente un groupe aryle éventuellement substitué par un ou plusieurs substituants choisis parmi les atomes d'halogène et les groupes C₁₋6 alkyle, C₁₋6 alkyloxy, C₁₋₆ alkylthio, C₁₋₆ fluoroalkyle, C₁₋₆-fluoroalkyloxy, -CN ;
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente, soit un atome d'azote éventuellement substitué par un groupe R_{c} ou R_{d}, soit un atome de carbone substitué par un groupe Rₑ₁ et un groupe R_{d} ou deux groupes Rₑ₂ ;
les atomes de carbone de A et de B étant éventuellement substitués par un ou plusieurs groupes R_{f} identiques ou différents l'un de l'autre ;
Rₐ, R_{b} et R_{c} sont définis tels que :
deux groupes Rₐ peuvent former ensemble un groupe C₁₋₆-alkylène ;
Rₐ et R_{b} peuvent former ensemble une liaison ou un groupe C₁₋₆-alkylène ;
Rₐ et R_{c} peuvent former ensemble une liaison ou un groupe C₁₋₆-alkylène ;
R_{b} et R_{c} peuvent former ensemble une liaison ou un groupe C₁₋₆-alkylène ;
R_{d} représente un groupe choisi parmi l'atome d'hydrogène et les groupes C₁₋₆-alkyle, C₃₋₇-cycloalkyle, C₃₋₇-cycloalkyl-C₁₋₆-alkyle, hydroxy-C₁₋₆-alkyle, C₁₋₆-alkyloxy-C₁₋₆-alkyle, C₁₋₆-alkylthio-C-₁₋₆-alkyle, C₁₋₆-fluoroalkyle, benzyle ;
Rₑ₁ représente un groupe -NR₄R₅ ou une monoamine cyclique comportant éventuellement un atome d'oxygène, la monoamine cyclique étant éventuellement substituée par un ou plusieurs substituant choisis parmi l'atome de fluor et les groupes C₁₋₆-alkyle, C₁₋₈-alkyloxy, hydroxyle ;
Deux Rₑ₂ forment avec l'atome de carbone qui les porte une monoamine cyclique comportant éventuellement un atome d'oxygène, cette monoamine cyclique étant éventuellement substituée par un ou plusieurs groupes R_{f} identiques ou différents l'un de l'autre ;
R_{f} représente un groupe C₁₋₆-alkyle, C₃₋₇-cycloalkyle, C₃₋₇Cycloalkyle-C₁₋₆alkyle, C₁₋₆-alkyloxy-C₁₋₆-alkyle, hydroxy-C₁₋₆-alkyle, C₁₋₆-fluoroalkyle ou benzyle ;
R₄ et R₅ représentent, indépendamment l'un de l'autre, un atome d'hydrogène ou un groupe C₁₋₆-alkyle, C₃₋₇-cycloalkyle, C₃₋₇-cycloalkyle-C₁₋₆-alkyle ;
- R₇ et R₈ représentent, indépendamment l'un de l'autre, un atome d'hydrogène ou un groupe un groupe C₁₋₆-alkyle ;
à l'état de base ou de sel d'addition à un acide.

2. Composé de formule générale (I) selon la revendication 1, **caractérisé en ce que** R₂ représente un phényle, éventuellement substitué par un ou plusieurs atomes d'halogène ou groupes C₁₋₈ alkyle, C₁₋₈ fluoroalkyle.

3. Composé de formule générale (I) selon l'une quelconque des revendications 1 ou 2, caractérisé en ce R₂ représente un phényle, 3-fluoro-phényle, 4-fluoro-phényle, 3,4-difluoro-phényle, 3,5-difluoro-phényle, 3,5-dichloro-phényle, 3,5-diméthyl-phényle, 3,5-di(trifluorométhyl)-phényle.

4. Composé de formule générale (I) selon l'une quelconque des revendications 1 à 3, **caractérisé en ce que** R₇ et R₈ représentent, indépendamment l'un de l'autre, un atome d'hydrogène ou un groupe méthyle.

5. Composé de formule générale (I) selon l'une quelconque des revendications 1 à 4, **caractérisé en ce que** :
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente un atome d'azote éventuellement substitué par un groupe R_{c} ou R_{d} ;
les atomes de carbone de A et de B étant éventuellement substitués par un ou plusieurs groupes R_{f} identiques ou différénts l'un de l'autre ;
- Rₐ et R_{b} peuvent former ensemble une liaison ou un groupe C₁₋₆-alkylène;
- Rₐ et R_{c} peuvent former ensemble une liaison ou un groupe C₁₋₆-alkylène ;
- R_{b} et R_{c} peuvent former ensemble une liaison ou un groupe C₁₋₆-alkylène ;
- R_{d} représente un substituant choisi parmi un groupe C₁₋₆alkyle, C₃₋₇-cycloalkyle, C₃₋₇-cycloalkyle-C₁₋₈-alkyle, hydroxy-C₁₋₆-alkyle, C₁₋₆-alkyloxy-C₁₋₆-alkyle, C₁₋₆-fluoroalkyle, benzyle,
- R_{f} représente un groupe C₁₋₆-alkyle, hydroxy-C₁₋₆-alkyle, C₁₋₆-alkyloxy-C₁₋₆-alkyle, C₁₋₆-fluoroalkyle.

6. Composé de formule générale (I) selon l'une quelconque des revendications 1 à 4, **caractérisé en ce que** l'amine cyclique formée par N-A-L-B- représente un groupe pipérazin-1-yle, 3-(*R*)-méthyl-pipérazin-1-yle, 3,3-diméthyl-pipérazin-1-yle, *cis*-3,5-diméthyl-pipérazin-1-yle, 4-méthyl-pipérazin-1-yle, 4-(2-hydroxy-éthyl)-pipérazin-1-yle, 4-(2-fluoroéthyl)-pipérazin-1-yle, 4-isopropyl-pipérazin-1-yle, 4-cyclopropyl-pipérazin-1-yle, 4-benzyl-pipérazin-1-yle, 4-(2-hydroxy-2-méthylpropyl)-pipérazin-1-yle, 4-(3-hydroxy-3-méthylbutyl)-pipérazin-1-yle, (*S*)-octahydro-pyrrolo[1,2-a]pyrazin-2-yle, (1*S*,4*S*)-2,5-diazabicyclo[2.2.1]hept-2-yle, 5-(2-hydroxy-éthyl)-(1*S*,4*S*)-2,5-diaza-bicyclo[2.2.1]hept-2-yle, 5-isopropyl-(1*S*,4*S*)-2,5-diazabicyclo[2.2.1]hept-2-yle, hexahydro-pyrrolo[3,4-c]pyrrol-2(1*H*)-yle, 5-méthyl-hexahydro-pyrrolo[3,4-*c*]pyrrol-2(1*H*)-yle, 5-(2-isopropyl)-hexahydropyrrolo[3,4-*c*]pyrrol-2(1*H*-yle, 5-(2-hydroxy-éthyl)-hexahydropyrrolo[3,4-*c*]pyrrol-2(1*H*)-yle, 5-(benzyl)-hexahydro-pyrrolo[3,4-c]pyrrol-2(1*H*)-yle, 3-(hydroxyméthyl)-pipérazin-1-yle, 3-fluorométhyl-pipérazin-1-yle.

7. Composé de formule générale (I) selon l'une quelconque des revendications 1 à 4, **caractérisé en ce que** :
- A représente un groupe C₁₋₇-alkylène ;
- B représente un groupe C₁₋₇-alkylène;
- L représente un atome de carbone substitué par deux groupes Rₑ₂ ;
les atomes de carbone de A et de B étant éventuellement substitués par un ou plusieurs groupes R_{f} identiques ou différents l'un de l'autre ;
- R_{f} représente un groupe C₁₋₆-alkyle ;
- Deux Rₑ₂ forment, avec l'atome de carbone qui les porte, un groupe azétidine, pyrrolidine, pipéridine ou morpholine.

8. Composé de formule générale (I) selon l'une quelconque des revendications 1 à 4, **caractérisé en ce que** l'amine cyclique formée par -N-A-L-B- représente un groupe 2,9-diaza-spiro[5.5]undéc-3-yle, 1-oxa-4,9-diazaspiro[5.5]undéc-9-yle.

9. Composé de formule générale (I) selon l'une quelconque des revendications 1 à 4, **caractérisé en ce que** :
- A représente un groupe C₁₋₇-alkylène ;
- B représente un groupe C₁₋₇alkylène :
- L représente un atome de carbone substitué par un groupe Rₑ₁ et un groupe R_{d} ;
- R_{d} représente un atome d'hydrogène;
- Rₑ₁ représente un groupe -NR₄R₅ ou une monoamine cyclique;
- R₄ et R₅ représentent, indépendamment l'un de l'autre, un atome d'hydrogène ou un groupe C₁₋₆-alkyle, C₃₋₇-cycloalkyle, C₃₋₇-cycloalkyle-C₁₋₆-alkyle.

10. Composé de formule générale (I) selon l'une quelconque des revendications 1 à 4, **caractérisé en ce que** :
- l'amine cyclique formée par -N-A-L-B- représente un groupe (*R*,*S*)-3-[pyrrolidin-1-yl]-pyrrolidin-1-yle ou 4-(pyrrolidin-1-yl)-pipéridin-1-yl.

11. Composé de formule générale (I) selon la revendication 1, **caractérisé en ce que** :
- R₂ représente un groupe phényle, 3-fluoro-phényle, 4-fluoro-phényle, 3,4-difluoro-phényle, 3,5-difluoro-phényle, 3,5-dichloro-phényle, 3,5-diméthyl-phényle, 3,5-di(trifluorométhyl)-phényle ;
- l'amine cyclique formée par -N-A-L-B- représente un groupe pipérazin-1-yle, 3-(*R*)-méthyl-pipérazin-1-yle, 3,3-diméthyl-pipérazin-1-yle, *cis*-3,5-diméthyl-pipérazin-1-yle, 4-méthyl-pipérazin-1-yle, 4-(2-hydroxy-éthyl)-pipérazin-1-yle, 4-(2-fluoroéthyl)-pipérazin-1-yle, 4-isopropyl-pipérazin-1-yle, 4-cyclopropyl-pipérazin-1-yle, 4-benzyl-pipérazin-1-yle, 4-(2-hydroxy-2-méthylpropyl)-pipérazin-1-yle, 4-(3-hydroxy-3-méthylbutyl)-pipérazin-1-yle, (*S*)-octahydro-pyrrolo[1,2-*a*]pyrazin-2-yle, (1*S*,4*S*)-2,5-diazabicyclo[2.2.1]hept-2-yle, 5-(2-hydroxy-éthyl)-(1*S*,4*S*)-2,5-diaza-bicyclo[2.2.1]hept-2-yle, 5-isopropyl-(1*S*,4*S*)-2,5-diazabicyclo[2.2.1]hept-2-yle, hexahydro-pyrrolo[3,4-*c*]pyrrol-2(1*H*)-yle, 5-méthyl-hexahydro-pyrrolo[3,4-*c*]pyrrol-2(1*H*)-yle, 5-(2-isopropyl)-hexahydropyrrolo[3,4-*c*]pyrrol-2(1*H*)-yle, 5-(2-hydroxy-éthyl)-hexahydropyrrolo[3,4-*c*]pyrrol-2(1*H*)-yle, 5-(benzyl)-hexahydro-pyrrolo[3,4-*c*]pyrrol-2(1*H*-yle, 3-(hydroxyméthyl)-pipérazin-1-yle,3-fluorométhyl-pipérazin-1-yle ;
- R₇ et R₈ représentent, indépendamment l'un de l'autre, un atome d'hydrogène ou un groupe un groupe méthyle.

12. Composé de formule générale (I) selon la revendication 1, **caractérisé en ce que** :
- R₂ représente un groupe 4-fluoro-phényle, 3,5-difluoro-phényle, 3,5-dichloro-phényle, 3,5-diméthyl-phényle, 3,5-di(trifluorométhyl)-phényle ;
- l'amine cyclique formée par -N-A-L-B- représente un groupe (*R*,*S*)-3-[pyrrolidin-1-yl]-pyrrolidin-1-yle ou 4-(pyrrolidin-1-yl)-pipéridin-1-yle ;
- R₇ et R₈ représentent un atome d'hydrogène.

13. Composé de formule générale (I) selon la revendication 1, **caractérisé en ce qu'**il est choisi parmi les composés suivants :
2-(4-Fluoro-phényl)-6-pipérazin-1-yl-3-pyridazin-4-yl-imidazo[1,2-*b*]pyridazine ;
2-(4-Fluoro-phényl)-6-((*R*)-3-méthyl-pipérazin-1-yl)-3-pyridazin-4-yl-imidazo[1,2-*b*]pyridazine ;
6-(3,3-Diméthyl-pipérazin-1-yl)-2-(4-fluoro-phényl)-3-pyridazin-4-yl-imidazo[1,2-*b*]pyridazine ;
6-((*cis*)-3,5-Diméthyl-pipérazin-1-yl)-2-(4-fluoro-phényl)-3-pyridazin-4-yl-imidalzo[1,2-*b*]pyridazine ;
2-(4-Fluoro-phényl)-6-(4-méthyl-pipérazin-1-yl)-3-pyridazin-4-yl-imidazo[1,2-*b*]pyridazine
2-{4-[2-(4-Fluoro-phényl)-3-pyndazin-4-yl-imidazo[1,2-*b*]pyridazin-6-yl]-pipérazin-1-yl}-éthanol ;
6-[4-(2-Fluoro-éthyl)-pipérazin-1-yl]-2-phényl-3-pyridazin-4-yl-imidazo[1,2-*b*]pyridazine ;
6-[4-(2-Fluora-éthyl)-pipérazin-1-yl]-2-(4-fluoro-phényl)-3-pyridazin-4-yl-imidazo[1,2-*b*]pyridazine ;
6-{4-Isopropyl-pipérazin-1-yl)-2-phényl-3-pyridazin-4-yl-imidazo[1,2-*b*]pyridazine ;
2-(3-Fluoro-phényl)-6-(4-isopropyl-pipérazin-1-yl)-3-pyridazin-4-yl-imidazo[1,2-*b*]pyridazine ;
2-(4-Fluoro-phényl)-6-(4-isopropyl-pipérazin-1-yl)-3-pyridazin-4-yl-imidazo[1,2-*b*]pyridazine ;
2-(4-Fluoro-phényl)-6-(4-isopropyl-pipérazin-1-yl)-7,8-diméthyl-3-pyridazin-4-yl-imidazo[1,2-*b*]pyridazine ;
2-(3,5-Diméthyl-phényl)-6-(4-isopropyl-pipérazin-1-yl)-3-pyridazin-4-yl-imidazo[1,2-*b*]pyridazine ;
2-(3,4-Difluoro-phényl)-6-(4-isopropyl-pipérazin-1-yl)-3-pyridazin-4-yl-imidazo[1,2-*b*]pyridazine ;
2-(3,5-Difluoro-phényl)-6-(4-isopropyl-pipérazin-1-yl)-3-pyridazin-4-yl-imidazo[1,2-*b*]pyridazine ;
2-(3,5-Dichloro-phényl)-6-(4-isopropyl-pipérazin-1-yl)-3-pyridazin-4-yl-imidazo[1,2-*b*]pyridazine ;
2-(3,5-Di(trifluorométhyl)-phényl)-6-(4-isopropyl-pipérazin-1-yl)-3-pyridazin-4-yl-imidazo[1,2-*b*]pyridazine ;
6-(4-Cyclopropyl-pipérazin-1-yl)-2-(4-fluoro-phényl)-3-pyridazin-4-yl-imidazo[1,2-*b*]pyridazine ;
6-(4-Benzyl-pipérazin-1-yl)-2-(4-fluoro-phényl)-3-pyridazin-4-yl-imidazo[1,2-*b*]pyridazine ;
1-{4-[2-(4-Fluoro-phényl)-3-pyridazin-4-yl-imidazo[1,2-*b*]pyridazin-6-yl]-pipérazin-1-yl}-2-méthyl-propan-2-ol ;
4-{4-[2-(4-Fluoro-phényl)-3-pyridazin-4-yl-imidazo[1,2-*b*]pyridazin-6-yl]-pipérazin-1-yl}-2-méthyl-butan-2-ol ;
2-(4-Fluoro-phényl)-6-(*S*)-hexahydro-pyrrolo[1,2-*a*]pyrazin-2-yl-3-pyridazin-4-yl-imidazo[1,2-*b*]pyridazine ;
6-((1*S*,4*S*)-2,5-Diaza-bicyclo[2.2.1]hept-2-yl)-2-(4-fluoro-phényl)-3-pyridazin-4-yl-imidazo[1,2-*b*]pyridazine ;
2-{5-[2-(4-Fluoro-phényl)-3-pyridazin-4-yl-imidazo[1,2-*b*]pyridazin-6-yl]-(1*S*,4*S*)-2,5-diaza-bicyclo[2.2.1]hept-2-yl}-éthanol ;
2-(4-Fluoro-phényl)-6-(5-isopropyl-(1*S*,4*S*)-2,5-diaza-bicyclo[2.2.1]hept-2-yl)-3-pyridazin-4-yl-imidazo[1,2-*b*]pyridazine ;
2-(4-Fluoro-phényl)-6-hexahydro-pyrrolo[3,4-*c*]pyrrol-2(1*H*)-yl-3-pyridazin-4-yl-imidazo[1,2-*b*]pyridazine ;
2-(4-Fluoro-phényl)-6-(5-méthyl-hexahydro-pyrrolo[3,4-*c*]pyrrol-2(1*H*)-yl)-3-pyridazin-4-yl-imidazo[1,2-*b*]pyridazine ;
2-(4-Fluoro-phényl)-6-(5-isopropyl-hexahydro-pyrrolo[3,4-*c*]pyrrol-2(1*H*-yl)-3-pyrdazin-4-yl-imidazo[1,2-*b*]pyridazine ;
2-{(5-[2-(4-Fluoro-phényl)-3-pyridazin-4-yl-imidazo[1,2-*b*]pyridazin-6-yl]-hexahydro-pyrrolo[3,4-*c*]pyrro(-2(1*H*)-yl}-éthanol ;
2-(4-Fluoro-phényl)-6-(5-benzyl-hexahydro-pyrrolo[3,4-*c*]pyrrol-2(1*H*)-yl)-3-pyridazin-4-yl-imidazo[1,2-*b*]pyridazine ;
9-[2-(4-Fluoro-phényl)-3-pyridazin-4-yl-imidazo[1,2-*b*]pyridazin-6-yl]-2,9-diaza-spiro[5.5]undécane ;
9-[2-(4-Fluoro-phényl)-3-pyridazin-4-yl-imidazo[1,2-*b*]pyridazin-6-yl]-1-oxa-4,9-diaza-spiro[5.5]undécane ;
6-[1,3']Bipyrrolidinyl-1'-yl-2-(4-fluoro-phényl)-3-pyridazin-4-yl-imidazo[1,2-*b*]pyridazine ;
2-(4-Fluoro-phényl)-3-pyridazin-4-yl-6-(4-pyrrolidin-1-yl-pipéridin-1-yl)-imidazo[1,2-*b*]pyridazine ;
2-(3,5-Diméthyl-phényl)-3-pyridazin-4-yl-6-(4-pyrrolidin-1-yl-pipéridin-1-yl)-imidazo[1,2-*b*]pyridazine;
2-(3,5-Difluoro-phényl)-3-pyridazin-4-yl-6-(4-pyrrolidin-1-yl-pipéridin-1-yl)-imidazo[1,2-*b*]pyridazine ;
2-(3,5-Dichloro-phényl)-3-pyridazin-4-yl-6-(4-pyrrolidin-1-yl-pipéridin-1-yl)-imidazo(1,2-*b*]pyridazine ;
2-(3,5-Di(trifluorométhyl)-phényl)-3-pyridazin-4-yl-6-(4-pyrrolidin-1-yl-pipéridin-1-yl)-imidazo[1,2-*b*]pyridazine ;
(+/-)-{4-[2-(4-Fluoro-phényl)-3-pyridazin-4-yl-imidazo[1,2-*b*]pyridazin-6-yl]-pipérazin-2-yl}-méthanol ;
(+/-)-6-(3-Fluorométhyl-pipérazin-1-yl)-2-(4-fluoro-phényl)-3-pyridazin-4-yl-imidazo[1,2-*b*]pyridazine ;
6-(5-Méthyl-hexahydro-pyrrolo[3,4-c]pyrrol-[3,4-*c*]pyrrol-2(1*H*)-2-phényl-3-pyridazin-4-yl-imidazo[1,2-*b*]pyridazine ;
2-(3-Fluoro-phényl)-6-(5-méthyl-hexahydro-pyrrolo[3,4-*c*]pyrrol-2(1*H*)-yl)-3-pyridazin-4-yl-imidazo[1,2-*b*]pyridazine ;
2-(4-Fluoro-phényl)-7-méthyl-6-(5-méthyl-hexahydro-pyrrolo[3,4-c]pyrrol-2(1*H*-yl)-3-pyridazin-4-yl-imidazo[1,2-*b*]pyridazine ;
2-(4-Fluoro-phényl)-8-méthyl-6-(5-méthyl-hexahydro-pyrrolo[3,4-*c*]pyrrol-2(1*H*)-yl)-3-pyridazin-4-yl-imidazo[1,2-*b*]pyridazine ;
2-(3,4-Difluoro-phényl)-6-(5-méthyl-hexahydro-pyrrolo[3,4-c]pyrrol-2(1*H*)-yl)-3-pyridazin-4-yl-imidazo[1,2-b]pyridazine.

14. Procédé de préparation d'un composé de formule (I) selon la revendication 1, **caractérisé en ce que** l'on fait réagir un composé de formule (IIa) 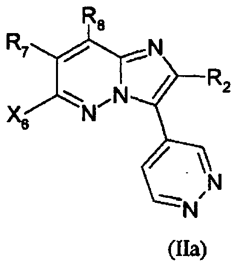 dans laquelle R₂, R₇ et R₈ sont tels que définis selon la revendication 1 et X₆ représente un groupe partant tel qu'un halogène, avec une amine de formule générale (IIb) 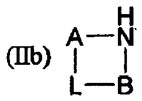 dans laquelle N, L, A et B sont tels que définis selon la revendication 1, dans un solvant polaire.

15. Procédé de préparation d'un composé de formule (I) selon la revendication 1, **caractérisé en ce que** l'on fait réagir un composé de formule (IIIa) 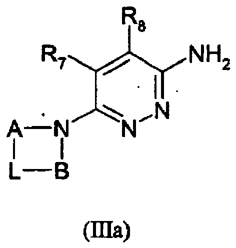 dans laquelle A, L, B, R₇ et R₈ sont tels que définis selon la revendication 1, avec un dérivé 2-bromo-2-(pyridazin-4-yl)-éthan-1-one de formule générale (IIIb) 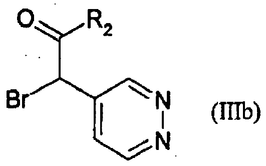 dans laquelle R₂ est tel que défini selon la revendication 1, dans un solvant polaire.

16. Procédé de préparation d'un composé de formule (I) selon la revendication 1, **caractérisé en ce que** l'on fait réagir un composé de formule générale (IVa) 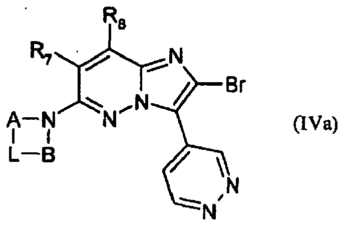 dans laquelle A, L, B, R₇ et R⁸ sont tels que définis selon la revendication 1, avec un dérivé d'aryle de formule générale (IVb)
M-R₂ (IVb)
dans laquelle R₂ est tel que défini selon la revendication 1 et M représente un groupe trialkylstannyle ou un groupe dihydroxyboryle ou dialkyloxyboryle, en présence d'un catalyseur dans un solvant.

17. Procédé de préparation d'un composé de formule (I) selon la revendication 1, **caractérisé en ce qu'**il comprend les étapes suivantes :
a) on fait réagir un composé de formule (V) 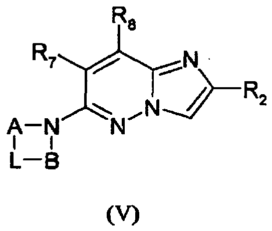 dans laquelle R₂, A, L, B, R₇ et R₈ sont tels que définis selon la revendication 1, avec de la pyridazine et un chloroformiate d'alkyle pour obtenir un composé de formule générale (VI) 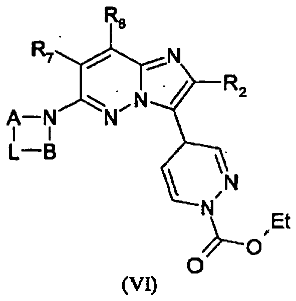 dans laquelle R₂, A, L, B, R₇ et R₈ sont tels que définis selon la revendication 1 ;
b) on fait réagir le composé de formule générale (VI) obtenu à l'étape précédente avec de l'ortho-chloranile dans un solvant.

18. Médicament, **caractérisé en ce qu'**il comprend un composé de formule (I) selon l'une quelconque des revendications 1 à 13, à l'état de base ou de sel d'addition à un acide pharmaceutiquement acceptable.

19. Composition pharmaceutique, **caractérisée en ce qu'**elle comprend un composé de formule (I) selon l'une quelconque des revendications 1 à 13, à l'état de base ou de sel d'addition à un acide pharmaceutiquement acceptable, ainsi qu'au moins un excipient pharmaceutiquement acceptable:

20. Composé de formule générale (I) selon l'une quelconque des revendications 1 à 13, pour son utilisation pour le traitement ou la prévention des désordres du sommeil ; des troubles du rythme circadien.

21. Composé de formule générale (I) selon l'une quelconque des revendications 1 à 13, pour son utilisation pour le traitement ou la prévention des maladies liées à la dépendance à des substances d'abus.

22. Composé de formule générale (I) selon l'une quelconque des revendications 1 à 13, pour son utilisation pour le traitement ou la préventionde la maladie d'Alzheimer.

23. Composé de formule générale (I) selon l'une quelconque des revendications 1 à 13, pour son utilisabon pour le traitement ou la prévention des maladies causées ou exacerbées par la prolifération des cellules.

24. Composé de formule générale (I) selon la revendication 23, **caractérisé en ce que** les cellules sont des cellules tumorales.

## Patentansprüche

1. Verbindung der allgemeinen Formel (I) 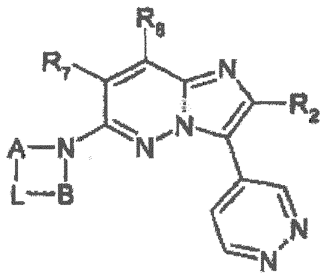 worin
- R₂ für eine Arylgruppe, die gegebenenfalls durch einen oder mehrere Substituenten, die aus Halogenatomen und C₁₋₆-Alkyl-, C₁₋₆-Alkyloxy-, C₁₋₆-Alkylthio, C₁₋₆-Fluoralkyl-, C₁₋₆-Fluoralkyloxy und -CN-Gruppen ausgewählt sind, substituiert ist, steht;
- A für eine C₁₋₇-Alkylengruppe, die gegebenenfalls durch eine oder zwei Gruppen Rₐ substituiert ist, steht;
- B für eine C₁₋₇-Alkylengruppe, die gegebenenfalls durch eine Gruppe R_{b} substituiert ist, steht;
- L entweder für ein Stickstoffatom, das gegebenenfalls durch eine Gruppe R_{c} oder R_{d} substituiert ist, oder für ein Kohlenstoffatom, das durch eine Gruppe Rₑ₁ und eine Gruppe R_{d} oder zwei Gruppen Rₑ₂ substituiert ist, steht;
wobei die Kohlenstoffatome von A und B gegebenenfalls durch eine oder mehrere Gruppen R_{f}, die gleich oder voneinander verschieden sein können, substituiert sind;
- Rₐ, R_{b} und Rₑ so definiert sind, dass:
zwei Gruppen Rₐ zusammen eine C₁₋₆-Alkylengruppe bilden können;
Rₐ und R_{b} zusammen eine Bindung oder eine C₁₋₆-Alkylengruppe bilden können;
Rₐ und R_{c} zusammen eine Bindung oder eine C₁₋₆-Alkylengruppe bilden können;
R_{b} und R_{c} zusammen eine Bindung oder eine C₁₋₆-Alkylengruppe bilden können;
- R_{d} für eine Gruppe steht, die aus einem Wasserstoffatom und C₁₋₆-Alkyl-, C₃₋₇-Cycloalkyl-C₃₋₇-Cycloalkyl-C₁₋₆-alkyl-, Hydroxy-C₁₋₆-alkyl-, C₁₋₆-Alkyloxy-C₁₋₆-alkyl-, C₁₋₆-Alkylthio-C₁₋₆-alkyl-, C₁₋₆-Fluoralkyl- und Benzylgruppen ausgewählt ist;
- Rₑ₁ für eine Gruppe -NR₄R₅ oder ein cyclisches Monoamin, das gegebenenfalls ein Sauerstoffatom umfasst, steht, wobei das cyclische Monoamin gegebenenfalls durch einen oder mehrere Substituenten, die aus einem Fluoratom und C₁₋₆-Alkyl-, C₁₋₆-Alkyloxy- und Hydroxylgruppen ausgewählt sind, substituiert ist;
- zwei Gruppen Rₑ₂ mit dem Kohlenstoffatom, das sie trägt, ein cyclisches Monoamin, das gegebenenfalls ein Sauerstoffatom umfasst, bilden, wobei dieses cyclische Monoamin gegebenenfalls durch eine oder mehrere Gruppen R_{f}, die gleich oder voneinander verschieden sein können, substituiert ist;
- R_{f} für eine C₁-₆-Alkyl-, C₃₋₇Cycloalkyl-, C₃₋₇-Cycloalkyl-C₁₋₆-alkyl-, C₁₋₆-Alkyloxy-C₁₋₆-alkyl-, Hydroxy-C₁₋₆-alkyl-, C₁₋₆-Fluoralkyl- oder Benzylgruppe steht;
- R₄ und R₅ unabhängig voneinander für ein Wasserstoffatom oder eine C₁₋₆-Alkyl-, C₃₋₇-Cycloalkyl- oder C₃₋₇-Cycloalkyl-Cᵢ-₆-alkylgruppe stehen;
- R₇ und R₈ unabhängig voneinander für ein Wasserstoffatom oder eine C₁₋₆-Alkylgruppe stehen; in Basenform oder in Form eines Säureadditionssalzes.

2. Verbindung der allgemeinen Formel (I) nach Anspruch 1, **dadurch gekennzeichnet, dass** R₂ für ein Phenyl, das gegebenenfalls durch ein oder mehrere Halogenatome oder eine oder mehrere C₁₋₆-Alkyl-oder C₁₋₆-Fluoralkylgruppen substituiert ist.

3. Verbindung der allgemeinen Formel (I) nach einem der Ansprüche 1 oder 2, **dadurch gekennzeichnet, dass** R₂ für ein Phenyl, 3-Fluorphenyl, 4-Fluorphenyl, 3,4-Difluorphenyl, 3,5-Difluorphenyl, 3,5-Dichlorphenyl, 3,5-Dimethylphenyl oder 3,5-Di(trifluormethyl)phenyl steht.

4. Verbindung der allgemeinen Formel (I) nach einem der Ansprüche 1 bis 3, **dadurch gekennzeichnet, dass** R₇ und R₈ unabhängig voneinander für ein Wasserstoffatom oder eine Methylgruppe stehen.

5. Verbindung der allgemeinen Formel (I) nach einem der Ansprüche 1 bis 4, **dadurch gekennzeichnet, dass**:
- A für eine C₁₋₇-Alkylengruppe, die gegebenenfalls durch eine oder zwei Gruppen Rₐ substituiert ist, steht;
- B für eine C₁₋₇-Alkylengruppe, die gegebenenfalls durch eine Gruppe R_{b} substituiert ist, steht;
- L für ein Stickstoffatom, das gegebenenfalls durch eine Gruppe R_{c} oder R_{d} substituiert ist, steht;
wobei die Kohlenstoffatome von A und B gegebenenfalls durch eine oder mehrere Gruppen R_{f}, die gleich oder voneinander verschieden sein können, substituiert sind;
- Rₐ und R_{b} zusammen eine Bindung oder eine C₁₋₆-Alkylengruppe bilden können;
- Rₐ und R_{c} zusammen eine Bindung oder eine C₁₋₆-Alkylengruppe bilden können;
- R_{b} und R_{c} zusammen eine Bindung oder eine C₁₋₆-Alkylengruppe bilden können;
- R_{d} für einen Substituenten steht, der aus einer C₁₋₆-Alkyl-, C₃₋₇-Cycloalkyl-, C₃₋₇-Cycloalkyl-C₁₋₆-alkyl-, Hydroxy-C₁₋₆-alkyl-, C₁₋₆-Alkyloxy-C₁₋₆-alkyl-, C₁₋₆-Fluoralkyl- oder Benzylgruppe ausgewählt ist;
- R_{f} für eine C₁₋₆-Alkyl-, Hydroxy-C₁₋₆-alkyl-, C₁₋₆-Alkyloxy-C₁₋₆-alkyl- oder C₁₋₆-Fluoralkylgruppe steht.

6. Verbindung der allgemeinen Formel (I) nach einem der Ansprüche 1 bis 4, **dadurch gekennzeichnet, dass** das durch -N-A-L-B- gebildete cyclische Amin für eine Piperazin-1-yl-, 3-(*R*)-Methylpiperazin-1-yl-, 3,3-Dimethylpiperazin-1-yl-, *cis*-3,5-Di-methylpiperazin-1-yl-, 4-Methylpiperazin-1-yl-, 4-(2-Hydroxyethyl)piperazin-1-yl-, 4-(2-Fluorethyl)-piperazin-1-yl-, 4-Isopropylpiperazin-1-yl-, 4-Cyclopropylpiperazin-1-yl-, 4-Benzylpiperazin-1-yl-, 4-(2-Hydroxy-2-methylpropyl)piperazin-1-yl-, 4-(3-Hydroxy-3-methylbutyl)piperazin-1-yl-, (*S*)-Octahydropyrrolo[1,2-*a*]pyrazin-2-yl-, (1*S*,4*S*)-2,5-Diazabicyclo[2.2.1]hept-2-yl-, 5-(2-Hydroxyethyl)-(1*S*,4*S*)-2,5-diazabicyclo[2.2.1]hept-2-yl-, 5-Isopropyl-(1*S*,4*S*)-2,5-diazabicyclo[2,2.1]hept-2-yl-, Hexahydropyrrolo[3,4-c]pyrrol-2-(1*H*)-yl-, 5-Methylhexahydropyrrolo[3,4-c]pyrrol-2-(1*H*)-yl-, 5-(2-Isopropyl)hexahydropyrrolo[3,4-*c*]pyrrol-2-(1*H*)-yl-, 5-(2-Hydroxyethyl)hexahydropyrrolo[3,4-*c*]-pyrrol-2-(1*H*)-yl-, 5-(Benzyl)hexahydro-pyrrolo-[3,4-*c*]pyrrol-2-(1*H*)-yl-, 3-(Hydroxymethyl)-piperazin-1-yl- oder 3-Fluormethylpiperazin-1-yl-gruppe steht.

7. Verbindung der allgemeinen Formel (I) nach einem der Ansprüche 1 bis 4, **dadurch gekennzeichnet, dass**:
- A für eine C₁₋₇-Alkylengruppe steht;
- B für eine C₁₋₇-Alkylengruppe steht;
- L für ein Kohlenstoffatom, das durch zwei Gruppen Rₑ₂ substituiert ist, steht;
wobei die Kohlenstoffatome von A und B gegebenenfalls durch eine oder mehrere Gruppen R_{f}, die gleich oder voneinander verschieden sein können, substituiert sind;
- R_{f} für eine C₁₋₆-Alkylgruppe steht;
- zwei Gruppen Rₑ₂ mit dem Kohlenstoffatom, das sie trägt, eine Azetidin-, Pyrrolidin-, Piperidin-oder Morpholingruppe bilden.

8. Verbindung der allgemeinen Formel (I) nach einem der Ansprüche 1 bis 4, **dadurch gekennzeichnet, dass** das durch -N-A-L-B- gebildete cyclische Amin für eine 2,9-Diazaspiro[5.5]undec-3-yl- oder 1-Oxa-4,9-diazaspiro[5.5]undec-9-ylgruppe steht.

9. Verbindung der allgemeinen Formel (I) nach einem der Ansprüche 1 bis 4, **dadurch gekennzeichnet, dass**:
- A für eine C₁₋₇-Alkylengruppe steht;
- B für eine C₁₋₇-Alkylengruppe steht;
- L für ein Kohlenstoffatom, das durch eine Gruppe Rₑ₁ und eine Gruppe R_{d} substituiert ist, steht;
- R_{d} für ein Wasserstoffatom steht;
- Rₑ₁ für eine Gruppe -NR₄R₅ oder ein cyclisches Monoamin steht;
- R₄ und R₅ unabhängig voneinander für ein Wasserstoffatom oder eine C₁₋₆-Alkyl-, C₃₋₇-Cycloalkyl- oder C₃₋₇-Cycloalkyl-C₁₋₆-alkylgruppe stehen.

10. Verbindung der allgemeinen Formel (I) nach einem der Ansprüche 1 bis 4, **dadurch gekennzeichnet, dass**:
- das durch -N-A-L-B- gebildete cyclische Amin für eine (*R*,*S*)-3-[Pyrrolidin-1-yl]-pyrrolidin-1-yl-oder 4-(Pyrrolidin-1-yl)piperidin-1-ylgruppe steht.

11. Verbindung der allgemeinen Formel (I) nach Anspruch 1, **dadurch gekennzeichnet, dass**:
- R₂ für eine Phenyl-, 3-Fluorphenyl-, 4-Fluorphenyl-, 3,4-Difluorphenyl-, 3,5-Difluorphenyl-, 3,5-Dichlorphenyl-, 3,5-Dimethylphenyl-oder 3,5-Di(trifluormethyl)phenylgruppe steht;
- das durch -N-A-L-B- gebildete cyclische Amin für eine Piperazin-1-yl-, 3-(*R*)-Methylpiperazin-1-yl-, 3,3-Dimethylpiperazin-1-yl-, *cis*-3,5-Dimethyl-piperazin-1-yl, 4-Methylpiperazin-1-yl-, 4-(2-Hydroxyethyl)piperazin-1-yl-, 4-(2-Fluorethyl)-piperazin-1-yl-, 4-Isopropylpiperazin-1-yl-, 4-Cyclopropylpiperazin-1-yl-, 4-Benzylpiperazin-1-yl-, 4-(2-Hydroxy-2-methylpropyl)piperazin-1-yl-, 4-(3-Hydroxy-3-methylbutyl)piperazin-1-yl-, (*S*)-Octahydropyrrolo[1,2-a]pyrazin-2-yl-, (1*S*,4*S*)-2,5-Diazabicyclo[2.2.1]hept-2-yl-, 5-(2-Hydroxyethyl)-(1*S*,4*S*)-2,5-diazabicyclo[2.2.1]hept-2-yl-, 5-Isopropyl-(1*S*,4*S*)-2,5-diazabicycla[2.2.1]hept-2-yl-, Hexahydropyrrolo[3,4-c]pyrrol-2-(1*H*)-yl-, 5-Methylhexahydropyrrolo[3,4-c]pyrrol-2-(1*H*)-yl-, 5-(2-Isopropyl)hexahydropyrrolo[3,4-*c*]pyrrol-2-(1*H*)-yl-, 5-(2-Hydroxyethyl)hexahydropyrrolo[3,4-*c*]pyrrol-2-(1*H*)-yl-, 5-(Benzyl)hexahydro-pyrrolo-[3,4-*c*]pyrrol-2-(1*H*)-yl-, 3-(Hydroxymethyl). piperazin-1-yl- oder 3-Fluormethylpiperazin-1-yl-gruppe steht;
- R₇ und R₈ jeweils unabhängig voneinander für ein Wasserstoffatom oder eine Methylgruppe stehen.

12. Verbindung der allgemeinen Formel (I) nach Anspruch 1, **dadurch gekennzeichnet, dass**:
- R₂ für eine 4-Fluorphenyl-, 3,5-Difluorphenyl-, 3,5-Dichlorphenyl-, 3,5-Dimethylphenyl- oder 3,5-Di(trifluormethyl)phenylgruppe steht;
- das durch -N-A-L-B- gebildete cyclische Amin für eine (*R*,*S*)-3-[Pyrrolidin-1-yl]pyrrolidin-1-yl-oder 4-(Pyrrolidin-1-yl)piperidin-1-ylgruppe steht;
- R₇ und R₈ für ein Wasserstoffatom stehen.

13. Verbindung der allgemeinen Formel (I) nach Anspruch 1, **dadurch gekennzeichnet, dass** sie aus den folgenden Verbindungen ausgewählt ist:
2-(4-Fluorphenyl)-6-piperazin-1-yl-3-pyridazin-4-yl-imidaza[1,2-*b*]pyridazin;
2-(4-Fluorphenyl)-5-((*R*)-3-methylpiperazin-1-yl)-3-pyridazin-4-yl-imidazo[1,2-*b*]pyridazin;
6-(3,3-Dimethylpiperazin-2-yl)-2-(4-fluorphenyl)-3-pyridazin-4-yl-imidazo[1,2-*b*]pyridazin;
6-(*cis*-3,5-Dimethylpiperazin-1-yl)-2-(4-fluorphenyl)-3-pyridazin-4-yl-imidazo[1,2-*b*]pyridazin;
2-(4-Fluorphenyl)-5-(4-methylpiperazin-1-yl)-3-pyridazin-4-yl-imidazo[1,2-*b*]pyridazin;
2-{4-[2-(4-Fluorphenyl)-3-pyridazin-4-yl-imidazo[1,2-b]pyridazin-5-yl]piperazin-1-yl}-ethanol;
6-[4-(2-Fluorethyl)piperazin-1-yl]-2-phenyl-3-pyridazin-4-yl-imidazo[1,2-*b*]pyridazin;
6-[4-(2-Fluorethyl)piperazin-1-yl]-2-(4-fluorphenyl)-3-pyridazin-4-yl-imidazo[1,2-*b*]pyridazin;
6-(4-Isopropylpiperazin-2-yl)-2-phenyl-3-pyridazin-4-yl-imidazo[1,2-*b*]pyridazin;
2-(3-Fluorphenyl)-6-(4-isopropylpiperazin-1-yl)-3-pyridazin-4-yl-imidazo[1,2-*b*]pyridazin;
2-(4-Fluorphenyl)-6-(4-isopropylpiperazin-1-yl)-3-pyridazin-4-yl-imidazo[1,2-*b*]pyridazin;
2-(4-Fluorphenyl)-6-(4-isopropylpiperazin-1-yl)-7,8-dimethyl-3-pyridazin-4-yl-imidazo[1,2-*b*]-pyridazin;
2-(3,5-Dimethylphenyl)-6-(4-isopropylpiperazin-1-yl)-3-pyridazin-4-yl-imidazo[1,2-*b*]pyridazin;
2-(3,4-Difluorphenyl)-6-(4-isopropylpiperazin-1-yl)-3-pyridazin-4-yl-imidazo[1,2-*b*]pyridazin;
2-(3,5-Difluorphenyl)-5-(4-isopropylpiperazin-1-yl)-3-pyridazin-4-yl-imidazo[1,2-*b*]pyridazin;
2-(3,5-Dichlorphenyl)-6-(4-isopropylpiperazin-1-yl)-3-pyridazin-4-yl-imidazo[1,2-*b*]pyridazini;
2-(3,5-Di(trifluormethyl)phenyl)-6-(4-isopropyl-piperazin-1-yl)-3-pyridazin-4-yl-imidazo[1,2-*b*]-pyridazin;
6-(4-Cyclopropylpiperazin-1-yl)-2-(4-fluorphenyl)-3-pyridazin-4-yl-imidazo[1,2-*b*]pyridazin;
6-(4-Benzylpiperazin-1-yl)-2-(4-fluorphenyl)-3-pyridazin-4-yl-imidazo[1,2-*b*]pyridazin;
1-{4-[2-(4-Fluorphenyl)-3-pyridazin-4-yl-imidazo[1,2-*b*]pyridazin-6-yl]piperazin-1-yl}-2-methylpropan-2-ol;
4-{4-[2-(4-Fluorphenyl)-3-pyridazin-4-yl-imidazo[1,2-*b*]pyridazin-6-yl]piperazin-1-yl}-2-methylbutan-2-ol;
2-(4-Fluorphenyl)-6-(*S*)-hexahydropyrrolo[1,2-*a*]pyrazin-2-yl-3-pyridazin-4-yl-imidazo[1,2-*b*]-pyridazin;
6-((1*S*,4*S*)-2,5-Diazabicyclo[2.2.1]hept-2-yl)-2-(4-fluorphenyl)-3-pyridazin-4-yl-imidazo[1,2-*b*]pyridazin;
2-{5-[2-(4-Fluorphenyl)-3-pyridazin-4-yl-imidazo[1,2-*b*]pyridazin-6-yl]-(1*S*,4*S*)-2,5-diaza-bicyclo[2.2.1]hept-2-yl}ethanol;
2-(4-Fluorphenyl)-6-(5-isopropyl-(1*S*,4*S*)-2,5-diazabicyclo[2.2.1]hept-2-yl)-3-pyridazin-4-yl-imidazo[1,2-*b*]pyridazin;
2-(4-Fluorphenyl)-6-hexahydropyrrolo[3,4-c]pyrrol-2(1*H*)-yl-3-pyridazin-4-yl-imidazo[1,2-*b*]pyridazin;
2-(4-Fluorphenyl)-6-(5-methylhexahydropyrrolo[3,4-*c*]pyrrol-2(1*H*)-yl)-3-pyridazin-4-yl-imidazo[1,2-*b*]pyridazin;
2-(4-Fluorphenyl)-6-(5-isopropylhexahydropyrrolo-[3,4-*c*]pyrrol-2(1*H*)-yl)-3-pyridazin-4-yl-imidazo[1,2-*b*]pyridazin;
2-{(5-[2-(4-Fluorphenyl)-3-pyridazin-4-yl-imidazo-[2,2-*b*]pyridazin-6-yl]-hexahydropyrrolo[3,4-*c*] pyrrol-2(1*H*)-yl}-ethanol;
2-(4-Fluorphenyl)-5-(5-benzylhexahydropyrrolo[3,4-c]pyrrol-2(1*H*)-yl)-3-pyridazin-4-yl-imidazo[1,2-*b*]pyridazin;
9-[2-(4-Fluorphenyl)-3-pyridazin-4-yl-imidazo[1,2-*b*]pyridazin-6-yl]-2,9-diazaspiro[5.5]undecan;
9-[2-(4-Fluorphenyl)-3-pyridazin-4-yl-imidazo[1,2-*b*]pyridazin-6-yl]-1-oxa-4,9-diazaspiro[5.5]undecan;
6-[1,3']Bipyrrolidinyl-1'-yl-2-(4-fluorphenyl)-3-pyridazin-4-yl-imidazo[1,2-*b*]pyridazin;
2-(4-Fluorphenyl)-3-pyridazin-4-yl-5-(4-pyrrolidin-1-yl-piperidin-1-yl)-imidazo[1,2-*b*]pyridazin;
2-(3,5-Dimethylphenyl)-3-pyridazin-4-yl-6-(4-pyrrolidin-1-yl-piperidin-1-yl)-imidazo[1,2-*b*]pyridazin;
2-(3,5-Difluorphenyl)-3-pyridazin-4-yl-6-(4-pyrrolidin-1-yl-piperidin-1-yl)-imidazo[1,2-*b*]-pyridazin;
2-(3,5-Dichlorphenyl)-3-pyridazin-4-yl-6-(4-pyrrolidin-1-yl-piperidin-1-yl)-imidazo[1,2-*b*]pyridazin;
2-(3,5-Di(trifluormethyl)phenyl)-3-pyridazin-4-yl-6-(4-pyrrolidin-1-yl-piperidin-1-yl)-imidazo[1,2-b]pyridazin;
(+/-)-{4-[2-(4-Fluorphenyl)-3-pyridazin-4-yl-imidazo[1,2-*b*]pyridazin-5-yl]piperazin-2-yl}-methanol;
(+/-)-6-(3-Fluormethylpiperazin-1-yl)-2-(4-fluorphenyl)-3-pyridazin-4-ylimidazo[1,2-*b*]pyridazin;
6-(5-Methylhexahydropyrrolo[3,4-*c*]pyrrol-2(1*H*)-yl)-2-phenyl-3-pyridazin-4-ylimidazo[1,2-*b*]pyridazin;
2-(3-Fluorphenyl)-6-(5-methylhexahydropyrrolo[3,4-*c*]pyrrol-2(1*H*)-yl)-3-pyridazin-4-ylimidazo[1,2-*b*]pyridazin;
2-(4-Fluorphenyl)-7-methyl-6-(5-methylhexahydro-pyrrolo[3,4-*c*]pyrrol-2(1*H*)-yl)-3-pyridazin-4-yl-imidazo[1,2-*b*]pyridazin;
2-(4-Fluorphenyl)-8-methyl-6-(5-methylhexahydro-pyrrolo[3,4-*c*]pyrrol-2(1*H*)-yl)-3-pyridazin-4-yl-imidazo[1,2-*b*]pyridazin;
2-(3,4-Difluorphenyl)-6-(5-methylhexahydropyrrolo-[3,4-*c*]pyrrol-2(1*H*)-yl)-3-pyridazin-4-ylimidazo-[1,2-b]pyridazin.

14. Verfahren zur Herstellung einer Verbindung der Formel (I) nach Anspruch 1, dadurch gekerinzeichnet, dass man eine Verbindung der Formel (IIa) 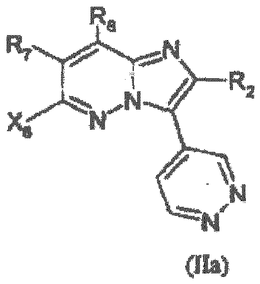 worin R₂, R₇ und R₈ wie in Anspruch 1 definiert sind und X₆ für eine Abgangsgruppe wie ein Halogen steht, in einem polaren Lösungsmittel mit einem Amin der allgemeinen Formel (IIb) 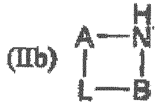 worin N, L, A und B wie in Anspruch 1 definiert sind, umsetzt.

15. Verfahren zur Herstellung einer Verbindung der Formel (I) nach Anspruch 1, **dadurch gekennzeichnet, dass** man eine Verbindung der Formel (IIIa) 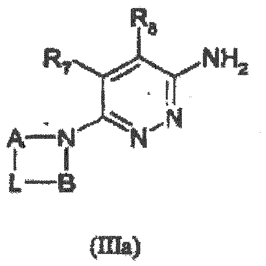 worin A, L, B, R₇ und R₈ wie in Anspruch 1 definiert sind, in einem polaren Lösungsmittel mit einem 2-Brom-2-(pyridazin-4-yl)ethan-1-onderivat der allgemeinen Formel (IIIb) 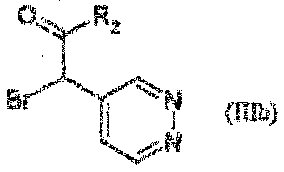 worin R₂ wie in Anspruch 1 definiert ist, umsetzt.

16. Verfahren zur Herstellung einer Verbindung der Formel (I) nach Anspruch 1, **dadurch gekennzeichnet, dass** man eine Verbindung der allgemeinen Formel (IVa) 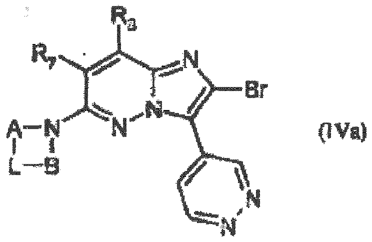 worin A, L, B, R₇ und R₈ wie in Anspruch 1 definiert sind, in Gegenwart eines Katalysators in einem Lösungsmittel mit einem Arylderivat der allgemeinen Formel (IVb)
M-R₂ (IVb),
worin R₂ wie in Anspruch 1 definiert ist und M für eine Trialkylstannylgruppe oder eine Dihydroxyboryl- oder Dialkyloxyborylgruppe steht, umsetzt.

17. Verfahren zur Herstellung einer Verbindung der Formel (I) nach Anspruch, **dadurch gekennzeichnet, dass** man:
a) eine Verbindung der Formel (V) 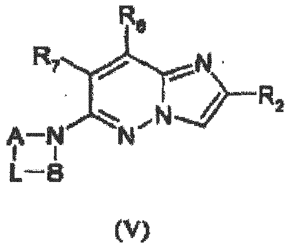 worin R₂, A, L, B, R₇ und R₈ wie in Anspruch 1 definiert sind, mit Pyridazin und einem Chlorameisensäurealkylester zu einer Verbindung der allgemeinen Formel (VI) 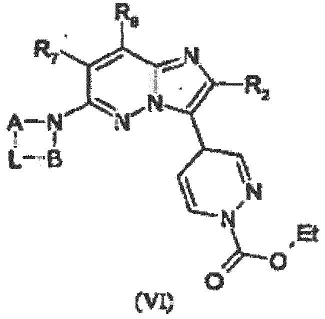 worin R₂, A, L, B, R₇ und R₈ wie in Anspruch 1 definiert sind, umsetzt;
b) die im vorhergehenden Schritt erhaltene Verbindung der allgemeinen Formel (VI) in einem Lösungsmittel mit ortho-Chloranil umsetzt.

18. Medikament, **dadurch gekennzeichnet, dass** es eine Verbindung der Formel (I) nach einem der Ansprüche 1 bis 13 in Basenform oder in Form eines Additionssalzes mit einer pharmazeutisch unbedenklichen Säure umfasst.

19. Pharmazeutische Zusammensetzung, **dadurch gekennzeichnet, dass** sie eine Verbindung der Formel (I) nach einem der Ansprüche 1 bis 13 in Basenform oder in Form eines Additionssalzes mit einer pharmazeutisch unbedenklichen Säure sowie mindestens einen pharmazeutisch unbedenklichen Hilfsstoff umfasst.

20. Verbindung der allgemeinen Formel (I) nach einem der Ansprüche 1 bis 13 zur Verwendung zur Behandlung oder Prävention von Schlafstörungen oder Störungen des zirkadianen Rhythmus.

21. Verbindung der allgemeinen Formel (I) nach einem der Ansprüche 1 bis 13 zur Verwendung zur Behandlung oder Prävention von Erkrankungen, die mit Drogenabhängigkeit assoziiert sind.

22. Verbindung der allgemeinen Formel (I) nach einem der Ansprüche 1 bis 13 zur Verwendung zur Behandlung oder Prävention von Alzheimer-Krankheit.

23. Verbindung der allgemeinen Formel (I) nach einem der Ansprüche 1 bis 13 zur Vervvendung zur Behandlung oder Prävention von Erkrankungen, die durch Zellproliferation verursacht oder verschlimmert werden.

24. Verbindung der allgemeinen Formel (I) nach Anspruch 23, **dadurch gekennzeichnet, dass** es sich bei den Zellen um Tumorzellen handelt.

## Claims

1. Compound of general formula (I) 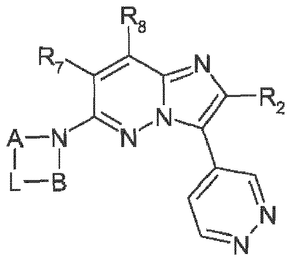 in which:
- R₂ represents an aryl group optionally substituted with one or more substituents chosen from halogen atoms and the groups C₁₋₆ alkyl, C₁₋₆ alkyloxy, C₁₋₆ alkylthio, C₁₋₆ fluoroalkyl, C₁₋₆-fluoroalkyloxy and -CN;
- A represents a group C₁₋₇-alkylene optionally substituted with one or two groups Rₐ;
- B represents a group C₁₋₇-alkylene optionally substituted with a group R_{b};
- L represents either a nitrogen atom optionally substituted with a group R_{c} or R_{d}, or a carbon atom substituted with a group Rₑ₁ and a group R_{d} or two groups R_{e2;}
the carbon atoms of A and B being optionally substituted with one or more groups R_{f}, which may be identical to or different from one another;
- Rₐ, R_{b} and R_{c} are defined such that:
two groups Rₐ may together form a group C₁₋₆-alkylene;
Rₐ and R_{b} may together form a bond or a group C₁₋₆-alkylene;
Rₐ and R_{c} may together form a bond or a group C₁₋₆-alkylene;
R_{b} and R_{c} may together form a bond or a group C₁₋₆-alkylene;
- R_{d} represents a group chosen from a hydrogen atom and the groups C₁₋₆-alkyl, C₃₋₇-cycloalkyl, C₃₋₇-cycloalkyl-C₁₋₆-alkyl, hydroxy-C₁₋₆-alkyl, C₁₋₆-alkyloxy-C₁₋₆-alkyl, C₁₋₆-alkylthio-C₁₋₆-alkyl, C₁₋₆-fluoroalkyl and benzyl;
- Rₑ₁ represents a group -NR₄R₅ or a cyclic monoamine optionally comprising an oxygen atom, the cyclic monoamine being optionally substituted with one or more substituents chosen from a fluorine atom and the groups C₁₋₆-alkyl, C₁₋₆-alkyloxy and hydroxyl;
- Two groups Rₑ₂ form, with the carbon atom that bears them, a cyclic monoamine optionally comprising an oxygen atom, this cyclic monoamine being optionally substituted with one or more groups R_{f}, which may be identical to or different from one another;
- R_{f} represents a group C₁₋₆-alkyl, C₃₋₇-cycloalkyl, C₃₋₇-cycloalkyl-C₁₋₆-alkyl, C₁₋₆-alkyloxy-C₁₋₆-alkyl, hydroxy-C₁₋₆-alkyl, C₁₋₆-fluoroalkyl or benzyl;
- R₄ and R₅ represent, independently of each other, a hydrogen atom or a group C₁₋₆-alkyl, C₃₋₇-cycloalkyl or C₃₋₇-cycloalkyl-C₁₋₆-alkyl ;
- R₇ and R₈ represent, independently of each other, a hydrogen atom or a group C₁₋₆-alkyl;
in the form of a base or of an addition salt with an acid.

2. Compound of general formula (I) according to Claim 1, **characterized in that** R₂ represents a phenyl optionally substituted with one or more halogen atoms or groups C₁₋₆ alkyl or C₁₋₆ fluoroalkyl.

3. Compound of the general formula (I) according to either one of Claims 1 and 2, **characterized in that** R₂ represents a phenyl, 3-fluorophenyl, 4-fluorophenyl, 3,4-difluorophenyl, 3,5-difluorophenyl, 3,5-dichlorophenyl, 3,5-dimethylphenyl or 3,5-di(trifluoromethyl)phenyl.

4. Compound of general formula (I) according to any one of Claims 1 to 3, **characterized in that** R₇ and R₈ represent, independently of each other, a hydrogen atom or a methyl group.

5. Compound of general formula (I) according to any one of Claims 1 to 4, **characterized in that**:
- A represents a group C₁₋₇-alkylene optionally substituted with one or two groups Rₐ;
- B represents a group C₁₋₇-alkylene optionally substituted with a group R_{b};
- L represents a nitrogen atom optionally substituted with a group R_{c} or R_{d};
the carbon atoms of A and of B being optionally substituted with one or more groups R_{f}, which may be identical to or different from each other;
- Rₐ and R_{b} may together form a bond or a group C₁₋₆-alkylene;
- Rₐ and R_{c} may together form a bond or a group C₁₋₆-alkylene;
- R_{b} and R_{c} may together form a bond or a group C₁₋₆-alkylene;
- R_{d} represents a substituent chosen from a group C₁₋₆-alkyl, C₃₋₇-cycloalkyl, C₃₋₇-cycloalkyl-C₁₋₆-alkyl, hydroxy-C₁₋₆-alkyl, C₁₋₆-alkyloxy-C₁₋₆-alkyl, C₁₋₆-fluoroalkyl or benzyl;
- R_{f} represents a group C₁₋₆-alkyl, hydroxy-C₁₋₆-alkyl, C₁₋₆-alkyloxy-C₁₋₆-alkyl or C₁₋₆-fluoroalkyl;

6. Compound of general formula (I) according to any one of Claims 1 to 4, **characterized in that** the cyclic amine formed by -N-A-L-B- represents a piperazin-1-yl, 3-(*R*)-methylpiperazin-1-yl, 3,3-dimethylpiperazin-1-yl, *cis-*3,5-dimethyipiperazin-1-yl, 4-methylpiperazin-1-yl, 4-(2-hydroxyethyl)piperazin-1-yl, 4-(2-fluoroethyl)piperazin-1-yl, 4-isopropylpiperazin-1-yl, 4-cyclopropylpiperazin-1-yl, 4-benzylpiperazin-1-yl, 4-(2-hydroxy-2-methylpropyl)piperazin-1-yl, 4-(3-hydroxy-3-methylbutyl)piperazin-1-yl, (*S*)-octahydropyrrolo[1,2-*a*]pyrazin-2-yl, (1*S*,4*S*)-2,5-diazabicyclo[2.2.1]hept-2-yl, 5-(2-hydroxyethyl)-(1*S*,4*S*)-2,5-diazabicyclo[2.2.1]hept-2-yl, 5-isopropyl-(1*S*,4*S*)-2,5-diazabicyclo[2.2.1]hept-2-yl, hexahydropyrrolo[3,4-*c*]pyrrol-2-(1*H*)-yl, 5-methyl-hexahydropyrrolo[3,4-*c*]pyrrol-2-(1*H*)-yl, 5-(2-isopropyl)hexahydropyrrolo[3,4-*c*]pyrrol-2-(1*H*)-yl, 5-(2-hydroxyethyl)hexahydropyrrolo[3,4-*c*]pyrrol-2-(1*H*)-yl, 5-(benzyl)hexahydro-pyrrolo[3,4-*c*]pyrrol-2-(1*H*)-yl, 3-(hydroxymethyl)piperazin-1-yl or 3-fluoromethylpiperazin-1-yl group.

7. Compound of general formula (I) according to any one of Claims 1 to 4, **characterized in that**:
- A represents a group C₁₋₇-alkylene;
- B represents a group C₁₋₇-alkylene;
- L represents a carbon atom substituted with two groups Rₑ₂;
the carbon atoms of A and of B being optionally substituted with one or more groups R_{f}, which may be identical to or different from each other;
- R_{f} represents a group C₁₋₆-alkyl;
- Two Rₑ₂ groups form, with the carbon atom which bears them, an azetidine, pyrrolidine, piperidine or morpholine group.

8. Compound of general formula (I) according to any one of Claims 1 to 4, **characterized in that** the cyclic amine formed by -N-A-L-B- represents a 2,9-diazaspiro[5.5]undec-3-yl or 1-oxa-4,9-diazaspiro[5.5]undec-9-yl group.

9. Compound of general formula (I) according to any one of Claims 1 to 4, **characterized in that**:
- A represents a group C₁₋₇-alkylene;
- B represents a group C₁₋₇-alkylene;
- L represents a carbon atom substituted with a group Rₑ₁ and a group R_{d};
- R_{d} represents a hydrogen atom;
- Rₑ₁ represents a group -NR_{d}R₅ or a cyclic monoamine;
- R₄ and R₅ represent, independently of each other, a hydrogen atom or a group C₁₋₆-alkyl, C₃₋₇-cycloalkyl or C₃-₇-cycloalkyl-C₁₋₆-alkyl.

10. Compound of general formula (I) according to any one of Claims 1 to 4, **characterized in that**:
- the cyclic amine formed by -N-A-L-B- represents a (*R*,*S*)-3-[pyrrolidin-1-yl]-pyrrolidin-1-yl or 4-(pyrrolidin-1-yl)piperidin-1-yl group.

11. Compound of general formula (I) according to Claim 1, **characterized in that**:
- R₂ represents a phenyl, 3-fluorophenyl, 4-fluorophenyl, 3,4-difluorophenyl, 3,5-difluorophenyl, 3,5-dichlorophenyl, 3,5-dimethylphenyl or 3,5-di(trifluoromethyl)phenyl group;
- the cyclic amine formed by -N-A-L-B- represents a piperazin-1-yl, 3-(*R*)-methylpiperazin-1-yl, 3,3-dimethylpiperazin-1-yl, *cis*-3,5-dimethylpiperazin-1-yl, 4-methylpiperazin-1-yl, 4-(2-hydroxyethyl)piperazin-1-yl, 4-(2-fluoroethyl)piperazin-1-yl, 4-isopropylpiperazin-1-yl, 4-cyclopropylpiperazin-1-yl, 4-benzylpiperazin-1-yl, 4-(2-hydroxy-2-methylpropyl)piperazin-1-yl, 4-(3-hydroxy-3-methylbutyl)piperazin-1-yl, (*S*)-octahydropyrrolo[1,2-*a*]pyrazin-2-yl, (1*S*,4*S*)-2,5-diazabicyclo[2.2.1]hept-2-yl, 5-(2-hydroxyethyl)-(1*S*,4*S*)-2,5-diazabicyclo[2.2.1]hept-2-yl, 5-isopropyl-(1*S*,4*S*)-2,5-diazabicyclo[2.2.1]hept-2-yl, hexahydropyrrolo[3,4-*c*]pyrrol-2-(1*H*)-yl, 5-methyl-hexahydropyrrolo[3,4-*c*]pyrrol-2-(1*H*)-yl, 5-(2-isopropyl)hexahydropyrrolo[3,4-*c*]pyrrol-2-(1*H*)-yl, 5-(2-hydroxyethyl)hexahydropyrrolo[3,4-*c*]pyrrol-2-(2*H*)-yl, 5-(benzyl)hexahydro-pyrrolo[3,4-*c*]pyrrol-2-(1*H*)-yl, 3-(hydroxymethyl)piperazin-1-yl or 3-fluoromethylpiperazin-1-yl group;
- R₇ and R₈ represent, independently of each other, a hydrogen atom or a methyl group.

12. Compound of general formula (I) according to Claim 1, **characterized in that**:
- R₂ represents a 4-fluorophenyl, 3,5-difluorophenyl, 3,5-dichlorophenyl, 3,5-dimethylphenyl or 3,5-di(trifluoromethyl)phenyl group;
- the cyclic amine formed by -N-A-L-B- represents a (*R*,*S*)-3-[pyrrolidin-1-yl]pyrrolidin-1-yl or 4-(pyrrolidin-1-yl)piperidin-1-yl group;
- R₇ and R₈ represent a hydrogen atom.

13. Compound of general formula (I) according to Claim 1, **characterized in that** it is chosen from the following compounds:
2-(4-fluorophenyl)-6-piperazin-1-yl-3-pyridazin-4-yl-imidazo[1,2-*b*]pyridazine;
2-(4-fluorophenyl)-6-((*R*)-3-methylpiperazin-1-yl)-3-pyridazin-4-yl-imidazo[1,2-*b*]pyridazine;
6-(3,3-dimethylpiperazin-7-yl)-2-(4-fluorophenyl)-3-pyridazin-4-yl-imidazo[1,2-*b*]pyridazine;
6-(*cis*-3,5-dimethylpiperazin-1-yl)-2-(4-fluorophenyl)-3-pyridazin-4-yl-imidazo[1,2-*b*]pyridazine;
2-(4-fluorophenyl)-6-(4-methylpiperazin-1-yl)-3-pyridazin-4-yl-imidazo[1,2-*b*]pyridazine
2-{4-[2-(4-fluorophenyl)-3-pyridazin-4-yl-imidazo[1,2-*b*]pyridazin-6-yl]piperazin-1-yl}-ethanol;
6-[4-(2-fluoroethyl)piperazin-1-yl]-2-phenyl-3-pyridazin-4-yl-imidazo[2,2-*b*]pyridazine;
6-[4-(2-fluoroethyl)piperazin-1-yl]-2-(4-fluorophenyl)-3-pyridazin-4-yl-imidazo[1,2-*b*]pyridazine;
6-(4-isopropylpiperazin-1-yl)-2-phenyl-3-pyridazin-4-yl-imidazo[1,2-*b*]pyridazine;
2-(3-fluorophenyl)-5-(4-isopropylpiperazin-1-yl)-3-pyridazin-4-yl-imidazo[1,2-*b*]pyridazine;
2-(4-fluorophenyl)-6-(4-isopropylpiperazin-1-yl)-3-pyridazin-4-yl-imidazo[1,2-*b*]pyridazine;
2-(4-fluorophenyl)-6-(4-isopropylpiperazin-1-yl)-7,8-dimethyl-3-pyridazin-4-yl-imidazo[1,2-*b*]pyridazine;
2-(3,5-dimethylphenyl)-6-(4-isopropylpiperazin-1-yl)-3-pyridazin-4-yl-imidazo[1,2-*b*]pyridazine;
2-(3,4-difluorophenyl)-6-(4-isopropylpiperazin-1-yl)-3-pyridazin-4-yl-imidazo[1,2-*b*]pyridazine;
2-(3,5-difluorophenyl)-6-(4-isopropylpiperazin-1-yl)-3-pyridazin-4-yl-imidazo[1,2-*b*]pyridazine;
2-(3,5-dichlorophenyl)-6-(4-isopropylpiperazin-1-yl)-3-pyridazin-4-yl-imidazo[1,2-*b*]pyridazine;
2-(3,5-di(trifluoromethyl)phenyl)-6-(4-isopropylpiperazin-1-yl)-3-pyridazin-4-yl-imidazo[1,2-*b*]pyridazine;
6-(4-cyclopropylpiperazin-1-yl)-2-(4-fluorophenyl)-3-pyridazin-4-yl-imidazo[1,2-*b*]pyridazine;
6-(4-benzylpiperazin-1-yl)-2-(4-fluorophenyl)-3-pyridazin-4-yl-imidazo[1,2-*b*]pyridazine;
1-{4-[2-(4-fluorophenyl)-3-pyridazin-4-yl-imidazo[1,2-*b*]pyridazin-6-yl]piperazin-1-yl)-2-methylpropan-2-ol;
4-{4-[2-(4-fluorophenyl)-3-pyridazin-4-yl-imidazo[1,2-*b*]pyridazin-6-yl]piperazin-1-yl)-2-methylbutan-2-ol;
2-(4-fluorophenyl)-6-(*S*)-hexahydropyrrolo[1,2-a]pyrazin-2-yl-3-pyridazin-4-yl-imidazo[1,2-*b*]pyridazine;
6-((2*S*,4*S*)-2,5-diazabicyclo[2.2.1]hept-2-yl)-2-(4-fluorophenyl)-3-pyridazin-4-yl-imidazo[1,2-b]pyridazine;
2-{5-[2-(4-fluorophenyl)-3-pyridazin-4-yl-imidazo[1,2-*b*]pyridazin-6-yl]-(1*S*,4*S*)-2,5-diazabicyclo[2.2.1]hept-2-yl}-ethanol;
2-(4-fluorophenyl)-6-(5-isopropyl-(1*S*,4*S*)-2,5-diazabicyclo[2.2.1]hept-2-yl)-3-pyridazin-4-yl-imidazo[1,2-*b*]pyridazine;
2-(4-fluorophenyl)-6-hexahydropyrrolo[3,4-*c*]pyrrol-2(1*H*)-yl-3-pyridazin-4-yl-imidazo[1,2-*b*]pyridazine;
2-(4-fluorophenyl)-6-(5-methylhexahydropyrrolo[3,4-*c*]pyrrol-2(1*H*)-yl)-3-pyridazin-4-yl-imidazo[1,2-*b*]pyridazine;
2-(4-fluorophenyl)-6-(5-isopropylhexahydropyrrolo[3,4-*c*]pyrrol-2(1*H*)-yl)-3-pyridazin-4-yl-imidazo[1,2-*b*]pyridazine;
2-{(5-[2-(4-fluorophenyl)-3-pyridazin-4-yl-imidazo[1,2-*b*]pyridazin-6-yl]-hexahydropyrrolo[3,4-*c*]pyrrol-2(1*H*)-yl}-ethanol;
2-(4-fluorophenyl)-6-(5-benzylhexahydropyrrolo[3,4-*c*]pyrrol-2(1*H*)-yl)-3-pyridazin-4-yl-imidazo[1,2-*b*]pyridazine;
9-[2-(4-fluorophenyl)-3-pyridazin-4-yl-imidazo[1,2-*b*]pyridazin-6-yl]-2,9-diazaspiro[5.5]undecane;
9-[2-(4-fluorophenyl)-3-pyridazin-4-yl-imidazo[1,2-*b*]pyridazin-6-yl]-1-oxa-4,9-diaza-spiro[5.5]undecane;
6-[1,3']bipyrrolidinyl-1'-yl-2-(4-fluorophenyl)-3-pyridazin-4-yl-imidazo[1,2-*b*]pyridazine;
2-(4-fluorophenyl)-3-pyridazin-4-yl-6-(4-pyrrolidin-1-yl-piperidin-1-yl)-imidazo[1,2-*b*]pyridazine;
2-(3,5-dimethylphenyl)-3-pyridazin-4-yl-6-(4-pyrrolidin-1-yl-piperidin-1-yl)-imidazo[1,2-*b*]pyridazine;
2-(3,5-difluorophenyl)-3-pyridazin-4-yl-5-(4-pyrrolidin-1-yl-piperidin-1-yl)-imidazo[1,2-*b*]pyridazine;
2-(3,5-dichlorophenyl)-3-pyridazin-4-yl-6-(4-pyrrolidin-1-yl-piperidin-1-yl)-imidazo[1,2-*b*]pyridazine;
2-(3,5-di(trifluoromethyl)phenyl)-3-pyridazin-4-yl-6-(4-pyrrolidin-1-yl-piperidin-1-yl)-imidazo[1,2-*b*]pyridazine;
(+/-)-{4-[2-(4-fluorophenyl)-3-pyridazin-4-ylimidazo[1,2-b]pyridazin-6-yl]piperazin-2-yl}methanol;
(+/-)-6-(3-fluoromethylpiperazin-1-yl)-2-(4-fluorophenyl)-3-pyridazin-4-ylimidazo[1,2-*b*]pyridazine;
6-(5-methylhexahydropyrrolo[3,4-*c*]pyrrol-2(1*H*)-yl)-2-phenyl-3-pyridazin-4-ylimidazo[1,2-*b*]pyridazine;
2-(3-fluorophenyl)-6-(5-methylhexahydropyrrolo[3,4-*c*]pyrrol-2(1*H*)-yl)-3-pyridazin-4-ylimidazo[1,2-*b*]pyridazine;
2-(4-fluorophenyl)-7-methyl-6-(5-methylhexahydropyrrolo[3,4-*c*]pyrrol-2-(1*H*)-yl)-3-pyridazin-4-ylimidazo[1,2-*b*]pyridazine;
2-(4-fluorophenyl)-8-methyl-6-(5-methylhexahydropyrrolo[3,4-c]pyrrol-2(1*H*)-yl)-3-pyridazin-4-ylimidazo[1,2-*b*]pyridazine;
2-(3,4-difluorophenyl)-6-(5-methylhexahydropyrrolo[3,4-*c*]pyrrol-2(1*H*)-yl)-3-pyridazin-4-ylimidazo[1,2-b]pyridazine.

14. Process for preparing a compound of formula (I) according to Claim 1, **characterized in that** a compound of formula (IIa) 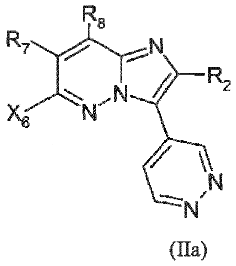 in which R₂, R₇ and R₈ are as defined in Claim 1 and X₆ represents a leaving group such as a halogen, is reacted with an amine of general formula (IIb) 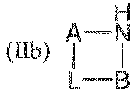 in which N, L, A and B are as defined in Claim 1, in a polar solvent.

15. Process for preparing a compound of formula (I) according to Claim 1, **characterized in that** a compound of formula (IIIa) 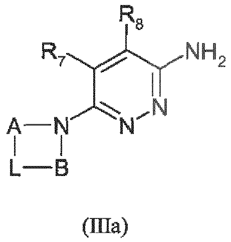 in which A, L, B, R₇ and R₈ are as defined in Claim 1, is reacted with a 2-bromo-2-(pyridazin-4-yl)ethan-1-one derivative of general formula (IIIb) 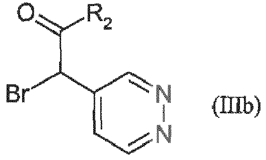 in which R₂ is as defined according to Claim 1, in a polar solvent.

16. Process for preparing a compound of formula (I) according to Claim 1, **characterized in that** a compound of general formula (IVa) 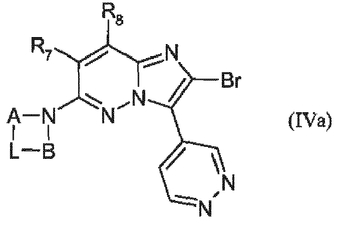 in which A, L, B, R₇ and R₈ are as defined in Claim 1, is reacted with an aryl derivative of general formula (IVb)
**M-R₂** (IVb)
in which R₂ is as defined according to Claim 1 and M represents a trialkylstannyl group or a dihydroxyboryl or dialkyloxyboryl group, in the presence of a catalyst in a solvent.

17. Process for preparing a compound of formula (I) according to Claim 1, **characterized in that** it comprises the following steps:
a) a compound of formula (V) 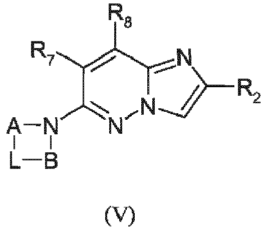 in which R₂, A, L, B, R₇ and R₈ are as defined according to Claim 1, is reacted with pyridazine and an alkyl chloroformate so as to obtain a compound of general formula (VI) 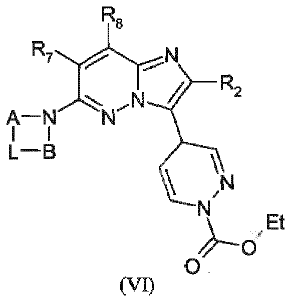 in which R₂, A, L, B, R₇ and R₈ are as defined according to Claim 1;
b) the compound of general formula (VI) obtained in the preceding step is reacted with ortho-chloranil in a solvent.

18. Medicament, **characterized in that** it comprises a compound of formula (I) according to any one of Claims 1 to 13, in the form of a base or an addition salt with a pharmaceutically acceptable acid.

19. Pharmaceutical composition, **characterized in that** it comprises a compound of formula (I) according to any one of Claims 1 to 13, in the form of a base or of an addition salt with a pharmaceutically acceptable acid, and also at least one pharmaceutically acceptable excipient.

20. Compound of general formula (I) according to any one of Claims 1 to 13, for use thereof for treating or preventing sleep disorders or circadian rhythm disorders.

21. Compound of general formula (I) according to one of Claims 1 to 13, for use thereof for treating or preventing diseases associated with a dependence on abuse substances.

22. Compound of general formula (I) according to any one of Claims 1 to 13, for use thereof for treating or preventing Alzheimer's disease.

23. Compound of general formula (I) according to any one of Claims 1 to 13, for use thereof for treating or preventing diseases caused or exacerbated by cell proliferation.

24. Compound of general formula (I) according to Claim 23, **characterized in that** the cells are tumour cells.
